# EUROPEAN PATENT SPECIFICATION

(11) **EP 0 728 139 B1**
(45) Date of publication and mention of the grant of the patent: **13.08.2003**
(21) Application number: 94928048.1
(22) Date of filing: 02.09.1994
(51) Int. Cl.: C07H 21/00, C07H 21/02, C07H 21/04, C12Q 1/68

(54) **AMINE-DERIVATIZED NUCLEOSIDES AND OLIGONUCLEOSIDES**
AMINODERIVATISIERTE NUKLEOSIDE UND OLIGONUKLEOSIDE
NUCLEOSIDES ET OLIGONUCLEOSIDES A FONCTION(S) AMINE

(30) Priority: 03.09.1993 US 117363
(43) Date of publication of application: 28.08.1996
(73) Proprietor: ISIS PHARMACEUTICALS, INC., Carlsbad, CA 92008 (US)
(72) Inventor: COOK, Phillip, Dan, San Marcos, California 92069 (US); MANOHARAN, Muthiah, Carlsbad, CA 92009 (US); GUINOSSO, Charles, J., Vista, CA 92083 (US)
(74) Representative: Hallybone, Huw George
(86) International application number: PCT/US94/10131
(87) International publication number: WO 95/006659

(56) References cited:
- WO-A-92/05186
- US-A- 4 910 300
- SCIENCE, Vol. 238, issued 04 December 1987, COREY et al., "Generation of a Hybrid Sequence-Specific Single Stranded Deoxyribonuclease", pages 1401-1403.

### SEQUENCE LISTING

(1) GENERAL INFORMATION:
   (i) APPLICANTS: ISIS Pharmaceuticals, Inc., et al.
   (ii) TITLE OF INVENTION: AMINE-DERIVATIZED NUCLEOSIDES AND OLIGONUCLEOSIDES
   (iii) NUMBER OF SEQUENCES: 41
   (iv) CORRESPONDENCE ADDRESS:
      (A) ADDRESSEE: Woodcock Washburn Kurtz Mackiewicz and Norris
      (B) STREET: One Liberty Place - 46th Floor
      (C) CITY: Philadelphia
      (D) STATE: PA
      (E) COUNTRY: U.S.A.
      (F) ZIP: 19103
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: WordPerfect 5.1
   (vi) CURRENT APPLICATION DATA:
      (A) APPLICATION NUMBER: N/A
      (B) FILING DATE: Herewith
      (C) CLASSIFICATION:
   (vii) PRIOR APPLICATION DATA:
      (A) APPLICATION NUMBER: 08/117,363
      (B) FILING DATE: 9/3/94
   (vii) PRIOR APPLICATION DATA:
      (A) APPLICATION NUMBER: 07/854,634
      (B) FILING DATE: 7/1/92
   (viii) ATTORNEY/AGENT INFORMATION:
      (A) NAME: Lucci, Joseph
      (B) REGISTRATION NUMBER: 33,307
      (C) REFERENCE/DOCKET NUMBER: ISIS-1691
   (ix) TELECOMMUNICATION INFORMATION:
      (A) TELEPHONE: 215-568-3100
      (B) TELEFAX: 215-568-3439
(2) INFORMATION FOR SEQ ID NO:1:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (xi) SEQUENCE DESCRIPTION: SEQ ID NO:1:
   GGCTGACTGC G 11
(2) INFORMATION FOR SEQ ID NO:2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:2:
   CTGTCTCCAT CCTCTTCACT 20
(2) INFORMATION FOR SEQ ID NO:3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:3:
   TTGCTTCCAT CTTCCTCGTC 20
(2) INFORMATION FOR SEQ ID NO:4:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:4:
   TGGGAGCCAT AGCGAGGC 18
(2) INFORMATION FOR SEQ ID NO:5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:5:
   CTGTCTCCAT CCTCTTCACT 20
(2) INFORMATION FOR SEQ ID NO:6:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:6:
   TCCAGGTGTC CGCATC 16
(2) INFORMATION FOR SEQ ID NO:7:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:7:
   GGACCGGAAG GTACGAG 17
(2) INFORMATION FOR SEQ ID NO:8:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:8:
   CCTGGCCTTC CATGCTC 17
(2) INFORMATION FOR SEQ ID NO:9:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (xi) SEQUENCE DESCRIPTION: SEQ ID NO:9:
   CCUGGCCUUC CAUGCUC 17
(2) INFORMATION FOR SEQ ID NO:10:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:10:
   CCCAGGCUCA GA 12
(2) INFORMATION FOR SEQ ID NO:11:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:11:
   GAGCUCCCAG GC 12
(2) INFORMATION FOR SEQ ID NO:12:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:12:
   CAUGCUGCAG CC 12
(2) INFORMATION FOR SEQ ID NO:13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:13:
   GCCTTTCGCG ACCCAACACU 20
(2) INFORMATION FOR SEQ ID NO:14:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (xi) SEQUENCE DESCRIPTION: SEQ ID NO:14:
(2) INFORMATION FOR SEQ ID NO:15:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:15:
   UGGGAGCCAT AGCGAGGC 18
(2) INFORMATION FOR SEQ ID NO:16:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:16:
   TGGGAGCCAU AGCGAGGC 18
(2) INFORMATION FOR SEQ ID NO:17:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:17:
   UCTGAGTAGC AGAGGAGCTC 20
(2) INFORMATION FOR SEQ ID NO:18:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:18:
   UGCCCAAGCT GGCATCCGTC A 21
(2) INFORMATION FOR SEQ ID NO:19:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:19:
   UGCGTTTGCT CTTCTTCTTG CG 22
(2) INFORMATION FOR SEQ ID NO:20:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:20:
   UGCATCCCCC AGGCCACCAT 20
(2) INFORMATION FOR SEQ ID NO:21:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:21:
   UCCCGCCTGT GACATGCATT 20
(2) INFORMATION FOR SEQ ID NO:22:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:22:
   GUTCTCGCTG GTGAGTTTCA 20
(2) INFORMATION FOR SEQ ID NO:23:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:23:
   UUGGGAGCCA TAGCGAGGC 19
(2) INFORMATION FOR SEQ ID NO:24:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:24:
   CCAAGCCUCA GA 12
(2) INFORMATION FOR SEQ ID NO:25:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:25:
   CCAGGCUCAG AT 12
(2) INFORMATION FOR SEQ ID NO:26:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:26:
   UGCATCCCCC AGGCCACCA 19
(2) INFORMATION FOR SEQ ID NO:27:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:27:
   UCAGTGCCTG CGCCGCGCTC G 21
(2) INFORMATION FOR SEQ ID NO:28:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:28:
   UCCGTCATCG CTCCTCAGGG 20
(2) INFORMATION FOR SEQ ID NO:29:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:29:
   UTTAGGATTC GTGCTCATGG 20
(2) INFORMATION FOR SEQ ID NO:30:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:30:
   UGTTCTCGCT GGTGAGTTTC A 21
(2) INFORMATION FOR SEQ ID NO:31:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:31:
   TGCATCCCCC AGGCCACCAU 20
(2) INFORMATION FOR SEQ ID NO:32:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:32:
   GTGCTCATGG TGCACGGTCU 20
(2) INFORMATION FOR SEQ ID NO:33:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:33:
   UGCATCCCCC AGGCCACCAU 20
(2) INFORMATION FOR SEQ ID NO:34:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:34:
   UGUUCUCGCT GGTGAGUUUC AU 22
(2) INFORMATION FOR SEQ ID NO:35:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (xi) SEQUENCE DESCRIPTION: SEQ ID NO:35:
   GCGTGUCTGG G 11
(2) INFORMATION FOR SEQ ID NO:36:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (xi) SEQUENCE DESCRIPTION: SEQ ID NO:36:
   GCGUGUCUGC G 11
(2) INFORMATION FOR SEQ ID NO:37:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (xi) SEQUENCE DESCRIPTION: SEQ ID NO:37:
   GGCTGUCTGC G 11
(2) INFORMATION FOR SEQ ID NO:38:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:38:
   GAGCTCCCAG GC 12
(2) INFORMATION FOR SEQ ID NO:39:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:39:
   CGACTATGCA AGTAC 15
(2) INFORMATION FOR SEQ ID NO:40:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (xi) SEQUENCE DESCRIPTION: SEQ ID NO:40:
   CGACTATGCA AAAAC 15
(2) INFORMATION FOR SEQ ID NO:41:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:41:
   ACCGAGGATCATGTCGTACGC 21

## Description

### FIELD OF THE INVENTION

This application is directed to nucleosides, oligonucleotides and oligonucleosides functionalized to include alkylamino functionality at a 3'-O-position or a 5'-O-position, and derivatives thereof. In certain embodiments, the compounds of the invention further include steroids, reporter molecules, reporter enzymes, lipophilic molecules, peptides or proteins attached to the nucleosides through the alkylamino group.

### BACKGROUND OF THE INVENTION

Messenger RNA (mRNA) directs protein synthesis. Antisense methodology is the complementary hybridization of relatively short oligonucleotides to mRNA or DNA such that the normal, essential functions of these intracellular nucleic acids are disrupted. Hybridization is the sequence-specific hydrogen bonding via Watson-Crick base pairs of oligonucleotides to RNA or single-stranded DNA. Such base pairs are said to be complementary to one another.

The naturally occurring events that provide the disruption of the nucleic acid function, discussed by Cohen in *Oligonucleotides: Antisense Inhibitors of Gene Expression,* CRC Press, Inc., Boca Raton, Florida (1989) are thought to be of two types. The first, hybridization arrest, denotes the terminating event in which the oligonucleotide inhibitor binds to the target nucleic acid and thus prevents, by simple steric hindrance, the binding of essential proteins, most often ribosomes, to the nucleic acid. Methyl phosphonate oligonucleotides (Miller, *et al.*, *Anti-Cancer Drug Design* **1987**, 2, 117) and α-anomer oligonucleotides are the two most extensively studied antisense agents which are thought to disrupt nucleic acid function by hybridization arrest.

The second type of terminating event for antisense oligonucleotides involves the enzymatic cleavage of the targeted RNA by intracellular RNase H. A 2'-deoxyribofuranosyl oligonucleotide or oligonucleotide analog hybridizes with the targeted RNA and this duplex activates the RNase H enzyme to cleave the RNA strand, thus destroying the normal function of the RNA. Phosphorothioate oligonucleotides are the most prominent example of an antisense agent that operates by this type of antisense terminating event.

Considerable research is being directed to the application of oligonucleotides and oligonucleotide analogs as antisense agents for diagnostics, research reagents and potential therapeutic purposes. At least for therapeutic purposes, the antisense oligonucleotides and oligonucleotide analogs must be transported across cell membranes or taken up by cells to express activity. One method for increasing membrane or cellular transport is by the attachment of a pendant lipophilic group.

Ramirez, *et al.*, *J. Am. Chem. Soc.* **1982**, *104*, 5483, introduced the phospholipid group 5'-O-(1,2-di-O-myristoyl-sn-glycero-3-phosphoryl) into the dimer TpT independently at the 3' and 5' positions. Subsequently Shea, et al., *Nuc. Acids Res.* **1990**, *18*, 3777, disclosed oligonucleotides having a 1,2-di-O-hexyldecyl-rac-glycerol group linked to a 5'-phosphate on the 5'-terminus of the oligonucleotide. Certain of the Shea, *et. al*. authors also disclosed these and other compounds in patent application PCT/US90/01002 (published as WO 90/10448). A further glucosyl phospholipid was disclosed by Guerra, *et al., Tetrahedron Letters* **1987,** *28*, 3581.

In other work, a cholesteryl group was attached to the inter-nucleotide linkage between the first and second nucleotides (from the 3' terminus) of an oligonucleotide. This work is disclosed in United States patent 4,958,013 and further by Letsinger, *et al., Proc. Natl. Acad. Sci. USA* **1989,** *86,* 6553. The aromatic intercalating agent anthraquinone was attached to the 2' position of a sugar fragment of an oligonucleotide as reported by Yamana, *et al., Bioconjugate Chem.* **1990,** *1*, 319. The same researchers placed pyrene-1-methyl at the 2' position of a sugar (Yamana *et. al., Tetrahedron Lett.* **1991,** *32,* 6347).

Lemairte, *et al., Proc. Natl. Acad. Sci. USA* **1986,** *84,* 648; and Leonetti, *et al., Bioconjugate Chem*. **1990,** *1*, 149. The 3' terminus of the oligonucleotides each include a 3'-terminal ribose sugar moiety. The poly(L-lysine) was linked to the oligonucleotide via periodate oxidation of this terminal ribose followed by reduction and coupling through a N-morpholine ring. Oligonucleotide-poly(L-lysine) conjugates are described in European Patent application 87109348.0. In this instance the lysine residue was coupled to a 5' or 3' phosphate of the 5' or 3' terminal nucleotide of the oligonucleotide. A disulfide linkage has also been utilized at the 3' terminus of an oligonucleotide to link a peptide to the oligonucleotide as is described by Corey, *et al., Science* **1987,** *238,* 1401; Zuckermann, *et al., J. Am. Chem. Soc.* **1988,** *110*, 1614; and Corey, *et al., J. Am. Chem. Soc*. **1989,** *111*, 8524.

Nelson, *et al., Nuc. Acids Res.* **1989,** *17*, 7187 describe a linking reagent for attaching biotin to the 3'-terminus of an oligonucleotide. This reagent, N-Fmoc-O-DMT-3-amino-1,2-propanediol is now commercially available from Clontech Laboratories (Palo Alto, CA) under the name 3'-Amine on. It is also commercially available under the name 3'-Amino-Modifier reagent from Glen Research Corporation (Sterling, VA). This reagent was also utilized to link a peptide to an oligonucleotide as reported by Judy, *et al., Tetrahedron Letters* **1991,** *32*, 879. A similar commercial reagent (actually a series of such linkers having various lengths of polymethylene connectors) for linking to the 5'-terminus of an oligonucleotide is 5'-Amino-Modifier C6. These reagents are available from Glen Research Corporation (Sterling, VA). These compounds or similar ones were utilized by Krieg, *et al*., *Antisense Research and Development* **1991**, *1*, 161 to link fluorescein to the 5'-terminus of an oligonucleotide. Other compounds of interest have also been linked to the 3'-terminus of an oligonucleotide. Asseline, *et al., Proc. Natl. Acad. Sci. USA* **1984,** *81*, 3297 described linking acridine on the 3'-terminal phosphate group of an poly (Tp) oligonucleotide via a polymethylene linkage. Haralambidis, *et al., Tetrahedron Letters* **1987,** *28*, 5199 report building a peptide on a solid state support and then linking an oligonucleotide to that peptide via the 3' hydroxyl group of the 3' terminal nucleotide of the oligonucleotide. Chollet, *Nucleosides & Nucleotides* **1990**, *9*, 957 attached an Aminolink 2 (Applied Biosystems, Foster City, CA) to the 5' terminal phosphate of an oligonucleotide. They then used the bifunctional linking group SMPB (Pierce Chemical Co., Rockford, Il) to link an interleukin protein to the oligonucleotide.

An EDTA iron complex has been linked to the 5 position of a pyrimidine nucleoside as reported by Dreyer, *et al., Proc. Natl. Acad. Sci. USA* **1985,** *82,* 968. Fluorescein has been linked to an oligonucleotide in the same manner as reported by Haralambidis, *et al., Nucleic Acid Research* **1987,** *15*, 4857 and biotin in the same manner as described in PCT application PCT/US/02198. Fluorescein, biotin and pyrene were also linked in the same manner as reported by Telser, *et al., J. Am. Chem. Soc*. **1989**, *111*, 6966. A commercial reagent, Amino-Modifier-dT, from Glen Research Corporation (Sterling, VA) can be utilized to introduce pyrimidine nucleotides bearing similar linking groups into oligonucleotides.

Cholic acid linked to EDTA for use in radioscintigraphic imaging studies was reported by Betebenner, *et. al., Bioconjugate Chem.* **1991,** *2*, 117. Manoharan et al. describe the conjugation of cholic acid to oligonucleotides (Annals of the New York Academy of Sciences, 1992, pages 306-309) and of cholic acid, biotin, fluorescein and digoxigenin to the 2'-O-position of adenosine incorporated into oligonucleotides (Tetrahedron letters, vol. 32, no. 49, 1991, pages 7171-7174).

### OBJECTS OF THE INVENTION

It is one object of this invention to provide nucleosides, oligonucleotides and oligonucleosides that include alkylamino chemical functionality at a 3'-O-position or a 5'-O-position.

It is a further object of the invention to provide compounds having improved transfer across cellular membranes.

It is another object to provide compounds that include intercalators, nucleic acid cleaving agents, cell surface phospholipids, and/or diagnostic agents.

It is yet another object to provide improvements in research and diagnostic methods and materials for assaying bodily states in animals, especially disease states.

It is an additional object of this invention to provide therapeutic and research materials having improved transfer and uptake properties for the treatment of diseases through modulation of the activity of DNA or RNA.

### BRIEF DESCRIPTION OF THE INVENTION

These and other objects are satisfied by the present invention, which provides compounds containing alkylamino chemical functionality. In one aspect, the invention provides nucleosides having base portions and ribofuranosyl sugar portions. Such nucleosides bear at a 3'-O-position. or a 5'-O-position a substituent having formula:

-R_{A}-N(R₁ₐ) (R_{1b})

where:
R_{A} is alkyl having from 1 to 10 carbon atoms;
R₁ₐ and R_{1b}, independently, are H, R₂, R_{A}, or an amine protecting group, or have formula C(X)-R₂, C(X)-R_{A}-R₂, C(X)-Q-R_{A}-R₂, C(X)-Q-R₂;
R₂ is a folate, a steroid molecule, a reporter molecule, a lipophilic molecule, a reporter enzyme, a peptide, a protein, or has formula -Q-(CH₂CH₂-Q-)ₓ-R₃;
X is O or S;
each Q is, independently, NH, O, or S;
x is 1 to 200;
R₃ is H, R_{A}, C(O)OH, C(O)OR_{A}, C(O)R₄, R_{A}-N₃, or R_{A}-NH₂; and
R₄ is Cl, Br, I, SO₂R₅ or has structure: 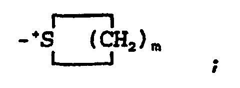
m is 2 to 7; and
R₅ is alkyl having 1 to 10 carbon atoms, provided that when one of R₁ₐ or R_{1b} is H, the other of R₁ₐ or R_{1b} is not H.

In another aspect, the invention provides oligonucleotides and oligonucleosides comprising a plurality of linked nucleosides, wherein each nucleoside includes a ribofuranosyl sugar portion and a base portion and at least one (preferably more than one) of the nucleosides bears at a 3'-O-position or a 5'-O-position a substituent having formula -R_{A}-N(R₁ₐ)(R_{1b}),
where:
R_{A} is alkyl having from 1 to 10 carbon atoms or (CH₂-CH₂-Q)ₓ;
R₁ₐ and R_{1b} are selected such that
R₁ₐ is H, R₂, an amine protecting group, or has formula C(X)-R₂, C(X)-R_{A}-R₂, C(X)-Q-R_{A}-R₂, or C(X)-Q-R₂,
R_{1b} is R₂, an amine protecting group, or has formula C(X)-R₂, C(X)-R_{A}-R₂, C(X)-Q-R_{A}-R₂, or C(X)-Q-R₂, or
R₁ₐ and R_{1b}, together, complete a cycloalkyl or cycloaryl moiety having two to six carbon atoms and one or two nitrogen atoms;
R₂ is a folate, a steroid molecule, a reporter molecule, a lipophilic molecule, a reporter enzyme, a peptide, a protein, or has formula -Q-(CH₂CH₂-Q-)ₓ-R₃;
X is O or S;
each Q is, independently, NH, O, or S;
x is 1 to 200;
R₃ is H, R_{A}, C(O)OH, C(O)OR_{A}, C(O)R₄, R_{A}-N₃, or R_{A}-NH₂;
R₄ is Cl, Br, I, SO₂R₅ or has structure: 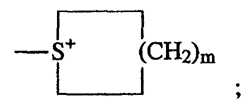
m is 2 to 7; and
R₅ is alkyl having 1 to 10 carbon atoms.

The oligonucleotides and oligonucleosides may be prepared by contacting nucleosides according to the invention for a time and under reaction conditions effective to form a covalent bond therebetween. In preferred embodiments, at least one of the nucleosides bears a phosphoramidate group at its 2'-O-position or at its 3'-O-position.

The compounds according to the invention may be prepared by contacting a nucleoside, oligonucleotide or oligonucleoside with derivatizing reagents. For example, a nucleoside, oligonucleotide or oligonucleoside bearing a 3'-hydroxy group or a 5'-hydroxy group may be contacted under basic conditions with a compound having formula L₁-R_{A}-N(R₁ₐ)(R_{1b}) wherein L₁ is a leaving group such as a halogen and at least one of R₁ₐ and R_{1b} is an amine protecting group.

The present invention also provides in vitro methods for detecting the presence or absence of an RNA in a biological sample suspected of containing said RNA, comprising contacting said sample with a compound of the present invention. The compound can be included in a composition that further includes an inert carrier for the compound.

### DETAILED DESCRIPTION OF THE INVENTION

This invention provides nucleosides, oligonucleotides and oligonucleosides containing alkylamino chemical functionality at a 3'-O-position or a 5'-O-position. The nucleoside subunits can be "natural" or "synthetic" moieties. Each nucleoside is formed from a naturally occurring or synthetic base and a naturally occurring or synthetic pentofuranosyl sugar group.

The term "oligonucleotide" refers to a polynucleotide formed from a plurality of linked nucleotide units. The nucleotides units each include a nucleoside unit. In the context of this invention, the term "oligonucleoside" refers to a plurality of nucleoside units that are linked together. In a generic sense, since each nucleotide unit of an oligonucleotide includes a nucleoside therein, the term "oligonucleoside" can be considered to be inclusive of oligonucleotides (*i.e*., nucleosides linked together via phosphate linking groups). In a further sense, the term "oligonucleoside" also refers to a plurality of nucleosides that are linked together via linkages other than phosphate linkages. The term "oligonucleoside" thus effectively includes naturally occurring species or synthetic species formed from naturally occurring subunits. For brevity, the term "oligonucleoside" will be used as encompassing both phosphate linked (oligonucleotides) and non-phosphate linked polynucleoside species.

Oligonucleosides according to the invention also can include modified subunits. Representative modifications include modification of a heterocyclic base portion of a nucleoside or a sugar portion of a nucleoside. Exemplary modifications are disclosed in the following United States Patent Applications: Serial No. 463,358, filed January 11, 1990, entitled Compositions And Methods For Detecting And Modulating RNA Activity; Serial No. 566,977, filed August 13, 1990, entitled Sugar Modified Oligonucleotides That Detect And Modulate Gene Expression; Serial No. 558,663, filed July 27, 1990, entitled Polyamine Conjugated Oligonucleotides, published as United States Patent 5,138,045; and Serial No. PCT/US91/00243, filed January 11, 1991, entitled Compositions and Methods For Detecting And Modulating RNA Activity, published as WO 91/10671. Each of these patent applications are assigned to the assignee of this invention.

The term oligonucleoside thus refers to structures that include modified portions, be they modified sugar moieties or modified base moieties, that function similarly to natural bases and natural sugars. Representative modified bases include deaza or aza purines and pyrimidines used in place of natural purine and pyrimidine bases; pyrimidines having substituent groups at the 5 or 6 position; and purines having altered or replacement substituent groups at the 2, 6 or 8 positions. Representative modified sugars include carbocyclic or acyclic sugars, sugars having substituent groups at their 2' position, and sugars having substituents in place of one or more hydrogen atoms of the sugar. Other altered base moieties and altered sugar moieties are disclosed in United States Patent 3,687,808 and PCT application PCT/US89/02323 (published as WO89/12060).

Altered base moieties or altered sugar moieties also include other modifications consistent with the spirit of this invention. Such oligonucleosides are best described as being structurally distinguishable and yet functionally interchangeable with naturally occurring or synthetic wild type oligonucleotides. All such oligonucleosides are comprehended by this invention so long as they function effectively to mimic the structure of a desired RNA or DNA strand.

For use in antisense methodology, the oligonucleosides of the invention preferably comprise from about 10 to about 30 subunits. It is more preferred that such oligonucleosides comprise from about 15 to about 25 subunits. As will be appreciated, a subunit is a base and sugar combination suitably bound to adjacent subunits through, for example, a phosphorous-containing (*e.g*., phosphodiester) linkage or some other linking moiety. The nucleosides need not be linked in any particular manner, so long as they are covalently bound. Exemplary linkages are those between the 3'- and 5'-positions or 2'- and 5'-positions of adjacent nucleosides. Exemplary linking moieties are disclosed in the following references: Beaucage, *et al., Tetrahedron* **1992,** *48*, 2223 and references cited therein; and United States Patent Applications: Serial No. 703,619, filed May 21, 1991, published as United States Patent 5,378,825; Serial No. 903,160, filed June 24, 1992. Each of the foregoing patent applications are assigned to the assignee of this invention.

It is preferred that the RNA or DNA portion which is to be modulated using oligonucleosides of the invention be preselected to comprise that portion of DNA or RNA which codes for the protein whose formation or activity is to be modulated. The targeting portion of the composition to be employed is, thus, selected to be complementary to the preselected portion of DNA or RNA, that is, to be an antisense oligonucleoside for that portion.

In accordance with one preferred embodiment of this invention, the compounds of the invention hybridize to HIV mRNA encoding the tat protein, or to the TAR region of HIV mRNA. In another preferred embodiment, the compounds mimic the secondary structure of the TAR region of HIV mRNA, and by doing so bind the tat protein. Other preferred compounds are complementary to sequences for herpes, papilloma and other viruses.

The nucleosides and oligonucleosides of the invention can be used in diagnostics, therapeutics and as research reagents and kits. They can be used in pharmaceutical compositions by including a suitable pharmaceutically acceptable diluent or carrier. They further can be used for treating organisms having a disease characterized by the undesired production of a protein. The organism should be contacted with an oligonucleotide having a sequence that is capable of specifically hybridizing with a strand of nucleic acid coding for the undesirable protein. Treatments of this type can be practiced on a variety of organisms ranging from unicellular prokaryotic and eukaryotic organisms to multicellular eukaryotic organisms. Any organism that utilizes DNA-RNA transcription or RNA-protein translation as a fundamental part of its hereditary, metabolic or cellular control is susceptible to therapeutic and/or prophylactic treatment in accordance with the invention. Seemingly diverse organisms such as bacteria, yeast, protozoa, algae, all plants and all higher animal forms, including warm-blooded animals, can be treated. Further, since each cell of multicellular eukaryotes can be treated since they include both DNA-RNA transcription and RNA-protein translation as integral parts of their cellular activity. Many of the organelles (*e.g*., mitochondria and chloroplasts) of eukaryotic cells also include transcription and translation mechanisms. Thus, single cells, cellular populations or organelles can also be included within the definition of organisms that can be treated with therapeutic or diagnostic oligonucleotides. As used herein, therapeutics is meant to include the eradication of a disease state, by killing an organism or by control of erratic or harmful cellular growth or expression.

In one aspect, the present invention is directed to nucleosides and oligonucleosides that bear at least one amine-containing substituent of formula -R_{A}-N(R₁ₐ)(R_{1b}) appended at 3'-O- and/or 5'-O-positions.

Each R_{A} according to the invention is an alkyl moiety independently selected to having 1 to 10 carbon atoms or R_{A} is a polyether, a polythioether or polyalkylamine. The term "alkyl" is intended to include straight chain and branched hydrocarbons. The preferred length of these hydrocarbons is 1 to 7 carbon atoms, more preferably 3 or 4 carbon atoms.

R₁ₐ and R_{1b} according to the invention are H, R₂, R_{A}, an amine protecting group, have formula C(X)-R₂, C(X)-R_{A}-R₂, C(X)-Q-R_{A}-R₂, C(X)-Q-R₂, or, together, complete a cycloalkyl or cycloaryl moiety having two to six carbon atoms and one or two nitrogen atoms such as an imidazole moiety. Protecting groups are known *per se* as chemical functional groups that can be selectively appended to and removed from functionalities, such as amine groups. These groups are present in a chemical compound to render such functionality inert to chemical reaction conditions to which the compound is exposed. *See, e.g.*, Greene and Wuts, Protective Groups in Organic Synthesis, 2d edition, John Wiley & Sons, New York, 1991. Numerous amine protecting groups are known in the art, including, but not limited to: phthalimide (PHTH), trifluoroacetate (triflate), allyloxycarbonyl (Alloc), benzyloxycarbonyl (CBz), chlorobenzyloxycarbonyl, t-butyloxycarbonyl (Boc), fluorenylmethoxycarbonyl (Fmoc), and isonicotinyloxycarbonyl (i-Noc) groups. (*see, e.g.,* Veber and Hirschmann, *et al., J. Org. Chem*. **1977**, *42*, 3286 and Atherton, *et al*., The Peptides, Gross and Meienhofer, Eds, Academic Press; New York, 1983; Vol. 9 pp. 1-38).

R₂ can include a folate a steroid molecule, a reporter molecule, a lipophilic molecule, a reporter enzyme, a peptide, a protein (*i.e*., a substituent consisting essentially of same), or a molecule having formula -Q-(CH₂CH₂-Q-)ₓ-R₃. For the purposes of this invention the terms "reporter molecule" and "reporter enzyme" are inclusive of those molecules or enzymes that have physical or chemical properties that allow them to be identified in gels, fluids, whole cellular systems, broken cellular systems and the like utilizing physical properties such as spectroscopy, radioactivity, colorimetric assays, fluorescence, and specific binding. Steroids include those chemical compounds that contain a perhydro-1,2-cyclopentanophenanthrene ring system. Proteins and peptides are utilized in their usual sense as polymers of amino acids. Normally peptides comprise such polymers that contain a smaller number of amino acids per unit molecule than do the proteins. Lipophilic molecules include naturally-occurring and synthetic aromatic and non-aromatic moieties such as fatty acids, esters, alcohols and other lipid molecules, substituted aromatic groups such as dinitrophenyl groups, cage structures such as adamantane and buckminsterfullerenes, and aromatic hydrocarbons such as benzene, perylene, phenanthrene, anthracene, naphthalene, pyrene, chrysene, and naphthacene.

Particularly useful as steroid molecules are the bile acids including cholic acid, deoxycholic acid and dehydrocholic acid; steroids including cortisone, digoxigenin, testosterone and cholesterol and even cationic steroids such as cortisone having a trimethylaminomethyl hydrazide group attached via a double bond at the 3 position of the cortisone rings. Particularly useful as reporter molecules are biotin, dinitrophenyl, and fluorescein dyes. Particularly useful as lipophilic molecules are alicyclic hydrocarbons, saturated and unsaturated fatty acids, waxes, terpenes and polyalicyclic hydrocarbons including adamantane and buckminsterfullerenes. Particularly useful as reporter enzymes are alkaline phosphatase and horseradish peroxidase. Particularly useful as peptides and proteins are sequence-specific peptides and proteins including phosphodiesterase, peroxidase, phosphatase and nuclease proteins. Such peptides and proteins include SV40 peptide, RNaseA, RNase H and Staphylococcal nuclease. Particularly useful as terpenoids are vitamin A, retinoic acid, retinal and dehydroretinol.

R₂ also can have formula -Q-(CH₂CH₂-Q-)ₓ-R₃, where Q is O er S. Subscript x can be 1 to 200, preferably 20 to 150, more preferably 10 to 50. Preferably, Q are selected to be O, such that R₂ constitutes a poly(ethyleneglycol) (PEG) group (*i.e*., R₃ = H) or a functionalized derivative thereof (*e.g.*, R₃ = C(O)Cl). R₃ can be H, R_{A}, C(O)OH, C(O)OR_{A}, C(O)R₄, R_{A}-N₃ or R_{A}-NH₂ where R₄ is Cl, Br, I, SO₂R₅ or a small thio-containing heterocycle having structure: 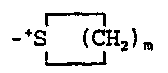 where m is 2 to 7. Representative PEG-containing R₂ groups are disclosed by Ouchi, *et al., Drug Design and Discovery* **1992,** *9*, 93, Ravasio, *et al., J. Org. Chem.* **1991,** *56*, 4329, and Delgardo *et. al., Critical Reviews in Therapeutic Drug Carrier Systems* **1992**, *9*, 249.

Oligonucleosides according to the invention can be assembled in solution or through solid-phase reactions, for example, on a suitable DNA synthesizer utilizing nucleosides according to the invention and/or standard nucleotide precursors. The nucleosides and nucleotide precursors can already bear alkylamino groups or can be later modified to bear such groups.

In the former case, compounds according to the invention are prepared by, for example, reacting nucleosides bearing at least one free 3'- or 5'-hydroxyl group under basic conditions with a compound having formula L₁-(CH₂)ₙ-N(R₁ₐ) (R_{1b}) where L₁ is a leaving group and at least one of R₁ₐ and R_{1b} is an amine protecting group. Displacement of the leaving group through nucleophilic attack of an oxygen anion produces the desired amine derivative. Leaving groups according to the invention include but are not limited to halogen, alkylsulfonyl, substituted alkylsulfonyl, arylsulfonyl, substituted arylsulfonyl, hetercyclcosulfonyl or trichloroacetimidate. A more preferred group includes chloro, fluoro, bromo, iodo, p-(2,4-dinitroanilino)benzenesulfonyl, benzenesulfonyl, methylsulfonyl (mesylate), p-methylbenzenesulfonyl (tosylate), p-bromobenzenesulfonyl, trifluoromethylsulfonyl (triflate), trichloroacetimidate, acyloxy, 2,2,2-trifluoroethanesulfonyl, imidazoleysulfonyl, and 2,4,6-trichlorophenyl, with bromo being preferred.

Suitably protected nucleosides can be assembled into an oligonucleosides according to known techniques. *See, e.g.,* Beaucage, *et al., Tetrahedron* **1992,** *48,* 2223.

Oligonucleosides according to the invention also can be prepared by assembling an oligonucleoside and appending alkylamino functionality thereto. For example, oligonucleosides having free hydroxyl groups can be assembled according to known techniques and then reacted with a reagent having formula L₁-(CH₂)ₙ-N(R₁ₐ)(R_{1b}). As will be recognized, however, greater selectivity can be achieved in terms of placement of alkylamino functionality within an oligonucleoside by introducing such functionality, as discussed above, on selected nucleosides and then using both the selected nucleosides and other nucleosides to construct an oligonucleoside.

Once assembled, an oligonucleoside bearing one or more groups having formula -R_{A}-N(R₁ₐ)(R_{1b}) wherein at least one of R₁ₐ and R_{1b} is a protecting group is treated with reagents effective to remove the protecting group. Once deprotected, the oligonucleoside can be contacted with electrophillic moieties such as, for example, succinimidyl esters and other activated carboxylic acids including C(=O)-O-succinimide and C(=O)-O-pentafluorophenyl, isothiocyanates, sulfonyl chlorides, halacetamides, phospholipid carbocyclic acid active esters, o-phenanthroline-5-iodoacetamide, fluorescein isothiocyanate, 1-pyrene butyric acid-N-hydroxy succinimide ester and carboxylic acid derivatives of PNA (carboxylic acid derivatives of peptide nucleic acids). Preferred electrophillic moieties include cholesteryl-3-hemisuccinate-N-hydroxy succinimide ester, pyrene-1-butyric acid-N-hydroxy succinimide ester and polyethylene glycol-propionic acid-N-hydroxy succimide ester.

Thus, the invention first builds the desired linked nucleoside sequence in the normal manner on the DNA synthesizer. One or more (preferably two or more) of the linked nucleosides are then functionalized or derivatized with the lipophilic steroid, reporter molecule, lipophilic molecule, reporter enzyme, peptide or protein.

Additional objects, advantages, and novel features of this invention will become apparent to those skilled in the art upon examination of the following examples, which are not intended to be limiting. All oligonucleotide sequences are listed in a standard 5' to 3' order from left to right. The term "Sephadex" is a Registered Trade Mark.

In the following examples, oligonucleotides having only 2'-O-positions substituted by -R_{A}-N(R₁)(R₂) are not part of this invention.

### EXAMPLE 1

### Oligonucleotides Having 2'-Protected-Amine Terminating Linking Group

### A. Preparation of 5-Dimethoxytrityl-2'-(O-Pentyl-N-phthalimido)-2'-Deoxyadenosine Phosphoramidite.

To introduce a functionalization at the 2' position of nucleotides within desired oligonucleotide sequences, 5'-dimethoxytrityl-2'-(O-pentyl-N-phthalimido)-2'-deoxyadenosine phosphoramidite was utilized to provide a linking group attached to the 2' position of nucleotide components of an oligonucleotide. This compound was synthesized generally in accordance with the procedures of patent application serial numbers PCT/US91/00243 (published as WO 91/10671) and 463,358, 1990, identified above, starting from adenosine. Briefly, this procedure treats adenosine with NaH in dimethylformamide (DMF) followed by treatment with N-(5-bromopentyl)phthalimide. Further treatment with (CH₃)₃SiCl, Ph-C(O)-Cl and NH₄OH yields N6-benzyl protected 2'-pentyl-N-phthalimido functionalized adenosine. Treatment with DIPA and CH₂Cl₂ adds a DMT blocking group at the 5' position. Finally phosphitylation gives the desired phosphoramidite compound. This compound was utilized in the DNA synthesizer as a 0.09M solution in anhydrous CH₃CN. Oligonucleotide synthesis was carried out in either an ABI 390B or an ABI 394 synthesizer employing the standard synthesis cycles with an extended coupling time of 10 minutes during coupling of Compound 2 into the oligonucleotide sequence. Coupling efficiency of greater than 98% was observed.

### B. 2'-Protected-Amine Linking Group Containing Phosphodiester Oligonucleotides

The following oligonucleotides having phosphodiester inter-nucleotide linkages were synthesized: wherein A* represents a nucleotide functionalized to incorporate a pentyl-N-phthalimido functionality. Oligomers 12 and 13 are antisense compounds to the E2 region of the bovine papilloma virus-1 (BPV-1). Oligomers 12 and 13 have the same sequence as Oligomer 3 in Application Serial Number 782,374, except for the 2' modification. The oligonucleotides were synthesized in either a 10 µmol scale or a 3 x 1 µmol scale in the "Trityl-On" mode. Standard deprotection conditions (30% NH₄OH, 55°C, 24 hours) were employed. The oligonucleotides were purified by reverse phase HPLC (Waters Delta-Pak C₄ 15 µm, 300A, 25x100 mm column equipped with a guard column of the same material). They were detritylated and further purified by size exclusion using a Sephadex G-25 column. NMR analyses by both proton and phosphorus NMR confirmed the expected structure for the Oligomers 9 and 10.

### C. 2'-Protected-Amine Linking Group Containing Phosphorothioate Oligonucleotides

The following oligonucleotides having phosphorothioate inter-nucleotide linkages were synthesized: and wherein A* represents a nucleotide functionalized to incorporate a pentyl-N-phthalimido functionality and the subscript "s" represents a phosphorothioate inter-nucleotide backbone linkage. Oligomer 14 is an antisense compound directed to the E2 region of the bovine papilloma virus-1 (BPV-1). Oligomers 15 and 16 are antisense compounds to ICAM. Oligomer 14 has the same sequence as Oligomer 3 in Application Serial Number 782,374, except for the 2' modification whereas Oligomers 15 and 16 have the same sequence as Oligomer 4 in Application Serial Number 782,374 except for the 2' modification. These oligonucleotides were synthesized as per the method of Example 1(B) except during the synthesis, for oxidation of the phosphite moieties, the Beaucage reagent (*i.e*., 3H-1,2-benzodithioate-3-one 1,1-dioxide, *see,* Radhakrishnan, *et al., J. Am. Chem. Soc.* **1990,** *112*, 1253) was used as a 0.24 M solution in anhydrous CH3CN solvent. The oligonucleotides were synthesized in the "Trityl-On" mode and purified by reverse phase HPLC utilizing the purification procedure of Example 1(B).

### D. 2'-O-Methyl Derivatized, 2'-Protected-Amine Linking Group Containing RNA Oligonucleotides

The following oligonucleotides having 2'-O-methyl groups on each nucleotide not functionalized with a 2' -protected amine functionalization were synthesized: and wherein A* represents a nucleotide functionalized to incorporate a pentyl-N-phthalimido functionality and where the remaining nucleotides except the 3'-terminus nucleotide are each 2'-O-methyl derivatized nucleotides. The 3'-terminus nucleotide in both Oligomers 17 and 18 is a 2'-deoxy nucleotide. Both Oligomers 17 and 18 are antisense compounds to the HIV-1 TAR region. The oligonucleotides were synthesized as per the method of Example 6 in Application Serial Number 782,374 (utilizing Compound 2 thereof) for introduction of the nucleotides containing the pentyl-N-phthalimido functionality and appropriate 2-O-methyl phosphoramidite nucleotides from Chemgenes Inc. (Needham, MA) for the remaining RNA nucleotides. The 3'-terminus terminal 2'-deoxy nucleotides were standard phosphoamidites utilized for the DNA synthesizer. The oligonucleotides were deprotected and purified as per the method of Example 1(B).

### EXAMPLE 2

### Functionalization Of Oligonucleotides At the 2' Position

### A. Functionalization with Biotin

### 1. Single Site Modification

About 10 O.D. units (A₂₆₀) of Oligomer 12 (*see*, Example 1) (approximately 60 nmols based on the calculated extinction coefficient of 1.6756 x 10⁵) was dried in a microfuge tube. The oligonucleotide was dissolved in 200 µl of 0.2 M NaHCO₃ buffer and D-biotin-N-hydroxysuccinimide ester (2.5 mg, 7.3 µmols) (Sigma, St. Louis, MO) was added followed by 40 µl DMF. The solution was let stand overnight. The solution was applied to a Sephadex G-25 column (0.7 x 15 cm) and the oligonucleotide fractions were combined. Analytical HPLC showed nearly 85% conversion to the product. The product was purified by HPLC (Waters 600E with 991 detector, Hamilton PRP-1 column 0.7 x 15 cm; solvent A: 50 mM TEAA pH 7.0; B: 45 mM TEAA with 80% acetonitrile: 1.5 ml flow rate: Gradient: 5% B for first 5 minutes, linear (1%) increase in B every minute thereafter) and further desalted on Sephadex G-25 to give the oligonucleotide: wherein A* represents a nucleotide functionalized to incorporate a biotin functionality linked via a 2'-O-pentyl-amino linking group to the 2' position of the designated nucleotide. HPLC retention times are shown in Table 1 below.

### 2. Multiple Site Modification

About 10 O.D. units (A₂₆₀) of Oligomer 13 (see, Example 1, approximately 60 nmols) was treated utilizing the method of Example 8(A)(1) in Application Serial Number 782,374 with D-biotin-N-hydroxysuccinimide ester (5 mg) in 300 µl of 0.2 M NaHCO₃ buffer/ 50 µl DMF. Analytical HPLC showed 65% of double labeled oligonucleotide product and 30% of single labeled products (from the two available reactive sites). HPLC and Sephadex G-25 purification gave the oligonucleotide: wherein A* represents nucleotides functionalized to incorporate a biotin functionality linked via a 2'-O-pentyl-amino linking group to the 2' position of the designated nucleotide. HPLC retention times for this product (and its accompanying singly labeled products) are shown in Table 1 below.

### B. Functionalization with Fluorescein

### 1. Single Site Modification

A 1M Na₂CO₃/1M NaHCO₃ buffer (pH 9.0) was prepared by adding 1M NaHCO₃ to 1 M Na₂CO₃. A 200 µl portion of this buffer was added to 10 O.D. units of Oligomer 12 (*see,* Example 1) in a microfuge tube. A 10 mg portion of fluorescein-isocyanate in 500 µl DMF was added to give a 0.05 M solution. A 100 µl portion of the fluorescein solution was added to the oligonucleotide solution in the microfuge tube. The tube was covered with aluminum foil and let stand overnight. The reaction mixture was applied to a Sephadex G-25 column (0.7 x 20 cm) that had been equilibrated with 25% (v/v) ethyl alcohol in water. The column was eluted with the same solvent. Product migration could be seen as a yellow band well separated from dark yellow band of the excess fluorescein reagent. The fractions showing absorption at 260 nm and 485 nm were combined and purified by HPLC as per the purification procedure of Example 2(A)(1). Analytical HPLC indicated 81% of the desired doubly functionalized oligonucleotide. The product was lyophilized and desalted on Sephadex to give the oligonucleotide: wherein A* represents a nucleotide functionalized to incorporate a fluorescein functionality linked via a 2'-O-pentyl-amino linking group to the 2' position of the designated nucleotide. HPLC retention times are shown in Table 1 below.

### 2. Multiple Site Modification

A 10 O.D. unit (A₂₆₀) portion of Oligomer 13 (from Example 1) was dissolved in 300 µl of the 1M Na₂HCO₃/ 1M Na₂CO₂ buffer of Example 2(B) (1) and 200 µl of the fluorescein-isothiocyanate stock solution of Example 2(B) (1) was added. The resulting solution was treated as per Example 2(B) (1). Analytical HPLC indicated 61% of doubly labeled product and 38% of singly labeled products. Work up of the reaction gave the oligonucleotide: wherein A* represents nucleotides functionalized to incorporate a fluorescein functionality linked via a 2'-O-pentyl-amino linking group to the 2' position of the designated nucleotide. HPLC retention times are shown in Table 1 below.

### C. Functionalization with Cholic Acid

### 1. Single Site Modification

A 10 O.D. unit (A₂₆₀) portion of Oligomer 12 (*see,* Example 1) was treated with cholic acid-NHS ester (Compound 1 in Application Serial Number 782,374, 5 mg, 9.9 µmols) in 200 µl of 0.2 M NaHCO₃ buffer/ 40 µl DMF. The reaction mixture was heated for 16 hours at 45°C. The product was isolated as per the method of Example 2(B)(1). Analytical HPLC indicated greater than 85% product formation. Work up of the reaction gave the oligonucleotide: wherein A* represents a nucleotide functionalized to incorporate a cholic acid functionality linked via a 2'-O-pentyl-amino linking group to the 2' position of the designated nucleotide. HPLC retention times are shown in Table 1 below.

### 2. Multiple Site Modification

A 10 O.D. unit (A₂₆₀) portion of Oligomer 13 (*see,* Example 1) was treated with cholic acid-NHS ester (Compound 1 in Application Serial Number 782,374, 10 mg, 19.8 µmols) in 300 µl of 0.2 M NaHCO₃ buffer/ 50 µl DMF. The reaction mixture was heated for 16 hours at 45°C. The product was isolated as per the method of Example 2(A)(1). Analytical HPLC revealed 58% doubly labeled product, 17% of a first singly labeled product and 24% of a second singly labeled product. Work up as per Example 2(A)(1) gave the oligonucleotide: wherein A* represents nucleotides functionalized to incorporate a cholic acid functionality linked via a 2'-O-pentyl-amino linking group to the 2' position of the designated nucleotide. HPLC retention times are shown in Table 1 below.

### D. Functionalization with Digoxigenin

### 1. Single Site Modification

A 10 O.D. unit (A₂₆₀) portion of Oligomer 12 (*see,* Example 1) was treated with digoxigenin-3-O-methylcarbonyl-ε-aminocaproic N-hydroxy succinimide ester (Boehringer Mannheim Corporation, Indianapolis, IN) in 200 µl of 0.1 M borate pH 8.3 buffer/ 40 µl DMF. The reaction mixture was let stand overnight. The product was isolated as per the method of Example 2(A)(1). Work up of the reaction gave the oligonucleotide: wherein A* represents a nucleotide functionalized to incorporate a digoxigenin functionality linked via a 2'-O-pentyl-amino linking group to the 2' position of the designated nucleotide. HPLC retention times are shown in Table 1 below.

### 2. Multiple Site Modification

A 10 O.D. units (A₂₆₀) portion of Oligomer 13 (see, Example 1) was treated with digoxigenin-3-O-methylcarbonyl-ε-aminocaproic N-hydroxy succinimide ester (Boehringer Mannheim Corporation, Indianapolis, IN) in 300 µl of 0.1 M borate pH 8.3 buffer/50 µl DMF. The reaction mixture was let stand overnight. The product was isolated as per the method of Example 2(A)(1). Work up as per Example 2(A)(1) gave the oligonucleotide: wherein A* represents nucleotides functionalized to incorporate a cholic acid functionality linked via a 2'-O-pentyl-amino linking group to the 2' position of the designated nucleotide. HPLC retention times are shown in Table 1 below.

**TABLE 1**

| **HPLC Retention Times Of Oligonucleotides Functionalized** **At 2' Position** | | | |
|---|---|---|---|
| Oligomer | Retention Time Minutes | | |
| | Mono Substitution | Multiple Substitution | |
| Oligomer 12¹ | 21.78 | | |
| Oligomer 13¹ | | 22.50 | |
| Oligomer 19² | 23.58 | | |
| Oligomer 20² | | 24.16^{a} | 25.19^{b} |
| Oligomer 21³ | 26.65 | | |
| Oligomer 22³ | | 26.99^{a} | 29.33^{b} |
| | | 27.55^{a} | |
| Oligomer 23⁴ | 30.10 | | |
| Oligomer 24⁴ | | 30.38^{a} | 37.00^{b} |
| | | 32.22^{a} | |
| Oligomer 25⁵ | 28.06 | | |
| Oligomer 26⁵ | | 28.14^{a} | 33.32^{b} |
| | | 29.24^{a} | |

| | | | |
|---|---|---|---|
| ^{a} Mono conjugated minor product; | | | |
| ^{b} Doubly conjugated major product; | | | |
| ¹ Parent Oligonucleotide - no 2' functionalization; | | | |
| ² 2' Biotin functionalization; | | | |
| ³ 2' Fluorescein functionalization; | | | |
| ⁴ 2' Cholic Acid functionalization; and | | | |
| ⁵ 2' Digoxigenin functionalization. | | | |

The conditions employed were as follows: Waters 600E with 991 detector, Hamilton PRP-1 column 0.7 x 15 cm; solvent A: 50 mM TEAA pH 7.0; B: 45 mM TEAA with 80% acetonitrile: 1.5 ml flow rate: Gradient: 5% B for first 5 minutes, linear (1%) increase in B every minute thereafter.

### PROCEDURE A

### Confirmation of Structure of Functionalized Oligonucleotides Containing A Tethered 2'-Amino Moiety

Oligonucleotides of the invention were digested with snake venom phosphodiesterase and calf-intestine alkaline phosphatase to their individual nucleosides. After digestion, the nucleoside composition was analyzed by HPLC. The HPLC analysis established that functionalized nucleotide compounds having the tethered 2'-amino moiety thereon were correctly incorporated into the oligonucleotide.

Snake venom phosphodiesterase [Boehringer-Mannheim cat. #108260, 1 mg (1.5 units)/0.5 ml] and alkaline phosphatase from calf intestine (1 unit/microliter, Boehringer-Mannheim cat. # 713023) in Tris-HCl buffer (pH 7.2, 50 mM) were used to digest the oligonucleotides to their component nucleosides. To 0.5 O.D. units of oligonucleotide in 50 µl buffer (nearly 40 µM final concentration for a 20 mer) was added 5 µl of snake venom phosphodiesterase (nearly 0.3 units/mL, final concentration) and 10 µl of alkaline phosphatase (app. 150 units/mL, final concentration). The reaction mixture was incubated at 37°C for 3 hours. Following incubation, the reaction mixture was analyzed by HPLC using a reverse phase analytical column (app. 30 x 2.5 cm); solvent A: 50 mM TEAA pH 7; solvent B: acetonitrile; gradient 100% for 10 minutes, then 5% B for 15 minutes, then 10% B and then wash. The results of these digestion are shown in Table 2 for representative oligonucleotides.

**TABLE 2**

| **OLIGONUCLEOTIDE ANALYSIS VIA ENZYMATIC DIGESTION** | | | | | |
|---|---|---|---|---|---|
| | Observed Ratios** | | | | |
| Oligomer | Abs. max. C | 267 G | 252 T | 267 A* | 260 A |
| Oligomer 10 | 2 | 1 | | 1 | |
| Oligomer 11 | 3 | 5 | 2 | 1 | |
| Oligomer 12 | 9 | 1 | 8 | 1 | 1 |
| Oligomer 13 | 9 | 1 | 8 | 2 | |

| | | | | | |
|---|---|---|---|---|---|
| * Nucleoside having 2'-O-linker attached thereto; and | | | | | |
| ** Corrected to whole numbers. | | | | | |

As is evident from Table 2, the correct nucleoside ratios are observed for the component nucleotides of the test oligonucleotides.

### PROCEDURE B

### Determination of Melting Temperatures (Tm's) of Cholic Acid Oligonucleotide Conjugates

The relative ability of oligonucleotides to bind to their complementary strand is compared by determining the melting temperature of the hybridization complex of the oligonucleotide and its complementary strand. The melting temperature (Tm), a characteristic physical property of double helices, denotes the temperature in degrees centigrade at which 50% helical versus coil (un-hybridized) forms are present. Tm is measured by using the UV spectrum to determine the formation and breakdown (melting) of hybridization. Base stacking, which occurs during hybridization, is accompanied by a reduction in UV absorption (hypochromicity). Consequently a reduction in UV absorption indicates a higher Tₘ. The higher the Tm, the greater the strength of the binding of the strands. Non-Watson-Crick base pairing has a strong destabilizing effect on the Tm. Consequently, absolute fidelity of base pairing is necessary to have optimal binding of an antisense oligonucleotide to its targeted RNA.

### 1. Terminal End Conjugates

### a. Synthesis

A series of oligonucleotides were synthesized utilizing standard synthetic procedures (for un-functionalized oligonucleotides) or the procedure of Example 3(A) in Application Serial Number 782,374, 1991, for oligonucleotides having a 5'-terminus amino linker bearing oligonucleotide or the procedure of Example 3(B) in Application Serial Number 782,374 for 5'-terminus cholic acid-bearing oligonucleotides. Each of the oligonucleotides had the following 5-LO antisense sequence: 5' TCC AGG TGT CCG CAT C 3'(SEQ ID NO:6). The nucleotides were synthesized on a 1.0 µmol scale. Oligomer 32 was the parent compound having normal phosphodiester internucleotide linkages. Oligomer 33 incorporated phosphorothioate inter-nucleotide linkages in the basic oligonucleotide sequence. Oligomer 34 is a an intermediate oligonucleotide having a 5'-aminolink at the 5'-terminus of the basic oligonucleotide sequence and Oligomer 35 was a similar 5'-aminolink compound incorporating phosphorothioate inter-nucleotide linkages. Oligomer 36 is a 5'-terminus cholic acid conjugate of the basic phosphodiester oligonucleotide sequence while Oligomer 37 is a similar 5'-cholic acid conjugate incorporating phosphorothioate inter-nucleotide linkages. Oligomers 32 and 33 were synthesized in a "Trityl-On" mode and were purified by HPLC. Oligomers 34 and 35 were synthesized as per Example 30(A) in Application Serial Number 782,374 without or with Beaucage reagent treatment, to yield phosphodiester or phosphorothioate inter-nucleotide linkages, respectively. Oligomers 36 and 37 were prepared from samples of Oligomers 34 and 35, respectively, utilizing a solution of cholic acid N-hydroxysuccinimide ester (Compound 1) 1 dissolved in DMF as per Example 3(B) in Application Serial Number 782,374. Oligomers 36 and 37 were purified by HPLC. The products were concentrated and desalted in a Sephadex G-25 column. Gel electrophoresis analyses also confirmed a pure product with the pure conjugate moving slower than the parent oligonucleotide or 5'-amino functionalized oligonucleotide.

### b. Melting Analysis

The test oligonucleotides [either the phosphodiester, phosphorothioate, cholic acid conjugated phosphodiester, cholic acid conjugated phosphorothioate or 5'-aminolink intermediate phosphodiester or phosphorothioate oligonucleotides of the invention or otherwise] and either the complementary DNA or RNA oligonucleotides were incubated at a standard concentration of 4 µM for each oligonucleotide in buffer (100 mM NaCl, 10 mM Na-phosphate, pH 7.0, 0.1 mM EDTA). Samples were heated to 90 degrees C and the initial absorbance taken using a Guilford Response II spectrophotometer (Corning). Samples were then slowly cooled to 15 degrees C and then the change in absorbance at 260 nm was monitored during the heat denaturation procedure. The temperature was elevated 1 degree/absorbance reading and the denaturation profile analyzed by taking the 1st derivative of the melting curve. Data was also analyzed using a two-state linear regression analysis to determine the Tm's. The results of these tests are shown in Table 3 as are the HPLC retention times of certain of the test compounds.

**TABLE 3**

| **Melting Temperature Of The Hybridization Complex Of** **The Oligonucleotide And Its Complementary Strand** | | | |
|---|---|---|---|
| Oligomer | Tm** | | HPLC Ret. Time* minutes |
| | DNA | RNA | |
| 32 | 62.6 | 62.0 | ---- |
| 33 | 55.4 | 54.9 | ---- |
| 34 | ND | ND | 13.6 |
| 35 | ND | ND | 17.0 |
| 36 | 63.4 | 62.4 | 22.0 |
| 37 | 56.3 | 55.8 | 22.5 |

| | | | |
|---|---|---|---|
| * HPLC conditions: Walters Delta Pak C-18 RP 2.5u column; at 0 min 100% 0.1 TEAA; at 30 min 50% TEAA and 50% Acetonitrile: Flow rate 1.0 ml/min. | | | |
| ** Tm at 4µM each strand from fit of duplicate melting curves to 2-state model with linear sloping base line. Conditions: 100 mM NaCl, 10 mM Phosphate, 0.1 mM EDTA, pH 7.0. ND = not determined | | | |

As is evident from Table 2, conjugates of cholic acid at the end of the oligonucleotide do not affect the Tm of the oligonucleotides.

### 2. Strands Incorporating 2'-O-Pentylamino Linker

### a. Synthesis

An oligonucleotide of the sequence: wherein A* represents a nucleotide functionalized to incorporate a pentylamino functionality at its 2'-position was synthesized in a one micromole scale utilizing the method of Example 1(B). The oligonucleotide was purified by reverse phase HPLC, detritylated and desalted on Sephadex G-25. PAGE gel analysis showed a single band. A further oligonucleotide, Oligomer 39, having the same sequence but without any 2'-O-amino linker was synthesis in a standard manner. A complementary DNA oligonucleotide of the sequence: was also synthesized in a standard manner as was a complementary RNA oligonucleotide of the sequence:

### b. Melting Analysis

Melting analysis was conducted as per the method of Procedure B(1)(b). The results are shown in Table 4.

**TABLE 4**

| **Melting Temperature Of The Hybridization Complex Of** **The Oligonucleotide And Its Complementary Strand** | | |
|---|---|---|
| Oligomer | Tm* | |
| | DNA¹ | RNA² |
| 38 | 54.5 | 58.0 |
| 39 | 60.6 | 56.9 |

| | | |
|---|---|---|
| * Tm at 4µM each strand from fit of duplicate melting curves to 2-state model with linear sloping base line. Conditions: 100 mM NaCl, 10 mM Phosphate, 0.1 mM EDTA, pH 7.0. | | |
| ¹ Against DNA complementary strand, Oligomer 40. | | |
| ² Against RNA complementary strand, Oligomer 41 | | |

As is evident from Table 4 against the RNA complementary strand the change in Tm's between the strand having 2'-amino linkers thereon and the unmodified strand is 1.1 degrees (0.22 change per modification). Against the DNA strand, the change is -6.1 degrees (-1.2 change per modification). When compared to the parent unmodified oligonucleotide the 2'-amino linker- containing strand has a stabilizing effect upon hybridization with RNA and a destabilizing effect upon hybridization with DNA.

Compounds of the invention were tested for their ability to increase cellular uptake. This was determined by judging either their ability to inhibit the expression of bovine papilloma virus-1 (BPV-1) or an assay involving luciferase production (for HIV-1).

### PROCEDURE C

### Determination of Cellular Uptake Judged By The Inhibition Of Expression of Bovine Papilloma Virus-1 (bpv-1) As Measured By an E2 Transactivation Assay

For this test, a phosphorothioate oligonucleotide analog of the sequence: was used as the basic sequence. This sequence is designed to be complementary to the translation initiation region of the E2 gene of bovine papilloma virus type 1 (BPV-1). Oligomer 42 served as the positive control and standard for the assay. Oligomer 3 (from Example 4 in Application Serial Number 782,374) served as a second test compound. It has the same basic sequence except it is a phosphorothioate oligonucleotide and further it has a cholic acid moiety conjugated at the 3'-end of the oligonucleotide. Oligomer 2 (from Example 2 in Application Serial Number 782,374) served as a third test compound. Again it is of the same sequence, it is a phosphorothioate oligonucleotide and it has a cholic acid moiety conjugated at the 5'-end. Oligomer 5 (from Example 5 in Application Serial Number 782,374) served as a fourth test compound. Once again it has the same sequence, is a phosphorothioate oligonucleotide and it has a cholic acid moiety conjugated at both the 3'-end and 5'-end. A fifth test compound was a phosphorothioate oligonucleotide with no significant sequence homology with BPV-1. A sixth test compound was a further phosphorothioate oligonucleotide with no significant sequence homology with BPV-1. The last test compound, the seventh test compound, was a phosphorothioate oligonucleotide with cholic acid conjugated to the 3'-end but having no significant sequence homology with BPV-1. Compounds five, six and seven served as negative controls for the assay.

For each test I-38 cells were plated at 5x10⁴ cells per cm² in 60 mm petri dishes. Eight hours after plating, medium was aspirated and replaced with medium containing the test oligonucleotide and incubated overnight. Following incubation, medium was aspirated and replaced with fresh medium without oligonucleotide and incubated for one hour. Cells were then transfected by the CaPO₄ method with 2 µg of pE2RE-1-CAT. After a four hour incubation period cells were glycerol shocked (15% glycerol) for 1 minute followed by washing 2 times with PBS. Medium was replaced with DMEM containing oligonucleotide at the original concentration. Cells were incubated for 48 hours and harvested. Cell lysates were analyzed for chloramphenicol acetyl transferase by standard procedures. Acetylated and nonacetylated ¹⁴C-chloramphenicol were separated by thin layer chromatography and quantitated by liquid scintillation. The results are expressed as percent acetylation.

Two lots of the positive control compound were found to acetylate at a level of 29% and 30%. The negative controls, test compounds five, six and seven, were found to acetylate at 59%, 58% and 47%, respectively. The 3'-cholic acid conjugate test compound, Oligomer 3, was found to acetylate to 23%, the 5'-cholic acid conjugate test compound, Oligomer 2, was found to acetylate to 36% and the test compound conjugated at both the 3'-end and the 5'-end, Oligomer 5, was found to acetylate to 27%.

The results of this test suggests that placement of a cholic acid moiety at the 3'-terminus of an oligonucleotide increase the activity. This in turn suggests that the increased activity was the result of increased cellular membrane transport.

### PROCEDURE D

### Determination of Cellular Uptake Judged By Inhibition of pHIVluc With Cholic Acid Linked 2'-O-Methyl Substituted Oligonucleotides

For this test the absence of an oligonucleotide in a test well served as the control. All oligonucleotides were tested as 2'-O-methyl analogs. For this test an oligonucleotide of the sequence: where each of the nucleotides of the oligonucleotide includes a 2'-O-methyl substituent group served as the basic test compound. The second test compound of the sequence: wherein CHA represents cholic acid and where each of the nucleotides of the oligonucleotide includes a 2'-O-methyl substituent group, was also of the same sequence as the first test compound. This second test compound included cholic acid conjugated to its 5'-end and was prepared as per the method of Example 3 in Application Serial Number 782,374 utilizing 2'-O-methyl phosphoramidite intermediates as identified in Example 1(C). The third test compound of the sequence: wherein CHA represents cholic acid and where each of the nucleotides of the oligonucleotide includes a 2'-O-methyl substituent group was also of the same sequence as the first test compound. The third test compound included cholic acid conjugated to its 3'-end and was prepared as per the method of Example 4 in Application Serial Number 782,374 utilizing 2'-O-methyl phosphoramidite intermediates as identified in Example 1(C). The fourth test compound was a 2'-O-Me oligonucleotide of a second sequence: where each of the nucleotides of the oligonucleotide includes a 2'-O-methyl substituent group. The fifth test compound was of sequence: wherein CHA represents cholic acid and where each of the nucleotides of the oligonucleotide includes a 2'-O-methyl substituent group. It was of the same sequence as the fifth test compound. This test compound included cholic acid conjugated to its 5'-end and was prepared as per the method of Example 3 in Application Serial Number 782,374 utilizing 2'-O-methyl phosphoramidite intermediates as identified in Example 1(C).

A sixth test compound was a randomized oligonucleotide of the sequence:

HeLa cells were seeded at 4x10⁵ cells per well in 6-well culture dishes. Test oligonucleotides were added to triplicate wells at 1 µM and allowed to incubate at 37°C for 20 hours. Medium and oligonucleotide were then removed, cells washed with PBS and the cells were CaPO₄ transfected. Briefly, 5µg of pHIVluc, a plasmid expressing the luciferase cDNA under the transcriptional control of the HIV LTR constructed by ligating the KpnI/HindIII restriction fragments of the plasmids pT3/T7luc and pHIVpap (NAR 19(12)) containing the luciferase cDNA and the HIV LTR respectively, and 6 µg of pcDEBtat, a plasmid expressing the HIV tat protein under the control of the SV40 promoter, were added to 500 µl of 250 mM CaCl₂, then 500 µl of 2x HBS was added followed by vortexing. After 30 minutes, the CaPO₄ precipitate was divided evenly between the six wells of the plate, which was then incubated for 4 hours. The media and precipitate were then removed, the cells washed with PBS, and fresh oligonucleotide and media were added. Incubation was continued overnight. Luciferase activity was determined for each well the following morning. Media was removed, then the cells washed 2X with PBS. The cells were then lysed on the plate with 200 µl of LB (1% Trit X-100, 25 mM Glycylglycine pH 7.8, 15 mM MgSO₄, 4 mM EGTA, 1mM DTT). A 75 µl aliquot from each well was then added to a well of a 96 well plate along with 75 µl of assay buffer (25 mM Glycylglycine pH 7.8, 15 mM MgSO₄, 4 mM EGTA, 15 mM KPO₄, 1 mM DTT, 2.5 mM ATP). The plate was then read in a Dynatec multiwell luminometer that injected 75 µl of Luciferin buffer (25 mM Glycylglycine pH 7.8, 15 mM MgSO₄, 4 mM EGTA, 4 mM DTT, 1 mM luciferin) into each well, immediately reading the light emitted (light units).

The random sequence compound (Oligomer 48) and the other non-cholic acid-conjugated test compounds (Oligomers 43 and 46) had comparable activity. The 5'-conjugate of the first sequence (Oligomer 44) also had activity comparable to the non-conjugated compounds. The 5'-conjugate of the second sequence (Oligomer 47) showed a three-fold increase in activity compared to the non-conjugated compounds and the 3'-conjugate of the first sequence (Oligomer 45) showed a further 3-fold increase in activity compared to Oligomer 47.

All the test cholic acid-bearing oligonucleotides showed significant inhibition of luciferase production compared to non-cholic acid- bearing oligonucleotides. This suggests that the increased activity was the result of increased cellular membrane transport of the cholic acid-bearing test oligonucleotides.

### EXAMPLE 3

### Preparation of 5'-O-[Dimethoxytrityl]-2'-O-[hexyl-(Ω-N-phthalimido)amino]uridine and 5'-O-[dimethoxytrityl]-3'-O-[hexyl(Ω-N-phthalimidoamino)uridine.

2',3'-*O*-Dibutyl stannylene-uridine was synthesized according to the procedure of Wagner *et. al.*, *J. Org. Chem*., **1974**, *39*, 24. This compound was dried over P₂O₅ under vacuum for 12 hours. To a solution of this compound (29 g, 42.1 mmols) in 200 ml of anhydrous DMF were added (16.8 g, 55 mmols) of 6-bromohexyl phthalimide and 4.5 g of sodium iodide and the mixture was heated at 130°C for 16 hours under argon. The reaction mixture was evaporated, co-evaporated once with toluene and the gummy tar residue was applied on a silica column (500 g). The column was washed with 2L of ethyl acetate (EtOAc) followed by eluting with 10% methanol (MeOH):90% EtOAc. The product, 2'- and 3'-isomers of O-hexyl-Ω-N-phthalimido uridine, eluted as an inseparable mixture (R_{f}=0.64 in 10% MeOH in EtOAc). By ¹³C NMR, the isomeric ration was about 55% of the 2' isomer and about 45% of the 3' isomer. The combined yield was 9.2 g (46.2%). This mixture was dried under vacuum and re-evaporated twice with pyridine. It was dissolved in 150 mL anhydrous pyridine and treated with 7.5 g of dimethyocytrityl chloride (22.13 mmols) and 500 mg of dimethylaminopyridine (DMAP). After 2 hour, thin layer chromatography (TLC; 6:4 EtOAc:Hexane) indicated complete disappearance of the starting material and a good separation between 2' and 3' isomers (R_{f}=0.29 for the 2' isomer and 0.12 for the 3' isomer). The reaction mixture was quenched by the addition of 5mL of CH₃OH and evaporated under reduced pressure. The residue was dissolved in 300mL CH₂Cl₂, washed successively with saturated NaHCO₃ followed by saturated NaCl solution. It was dried over Mg₂SO₄ and evaporated to give 15 g of a brown foam which was purified on a silica gel (500 g) to give 6.5 g of the 2'-isomer and 3.5 g of the 3' isomer.

### EXAMPLE 4

### Preparation of 5'-O-Dimethoxytrityl-2'-O-[hexyl-(Ω-N-phthalimido)amino]uridine-3'-O-(2-cyanoethyl-N,N-diisopropyl)-phosphoramidite.

The 5'-dimethoxytrityl-2'-[O-hexyl-(Ω-N-phthalimido)-amino]uridine (4 g, 5.2 mmole) was dissolved in 40 mL of anhydrous CH₂Cl₂. To this solution diisopropylaminetetrazolide (0.5 g, 2.9 mmol) was added and stirred overnight. TLC (1:1 EtoAC/hexane) showed complete disappearance of starting material. The reaction mixture was transferred with CH₂Cl₂ and washed with saturated NaHCO₃ (100 mL) followed by saturated NaCl solution. The organic layer was dried over anhydrous Na₂SO₄ and evaporated to yield 6.4 g of a crude product which was purified in a silica column (200 g) using 1:1 hexane/EtOAc to give 4.6 g (4.7 mmol, 90%) of the desired phosphoramidite.

### EXAMPLE 5

### Preparation of 5'-O-(Dimethoxytrityl)-3'-O-[hexyl-(Ω-N-phthalimido)amino]uridine-2'-O-succinyl-aminopropyl controlled pore glass.

Succinylated and capped aminopropyl controlled pore glass (CPG; 500Å pore diameter, aminopropyl CPG, 1.0 grams prepared according to Damha *et. al*., *Nucl. Acids Res.* **1990,** *18,* 3813.) was added to 12 ml anhydrous pyridine in a 100 ml round-bottom flask. 1-(3-Dimethylaminopropyl)-3-ethyl-carbodiimide (DEC; 0.38 grams, 2.0 mmol)], triethylamine (TEA; 100 µl, distilled over CaH₂), dimethylaminopyridine (DMAP; 0.012 grams, 0.1 mmol) and nucleoside 5'-O-dimethoxytrityl-3'-O-[hexyl-(Ω-N-phthalimidoamino)]uridine (0.6 grams, 0.77 mmol) were added under argon and the mixture shaken mechanically for 2 hours. More nucleoside (0.20 grams) was added and the mixture shaken an additional 24 hours. CPG was filtered off and washed successively with dichloromethane, triethylamine, and dichloromethane. The CPG was then dried under vacuum, suspended in 10 ml piperidine and shaken 15 minutes. The CPG was filtered off, washed thoroughly with dichloromethane and again dried under vacuum. The extent of loading (determined by spectrophotometric assay of dimethoxytrityl cation in 0.3 M p-toluenesulfonic acid at 498 nm) was approximately 28 µmol/g. The 5'-O-(dimethoxytrityl)-3'-O-[hexyl-(Ω-N-phthalimidoamino]uridine-2'-O-succinyl-aminopropyl controlled pore glass was used to synthesize the oligomers 5'-GACU*-3' and 5'-GCC TTT CGC GAC CCA ACA CU*-3' (SEQ ID NO:13) (where the * indicated the derivatized nucleotide) in an ABI 380B DNA synthesizer using phosphoramidite chemistry standard conditions. 45 and 200 O.D.'s of the 4-mer and 20-mer, respectively, were obtained from two and three 1 µmol syntheses after purification by RP-HPLC and desalting.

The oligomer 5'-GACU*-3' was used to confirm the structure of 3'-O-hexylamine tether introduced into the oligonucleotide by NMR. As expected a multiplet signal was observed between 1.0-1.8 ppm in ¹H NMR. The oligomer 5'-GCC TTT CGC GAC CCA ACA CU*- 3' belongs to a HCV sequence and it was used to show the nuclease resistance properties of the 3'-O-amino tether [see example 39].

### EXAMPLE 6

### Preparation of 5'-O-(Dimethoxytrityl)-2'-O-[hexyl-(Ω-N-phthalimido)amino] 3'-O-succinyl-aminopropyl controlled pore glass..

The procedure of Example 5 was repeated, except that 5'-O-(Dimethoxytrityl)-2'-O-[hexyl-(Ω-N-phthalimidoamido)-amino]uridine was used in the loading process.

### EXAMPLE 7

### Preparation of 5'-O-(Dimethoxytrityl)-2'-O-(hexylamino)-uridine.

5'-O-(dimethoxytrityl)-2'-O-[hexyl-(Ω-N-phthalimido amino)]uridine (4.5 grams, 5.8 mmol) was dissolved in 200 ml methanol in a 500 ml flask. Hydrazine (1 ml, 31 mmol) was added to the stirring reaction mixture. The mixture was heated to 60-65° in an oil bath and refluxed 14 hours. Solvent was evaporated *in vacuo*. The residue was dissolved in dichloromethane (250 ml) and extracted twice with an equal volume NH₄OH. The organic layer was evaporated to yield 4.36 grams of crude product, and NMR indicated that the product was not completely pure. R_{f}=0 in 100% ethyl acetate. The product was used in subsequent reactions without further purification.

### EXAMPLE 8

### Preparation of 5'-O-(dimethoxytrityl)-3'-O-[hexylamino] uridine.

The procedure of Example 7 was repeated, except that 5'-O-(dimethoxytrityl)-3'-O-[hexyl-(Ω-N-phthalimido-amino)] uridine was used as the starting material.

### EXAMPLE 9

### Preparation of 5'-O-(dimethoxytrityl)-2'-O-[hexyl-N-(1-pyrene propyl carbonyl)amino]uridine

5'-O-Dimethoxytrityl-2'-O-(hexylamino)uridine (0.5g, 0.78 mmol) was dissolved in anhydrous DMF (15 mL). 1-Hydroxybenzotriazole (0.16 grams, 1.17 mmol) and 1-pyrene-butyric acid pentafluorophenyl ester (0.53 grams, 1.17 mmol) were added to the reaction mixture. The mixture was stirred under argon at room temperature for 2 hours, after which it was concentrated *in vacuo.* Residual DMF was coevaporated with toluene. The residue was dissolved in dichloromethane (50 mL) and washed with an equal volume saturated NaHCO₃. The aqueous layer was washed with dichloromethane and the combined organic extracts washed with an equal volume saturated NaCl. The aqueous layer was washed with dichloromethane and the combined organic layers dried over MgSO₄ and concentrated. The residue was chromatographed on a silica gel column, eluting with a gradient of 50% ethyl acetate in hexanes to 100% ethyl acetate. The desired product (0.83 grams, 58%) eluted with 100% ethyl acetate (R_{f} 0.46 by thin-layer chromatography (TLC)).

### EXAMPLE 10

### Preparation of 5'-O-[Dimethoxytrityl]-2'-O-[hexyl-N-(1-pyrene propyl carbonyl)amino]uridine-3'-O-(2-cyanoethyl-N, N-diisopropyl)phosphoramidite

5'-O-[Dimethoxytrityl]-2'-O-[hexyl-N-(1-pyrene propyl carbonyl)amino] uridine (0.80 grams, 0.87 mmol) was dissolved in 20 mL dry dichloromethane. 2-Cyanoethyl *N,N,N',N'*-tetraisopropylphosphorodiamidite (purchased from Sigma Chemical Co; 800 µL, 2.4 mmol) and diisopropylamine tetrazolide (0.090 grams, 0.52 mmol) were added to the mixture, which was stirred under argon for 20 hours The reaction mixture was then concentrated in vacuo and the residue dissolved in dichloromethane (75 mL). The solution was washed with an equal volume of saturated NaHCO₃. The aqueous layer was washed with dichloromethane (20 mL) and the combined organic layers washed with an equal volume of saturated NaCl. The aqueous layer was washed with dichloromethane (20 mL) and the combined organic layers dried over MgSO₄ and concentrated. The residue was chromatographed on a silica gel column, eluting with a gradient of 50% ethyl acetate in hexanes to 100% ethyl acetate. The desired product (750 mg, 78% yield, R_{f} 0.54 by TLC in 100% ethyl acetate) eluted with 100% ethyl acetate.

### EXAMPLE 11

### Preparation of 2'-O-[hexyl-N-(1-pyrene-propyl-carbonyl) amino] uridine.

5'-O-dimethoxytrityl-2'-O-[hexyl-N-(1-pyrene-propylcarbonyl)amino]uridine (1.0 g) was dissolved in 20 mL CH₂Cl₂ and kept in ice-bath for 10 minutes. To the cold solution, 5 mL of 80% acetic acid in water was added and the solution was left to stand for 30 minutes. It was then evaporated to dryness and loaded into a silica column and eluted with 10% methanol in methylene chloride to give 2'-O-[hexyl-N-(1-pyrene-propyl-carbonyl)amino]uridine.

### EXAMPLE 12

### Preparation of 5'-O-(dimethoxytrityl)-2'-O-[hexyl-N-(1-pyrene propylcarbonyl)amino]uridine-3'-O-[succinylaminopropyl]-controlled pore glass.

Succinylated/capped aminopropyl controlled pore glass was dried under vacuum for 3 hours immediately before use. A portion (0.3 g) was added to 3 ml anhydrous pyridine in a 50 ml round-bottom flask. DEC (0.12 grams, 0.63 mmol), TEA (25 µl, distilled over CaH₂), DMAP (0.005 grams, mmol) and 5'-O-(dimethoxytrityl)-3'-O-[hexyl-N-(1-pyrene propyl carbonyl] amino]uridine (0.21 grams, 0.22 mmol) were added under argon and the mixture shaken mechanically for 19 hours. More nucleoside (0.025 grams) was added and the mixture shaken an additional 5.5 hours. Pentachlorophenol (0.045 grams, mmol) was added and the mixture shaken 18 hours. CPG was filtered off and washed successively with dichloromethane, triethylamine, and dichloromethane. The resulting CPG was then dried under vacuum, suspended in 15 ml piperidine and shaken 30 minutes. CPG was filtered off, washed thoroughly with dichloromethane and again dried under vacuum. The extent of loading (determined by spectrophotometric assay of dimethoxytrityl cation in 0.3 M p-toluenesulfonic acid at 498 nm) was approximately 27 µmol/g. The product solid support was subsequently used to synthesize the oligomers.

### EXAMPLE 13

### Preparation of 5'-O-dimethoxytrityl-3'-O-[hexyl-N-(1-pyrene propyl carbonyl] amino] uridine-2'-O-(succinyl amino propyl) controlled pore glass.

The procedure of Example 12 is repeated, except that 5'-O-dimethoxytrityl-3'-O-[hexyl-N-(1-pyrene propyl carbonyl] amino] uridine is used.

### EXAMPLE 14

### Preparation of 5'-O-(dimethoxytrityl)-2'-O-[hexyl-N-(5-thio carbonyl-3,6-dipivolyl-fluorescein)amino]uridine.

Fluorescein isothiocyanate (Isomer I, available from Cal Biochem, La Jolla, CA) was treated with 12 equivalents of pivaloyl chloride in Et₃N/THF to give di-O-pivaloyl fluorescein isothiocyanate. This compound was purified in silica gel column using 3:1 hexane:ethyl acetate. Nucleoside. 5'-O-(dimethoxytrityl)-2'-O-(hexylamino)uridine was then condensed with dipivolyl fluorescein isothiocyanate in CH₂Cl₂/pyrimidine. The resultant compound 5'-O-(dimethoxytrityl)-2'-O-[hexyl-N-(5-thiocarbonyl-3,6-dipivolylfluorescein)amino]uridine is then purified by using 100% ethyl acetate, in a silica column.

### EXAMPLE 15

### Preparation of 5'-O-dimethoxytrityl-2'-O-[hexyl-N-(5-thiocarbonyl-3,6-di-pivaloyl fluorescein)amino]uridine-3'-O-(2-cyanoethyl, N-N-diisopropyl phosphoramidite.

5'-O-(dimethoxytrityl)-2'-O-[hexyl-N-(5-thiocarbonyl-3,6-dipivolyl fluorescein)amino]uridine (0.75 grams, 0.672 mmol) was dissolved in dry dichloromethane (20 mL). 2-Cyanoethyl *N,N,N',N'*-tetraisopropylphosphorodiamidite (700 µL, 2.2 mmol) and diisopropylamine tetrazolide were added to the mixture, which was stirred under argon for 16 hours. The reaction mixture was then concentrated *in vacuo* and the residue dissolved in dichloromethane (75 mL) followed by washing with an equal volume of saturated NaHCO₃. The aqueous layer was washed with dichloromethane (50 mL) and the combined organic layers washed with an equal volume of saturated NaCl. The aqueous layer was washed with dichloromethane (50 mL) and the combined organic layers dried over MgSO₄ and concentrated. The residue was chromatographed on a silica gel column, eluting with a gradient of 25% ethyl acetate in hexanes to 100% ethyl acetate. The desired product (670 mg, 77% yield, R_{f} 0.79 by TLC) eluted with 100% ethyl acetate.

### EXAMPLE 16

### Preparation of 5'-O-(dimethoxytrityl)-2'-O-[hexyl-N-(5-thiocarbonyl-3,6-di-pivaloyl fluorescein)amino]uridine-3'-O-(succinylaminopropyl) controlled pore glass.

Succinylated and capped aminopropyl controlled pore glass (CPG) is dried under vacuum for 3 hours immediately before use. CPG (0.3 grams) is added to 3 ml anhydrous pyridine in a 50 ml round-bottom flask. DEC (0.12 grams, 0.63 mmol), TEA (25 µl, distilled over CaH₂, DMAP (dimethyl amino pyridine) (0.005 grams, 0.04 mmol) and 5'-O-dimethoxytrityl-2'-O-[hexyl-N-(5-thiocarbonyl-3,6-di-pivaloyl fluorescein) amino] uridine (0.21 grams, 0.19 mmol) are added under argon and the mixture shaken mechanically for 19 hours. More nucleoside (0.025 grams) is added and the mixture shaken an additional 5.5 hours. Pentachlorophenol (0.045 grams, 0.17 mmol) is added and the mixture shaken 18 hours. CPG is filtered off and washed successively with dichloromethane, triethylamine, and dichloromethane. CPG then is dried under vacuum, suspended in 15 mL piperidine and shaken 30 minutes. CPG is filtered off, washed thoroughly with dichloromethane, and again dried under vacuum. The extent of loading is then determined by spectrophotometric assay of dimethoxytrityl cation in 0.3 M p-toluenesulfonic acid at 498 nm.

### EXAMPLE 17

### Preparation of 5'-O-(dimethoxytrityl)-2'-O-[hexyl-N-(3-oxycarbonyl-cholesteryl)amino]uridine.

Nucleoside 5'-O-(dimethoxytrityl)-2'-O-[hexylamino]-uridine (3.85 g, 6.0 mmol) was dissolved in anhydrous pyridine/dichloromethane 50/50 (v/v) (20 mL). Cholesteryl chloroformate (Fluka, 3.0 g, 6.68 mmol) was dissolved in anhydrous dichloromethane (20 ml) and added slowly under argon with a syringe to the stirring reaction mixture. The mixture was stirred under argon at room temperature for 2 h after which it was concentrated *in vacuo*. Residual DMF was coevaporated with toluene. The residue was dissolved in dichloromethane (50 mL) and washed with an equal volume saturated NaHCO₃. The aqueous layer was washed with dichloromethane and the combined organic extracts washed with an equal volume saturated NaCl. The aqueous layer was washed with dichloromethane and the combined organic layers dried over MgSO₄ and concentrated. The residue was chromatographed on a silica gel column with a gradient of 25% ethyl acetate in hexanes to 100% ethyl acetate. The desired product (3.78 g, 60%) eluted with 100% ethyl acetate (R_{f} 0.41 by TLC).

### EXAMPLE 18

### Preparation of 5'-O-(dimethoxytrityl)-2'-O-[hexyl-N-(3-oxycarbonyl-cholesteryl)amino]uridine-3'-O-[2-cyanoethyl-N,N-diisopropyl]phosphoramidite

Nucleoside 5'-O-(dimethoxytrityl)-2'-O-[hexyl-N-(3-oxycarbonyl-cholesteryl)amino]uridine (3.44 g, 3.3 mmol) was dissolved in dry dichloromethane (75 mL). 2-cyanoethyl *N,N,N'N'*-tetraisopropylphosphorodiamidite (Sigma, 2.1 ml, 6.6 mmol) and diisopropylamine tetrazolide (0.29 g, 1.7 mmol) were added to the mixture, which was stirred under argon for 16 H. Dichloromethane (75 mL) was added to the solution, which was washed with an equal volume of saturated NaHCO₃. The aqueous layer was washed with an equal volume of dichloromethane. The aqueous layer was washed with dichloromethane (30 ml) and the combined organic layers washed with an equal volume of saturated NaCl. The aqueous layer was washed with dichloromethane (30 mL) and the combined organic layers dried over Mg₂SO₄ and concentrated *in vacuo*. The residue was chromatographed on a silica gel column with a gradient of 25% ethyl acetate in hexanes to 70% ethyl acetate. The desired product (3.35 g, 82% yield, R_{f}=0.71 by TLC in 50% ethyl acetate in hexanes) eluted with 50% ethyl acetate.

### EXAMPLE 19

### Preparation of 5'-O-(dimethoxytrityl)-2'-O-[hexyl-N-(3-oxycarbonyl-cholesteryl)amino]uridine-3'-O-(succinyl aminopropyl)-controlled pore glass.

Succinylated and capped controlled pore glass (0.3 grams) is added to 2.5 ml anhydrous pyridine in a 15 ml pear-shaped flask. DEC (0.07 grams, 0.36 mmol), TEA (100 µl, distilled over CaH₂), DMAP (0.002 grams, 0.016 mmol) and 5'-O-(dimethoxytrityl)-2'-O-[hexyl-N-(3-oxycarbonyl-cholesteryl)-amino]uridine (0.25 grams, 0.23 mmol) are added under argon and the mixture shaken mechanically for 16 hours. More nucleoside (0.20 grams) is added and the mixture shaken an additional 18 hours. Pentachlorophenol (0.03 grams, 0.11 mmol) is added and the mixture shaken 9 hours. CPG is filtered off and washed successively with dichloromethane, triethylamine, and dichloromethane. CPG is then dried under vacuum, suspended in 10 ml piperidine and shaken 15 minutes. CPG is filtered off, washed thoroughly with dichloromethane and again dried under vacuum. The extent of loading is determined by spectrophotometric assay of dimethoxytrityl cation in 0.3 M p-toluenesulfonic acid at 498 nm as approximately 39 µmol/g.

### EXAMPLE 20

### Preparation of 5'-O-(dimethoxytrityl)-2'-O-[hexyl-N-(2,4-dinitrophenyl)amino]uridine.

5'-O-(dimethoxytrityl)-2'-O-(hexylamino)uridine (0.88 grams, 1.37 mmol) was dissolved in methanol (20 mL). 2,4-Dinitrofluorobenzene (DNFB, 0.25 grams, 1.37 mmol) was added and the mixture shaken on a mechanical shaker. The reaction was monitored by TLC. After 90 min, another 0.25 grams of DNFB was added and the reaction mixture shaken an additional 30 min, followed by addition of another 0.25 grams of DNFB. After shaking 2.5 hours, the mixture was concentrated *in vacuo* and chromatographed on a silica gel column, eluting with a gradient of 25% ethyl acetate in hexanes to 100% ethyl acetate. The desired product (0.51 grams, 46%) eluted with 100% ethyl acetate (R_{f} 0.85 by TLC).

### EXAMPLE 21

### Preparation of 5'-O-(dimethoxytrityl)-2'-O-(hexyl-N-(2,4-dinitrophenyl)amino]uridine-3'-O-(2-cyanoethyl-N,N-diisopropyl)phosphoramidite.

5'-O-(dimethoxytrityl)-2'-O-[hexyl-N-(2,4-dinitrophenyl)amino]uridine (0.45 grams, 0.55 mmol) was dissolved in dry dichloromethane (12 mL). 2-Cyanoethyl *N,N,N',N'-*tetraisopropylphosphorodiamidite (380 µL, 1.2 mmol) and diisopropylamine tetrazolide (0.041 grams, 0.024 mmol) were added to the mixture, which was stirred under argon for 16 hours. The reaction mixture was then concentrated *in vacuo* and the residue dissolved in dichloromethane (75 mL) followed by washing with an equal volume of saturated NaHCO₃. The aqueous layer was washed with dichloromethane (25 mL) and the combined organic layers washed with an equal volume of saturated NaCl. The aqueous layer was washed with dichloromethane (25 mL) and the combined organic layers dried over MgSO₄ and concentrated. The residue was chromatographed on a silica gel column, eluting with a gradient of 20% ethyl acetate in hexanes to 100% ethyl acetate. The desired product (510 mg foam, 93% yield, R_{f} 0.70 by TLC) eluted with 100% ethyl acetate. ³¹PNMR (CDCl₃): 150.56 and 150.82 ppm.

### EXAMPLE 22

### Preparation of 5'-O-(dimethoxytrityl) -2'-O-[hexyl-N- (2,4-dinitrophenyl)amino]uridine-3'-O-(succinyl aminopropyl) controlled pore glass.

Succinylated and capped controlled pore glass (0.3 grams) is added to 3 ml anhydrous pyridine in a 50 ml round-bottom flask. DEC (0.12 grams, mmol), TEA (25 µl, distilled over CaH₂), DMAP (0.005 grams, 0.041 mmol) and 5'-O-(dimethoxytrityl)-2'-O-[hexyl-N-(2,4-dinitrophenyl)amino]uridine (0.21 grams, 0.26 mmol) are added under argon and the mixture shaken mechanically for 19 hours. More nucleoside (0.025 grams) is added and the mixture shaken an additional 5.5 hours. Pentachlorophenol (0.045 grams, 0.16 mmol) is added and the mixture shaken 18 hours. CPG is filtered off and washed successively with dichloromethane, triethylamine, and dichloromethane. CPG then is dried under vacuum, suspended in 15 ml piperidine and shaken for 15 minutes. CPG is filtered off, washed thoroughly with dichloromethane, and again dried under vacuum. The extent of loading is determined by spectrophotometric assay of dimethoxytrityl cation in 0.3 M p-toluenesulfonic acid at 498 nm, as approximately 29 µmol/gm.

### EXAMPLE 23

### Preparation of 5'-O-(dimethoxytrityl)-2'-O-[hexyl-N-(Nα-Nimid-Di-FMOC-L-Histidyl)amino]uridine.

Nucleoside 5'-O-(dimethoxytrityl)-2'-O-(hexylamino)-uridine (0.97 g, 1.51 mmol) was dissolved in dichloromethane (25 mL) and cooled to 0°C in an ice bath. Nα,Nimid-Di-FMOC-L-histine pentafluorophenyl ester (2.4 g, 3.1 mmol, purchased from Sigma) and 1-hydroxybenzotriazole (0.32 g, 0.24 mmol, purchased from Fluka) were added to the stirred reaction mixture stirred under argon. After 15 minutes, the ice bath was removed and the mixture stirred under argon at room temperature for 72 h. The mixture was concentrated *in vacuo* and chromatographed on a silica gel column, eluting with a gradient of 50% ethyl acetate in hexanes to 70% ethyl acetate in hexanes. The desired product (0.53 g, 28%) eluted with 70% ethyl acetate (R_{f} 0.53 by TLC in 100% ethyl acetate).

### EXAMPLE 24

### Preparation of 5'-O-(dimethoxytrityl)-2'-O-[hexyl-N-(Nα-Nimid-Di-FMOC-L-histidyl)-amino]-uridine-3'-O-[2-cyanoethyl-N,N-diisopropyl]phosphoramidite.

5'-O-Dimethoxytrityl-2'-O-[hexyl-N-(Nα-Nimid-Di-FMOC-L-histidyl)amino]uridine (1.9 g, 1.6 mmol) is dissolved in dry dichloromethane (20mL). 2-Cyanoethyl *N,N,N',N'*-tetraisopropylphosphorodiamidite (800 µL, 2.4 mmol) and diisopropylamine tetrazolide (0.090 grams, 0.52 mmol) are added to the mixture, which is stirred under argon for 20 hours. The reaction mixture then is concentrated *in vacuo* and the residue dissolved in dichloromethane (75 mL). The solution is washed with an equal volume of saturated NaHCO₃. The aqueous layer is washed with dichloromethane (20 mL) and the combined organic layers washed with an equal volume of saturated NaCl. The aqueous layer is washed with dichloromethane (20 mL) and the combined organic layers dried over MgSO₄ and concentrated. The residue is chromatographed on a silica gel column, eluting with a gradient of 50% ethyl acetate in hexanes to 100% ethyl acetate. The desired product elutes with 100% ethyl acetate.

### EXAMPLE 25

### Preparation of 5'-O-(dimethoxytrityl)-2'-O-[hexyl-N-(Nα-Nimid-Di-FMOC)-L-histidyl)amino]uridine-3'-0-[succinylaminopropyl] controlled pore glass.

Succinylated and capped controlled pore glass (dried under vacuum for 3 hours immediately before use; 0.3 grams) is added to 3 ml anhydrous pyridine in a 50 ml round-bottom flask. DEC (0.12 grams, 0.63 mmol), TEA (25 µl, distilled over CaH₂), DMAP (0.005 grams, 0.04 mmol) and 5'-O-(dimethoxytrityl)-2'-O-[hexyl-N-(Nα-Nimid-Di-FMOC)-L-histidyl)amino]-uridine (0.21 grams, 0.17 mmol) are added under argon and the mixture shaken mechanically for 19 hours. More nucleoside (0.025 grams) is added and the mixture shaken an additional 5.5 hours. Pentachlorophenol (0.045 grams, 0.17 mmol) is added and the mixture shaken 18 hours. CPG is filtered off and washed successively with dichloromethane, triethylamine, and dichloromethane. CPG then is dried under vacuum, suspended in 15 ml piperidine and shaken 15 minutes. CPG is filtered off, washed thoroughly with dichloromethane and again dried under vacuum. The extent of loading is determined by spectrophotometric assay of dimethoxytrityl cation in 0.3 M p-toluenesulfonic acid at 498 nm. to be approximately 27 µmol/g.

### EXAMPLE 26

### Preparation of 5'-O-(dimethoxytrityl)-2'-O-[hexyl-N-(Ω-methylpolyethylene glycol-propionoyl)amino]uridine.

Nucleoside 5'-O-(dimethoxytrityl)-2'-O-[hexylamino]-uridine, (1 g, 1.55 mmol) is dissolved in anhydrous DMF (15 mL). 1-Hydroxybenzotriazole (0.24 g, 1.75 mmol) and polyethylene glycol-propionic acid-NHS-ester (1.23 g, 1.75 mmol) are added to the reaction mixture. The mixture is stirred under argon at room temperature for 2 hours after which it is concentrated *in vacuo*. Residual DMF is coevaporated with toluene. The residue is dissolved in dichloromethane (50 mL) and then washed with an equal volume saturated NaHCO₃. The aqueous layer is washed with dichloromethane and the combined organic extracts washed with an equal volume saturated NaCl. The aqueous layer is washed with dichloromethane and the combined organic layers dried over MgSO₄ and concentrated. The residue is chromatographed on a silica gel column, eluting with a gradient of 50% ethyl acetate in hexanes to 100% ethyl acetate. The desired product (1.08 g, 58%) eluted with 100% ethyl acetate.

### EXAMPLE 27

### Preparation of 5'-O-(dimethoxytrityl)-2'-O-[hexyl-N-(Ω-methylpolyethylene glycol-propionoyl)amino]uridine-3'-O-(2-cyanoethoxy-N,N-diisopropyl)phosphoramidite.

5'-O-(Dimethoxytrityl)-2'-O-[hexyl-N-(Ω-methylpolyethylene glycol-propionoyl)amino]uridine (1.04 grams, 0.87 mmol) is dissolved in dry dichloromethane (20mL). 2-Cyanoethyl *N,N,N',N'*-tetraisopropylphosphorodiamidite (800 µL, 2.4 mmol) and diisopropylamine tetrazolide (0.090 grams, 0.52 mmol) are added to the mixture, which is stirred under argon for 20 hours. The reaction mixture then is concentrated *in vacuo* and the residue dissolved in dichloromethane (75 mL). The solution is washed with an equal volume of saturated NaHCO₃. The aqueous layer is washed with dichloromethane (20 mL) and the combined organic layers washed with an equal volume of saturated NaCl. The aqueous layer is washed with dichloromethane (20 mL) and the combined organic layers dried over MgSO₄ and concentrated. The residue is chromatographed on a silica gel column, eluting with a gradient of 50% ethyl acetate in hexanes to 100% ethyl acetate. The desired product elutes with 100% ethyl acetate.

### EXAMPLE 28

### Preparation of 5'-O-(dimethoxytrityl)-2'-O-[hexyl-N-(Ω-methylpolyethylene glycol-propionoyl)amino]uridine-3'-O-(succinylaminopropyl) controlled pore glass.

Succinylated and capped controlled pore glass (CPG) is dried under vacuum for 3 hours immediately before use. Controlled pore glass (0.3 grams) is added to 3 ml anhydrous pyridine in a 50 ml round-bottom flask. DEC (0.12 grams, 0.67 mmol), TEA (25 µl, distilled over CaH₂), DMAP (0.005 grams, mmol) and 5'-O-(dimethoxytrityl)-2'-O-[hexyl-N-(w-methylpolyethylene glycol-propionoyl]amino]uridine (0.21 grams, 0.175 mmol) are added under argon and the mixture shaken mechanically for 19 hours. More nucleoside (0.025 grams) is added and the mixture shaken an additional 5.5 hours. Pentachlorophenol (0.045 grams, 0.17 mmol) is added and the mixture shaken 18 hours. CPG is filtered off and washed successively with dichloromethane, triethylamine, and dichloromethane. CPG then is dried under vacuum, suspended in 15 ml piperidine, and shaken 15 minutes. CPG is filtered off, washed thoroughly with dichloromethane, and again dried under vacuum. The extent of loading is determined by spectrophotometric assay of dimethoxytrityl cation in 0.3 M p-toluenesulfonic acid at 498 nm. to be approximately 18 µmol/g.

### EXAMPLE 29

### Preparation of Macrocycle derivatized nucleoside

5'-O-(dimethoxytrityl)-2'-O-(hexylamine)uridine is treated as per the procedure of Example 3 with the macrocycle 4-{1,4,8,11-tetraza-[tri-(trifluoroacetyl)cyclotetradec-1-yl]}methyl benzoic acid-N-hydroxy succinimide ester (prepared according to Simon Jones, *et. al., Bioconjugate Chem.* **1991,** *2*, 416) to yield the product.

### EXAMPLE 30

### Preparation of macrocycle derivatized uridine phosphoramidite

The nucleoside product of Example 29 is treated as per the procedure of Example 4 to yield the product.

### EXAMPLE 31

### Preparation of CPG derivatized with macrocycle derivatized nucleoside

The nucleoside product of Example 29 is treated as per the procedure of Example 5 to yield the product.

### EXAMPLE 32

### Preparation of 5'-O-(dimethoxyltrityl)-2'-O-(hexyl-N-(folate)-amino)uridine

5'-O-(Dimethoxytrityl)-2'-O-(hexylamine)uridine is treated as per the procedure of Example 3 with folic acid pentafluorophenyl ester (protected with an isobutyryl protecting group) to yield the product.

### EXAMPLE 33

### Preparation of 5'-O-(dimethoxyltrityl)-2'-O-[hexyl-N-(folate)-amino]uridine-3'-O-(2-cyanoethoxy-N,N-diisopropyl)phosphoramidite

The nucleoside product of Example 29 is treated as per the procedure of Example 4 to yield the product.

### EXAMPLE 34

### Preparation of CPG derivatized with 5'-O-(dimethoxyltrityl)-2'-O-(hexyl-N-(folate)amino)uridine nucleoside

The nucleoside product of Example 32 is treated as per the procedure of Example 5 to yield the product.

### EXAMPLE 35

### Preparation of 5'-O-(dimethoxytrityl)-2'-O-{hexyl-N-[2-methoxy-6-chloro-9(Ω-amino-caproyl)acridine]amino}uridine.

6,9-Dichloro-2-methoxyacridine (Aldrich, 10 g, 36 mmol) and phenol (2.5 g) were placed together on a round-bottom flask with a stirring bar and to this 6-amino-hexanoic acid (9.3 g, 71 mmol) was added and the flask was heated to 100° (oil bath) for 2 hours. TLC (10% methanol in methylene chloride) showed complete disappearance of starting material. The reaction mixture was cooled and poured into 200 mL of methanol. The product isolates out as a yellow solid (about 10 g). This compound was then converted into its pentafluorophenyl ester.

5'-O-(Dimethoxytrityl)-2'-(hexylamino)uridine (0.5g, 0.78 mmol) is dissolved in anhydrous DMF (15 mL). 1-Hydroxybenzotriazole (0.16 grams, 1.17 mmol) and 2-methoxy-6-chloro-9-(Ω-caproyl-amino) acridine pentafluorophenyl ester (0.53 grams, 1.17 mmol) are added to the reaction mixture. The mixture is stirred under argon at room temperature for 2 h, after which it is concentrated *in vacuo.* Residual DMF is coevaporated with toluene. The residue is dissolved in dichloromethane (50 mL) and washed with an equal volume saturated NaHCO₃. The aqueous layer is washed with dichloromethane and the combined organic extracts washed with an equal volume saturated NaCl. The aqueous layer is washed with dichloromethane and the combined organic layers dried over MgSO₄ and concentrated. The residue is chromatographed on a silica gel column, eluting with a gradient of 50% ethyl acetate in hexanes to 100% ethyl acetate. The desired product elutes with 100% ethyl acetate.

### EXAMPLE 36

### Preparation of 5'-O-(dimethoxytrityl)-2'-O-{hexyl-N-[2-methoxy-6-chloro-9-(Ω-amino-caproyl)acridine]amino}uridine-3'-O-(2-cyanoethyl-N-N-diisopropyl) phosphoramidite.

5'-O-Dimethoxytrityl-2'-O-{hexyl-N-[2-methoxy-6-chloro-9-(w-amino-caproyl)acridine]amino}uridine (0.80 grams, 0.77 mmol) is dissolved in dry dichloromethane (20mL). 2-Cyanoethyl *N,N,N',N'*-tetraisopropylphosphorodiamidite (800 µL, 2.4 mmol) and diisopropylamine tetrazolide (0.090 grams, 0.52 mmol) are added to the mixture, which is stirred under argon for 20 hours. The reaction mixture is then concentrated *in vacuo* and the residue dissolved in dichloromethane (75 mL). The solution is washed with an equal volume of saturated NaHCO₃. The aqueous layer is washed with dichloromethane (20 mL) and the combined organic layers washed with an equal volume of saturated NaCl. The aqueous layer is washed with dichloromethane (20 mL) and the combined organic layers dried over MgSO₄ and concentrated. The residue is chromatographed on a silica gel column, eluting with a gradient of 50% ethyl acetate in hexanes to 92% ethyl acetate. The desired product elutes with 100% ethyl acetate.

### EXAMPLE 37

### Preparation of 5'-O-(dimethoxytrityl)-2'-O-{hexyl-N-[2-methoxy-6-chloro-9-(Ω-aminocaproyl)acridine]amino}uridine-3'-O-(succinyl aminopropyl) controlled pore glass.

Succinylated and capped controlled pore glass (0.3 grams) is added to 3 ml anhydrous pyridine in a 50 ml round-bottom flask. DEC (0.12 grams, 0.67 mmol), TEA (25 µl, distilled over CaH₂), DMAP (0.005 grams, 0.04 mmol) and 5'-O-dimethoxytrityl-2'-O-{hexyl-N-[2-methoxy-6-chloro-9-(Ω-aminocaproyl)acridine]amino}uridine (0.21 grams, 0.17 mmol) are added under argon and the mixture shaken mechanically for 19 hours. More nucleoside (0.025 grams) is added and the mixture shaken an additional 5.5 hours. Pentachlorophenol (0.045 grams, 0.17 mmol) is added and the mixture shaken 18 hours. CPG is filtered off and washed successively with dichloromethane, triethylamine, and dichloromethane. CPG is then dried under vacuum, suspended in 15 ml piperidine and shaken 15 minutes. CPG is filtered off, washed thoroughly with dichloromethane and again dried under vacuum. The extent of loading is determined by spectrophotometric assay of dimethoxytrityl cation in 0.3 M p-toluenesulfonic acid at 498 nm. to be approximately 27 µmol/g.

### EXAMPLE 38

### Preparation of 5'-O-(dimethoxytrityl)-2'-O-[(hexyl-N,N-dimethyl)amino]uridine.

5'-O-(dimethoxytrityl)-2'-O-(hexylamino)uridine (0.19 grams, 0.29 mmol) is dissolved in 4 ml methanol. Sodium acetate pH 4.0 (2 ml), sodium cyanoborohydride (0.02 grams, 0.3 mmol) and 37% formaldehyde in water (300 µl) are added to the reaction mixture, which is stirred 2 hours, after which it is concentrated *in vacuo*. The residue is dissolved in dichloromethane (50 mL) and washed with an equal volume saturated NaHCO₃. The aqueous layer is washed with dichloromethane and the combined organic extracts washed with an equal volume saturated NaCl. The aqueous layer is washed with dichloromethane and the combined organic layers dried over MgSO₄ and concentrated. The residue is chromatographed on a silica gel column, eluting with a gradient of 50% ethyl acetate in hexanes to 100% ethyl acetate. The desired product (0.15 grams, 80%) elutes with 10% Methanol-90% ethyl acetate.

### EXAMPLE 39

### Oligonucleotides Having 3'-Alkylamino Group

3'-O-Hexyl-(N-phthalimido)-aminouridine-CPG, *i.e*. the 5'-O-dimethoxytrityl-3'-O-[hexyl-(Ω-N-phthalimido amino)]-uridine-2'-O-(succinyl-aminopropyl) controlled pore glass from Example 5, was used to synthesize the following oligonucleotides: wherein "*" denotes the 3'-O hexylamino-modified nucleoside.

Standard commercial phosphoramidites were used with the synthesis cycle times specified by the manufacturer in a 380B ABI instrument (Applied Biosystems).

Oligomer 49 was used for structural proof of 3'-O-alkylamine-bearing oligonucleotides at the 3'-terminal end. It showed the expected three ³¹P NMR signals (-0.5 ppm, -0.25 ppm, -0,2 ppm) and seven lines in the trace aromatic base region in ¹H NMR its spectrum.

Oligomer 51 was used to demonstrate the nuclease resistance offered by this the alkylamino group and also for further conjugation. The oligomer was treated with pyrene-butyric acid-N-hydroxy succinimide ester in 0.2 M NaHCO₃ buffer/DMF. The product, Conjugate 1, was purified by HPLC and size exclusion methods. HPLC retention times (eluting with a gradient of 5% CH₃CN for 10 minutes then 5%-40% CH₃CN for 50 minutes) were as follows:

| | **Retention Time** **(min.)** |
|---|---|
| Oligomer 50 | 25.99 |
| Oligomer 51 | 25.91 |
| Conjugate 1 | 49.35 |

The nuclease stability of Oligomer 51 and the conjugate were tested against Oligomer 50 in HeLa cytoplasmic/nuclear extracts. The cell extract was diluted 1.4 times. The final concentration of oligonucleotide was 20 µM. The half lives of the oligonucleotides were as follows:

| | **t**_{**1/2**} **(hours)** |
|---|---|
| Oligomer 50 | 1.0 |
| Oligomer 51 | 3.5 |
| Conjugate 1 | 3.6 |

The half life of phosphodiester Oligomer 50 increased 3-4 times by simple modification at the 3'-end with the hexylamino group, by itself, or by further conjugation.

### EXAMPLE 40

### Oligonucleotides Having 2'-O-Alkylamino Group

**A.** The phosphoramidite from Example 4, 5'-O-(dimethoxytrityl)-2'-O-[hexyl-(Ω-N-phthalimido) amino] -uridine-3'-O-[(2-cyanoethyl)-N,N-diisopropyl]phosphoramidite was made as a 0.2 M solution in anhydrous CH₃CN and used to synthesize the following oligonucleotides in an ABI DNA synthesizer, model 380 B. During the modified amidite coupling, the reaction time was increased to 10 minutes. A coupling efficiency of approximately 90% was observed. After deprotection with concentrated ammonium hydroxide (55°C, 16 hours) the oligonucleotides were purified by reverse phase HPLC and desalting column (Sephadex G-25).

### B. GCGTGTU'CTGCG where U' is 2'-O-[hexyl-N-(1-pyrenepropyl-carbonyl)amino uridine, Conjugate 2 (Oligomer 52 - pyrene butyrate conjugate).

To 20 O.D. of Oligomer 52 in 200 µL of 0.2 M NaHCO₃ buffer, 5 ml of pyrene-butyric acid-N-hydroxy succinimide ester in an Eppendorf tube was added followed by 200 µL of DMF. The tube was shaken overnight. The reaction was purified by size exclusion and HPLC to yield 18 O.D. of product.

### C. GCGTGTU"CTGCG where U" is 2'-O-[6-bromoacetamidohex-lyl]-uridine, Conjugate 3 (Oligomer 52 - bromoacetate conjugate).

To 12 O.D. of Oligomer 52 in 100 µL of 0.2 M NaHCO₃ buffer, 2 mg bromoacetic acid-NHS ester (N-hydroxy succinimidyl bromoacetate) was added. After leaving the reaction to stand overnight, it was purified by size exclusion and HPLC to yield 7.5 O.D. of product.

### D. GCGTGTU^CTGCG where U^ is 2'-O-[hexyl-N-(polyethylene glycol)-propionoyl]amino uridine, Conjugate 4 (Oligomer 52 - PEG conjugate).

To 24 O.D. of Oligomer 52 in 200 µL of 0.2 M NaHCO₃ buffer, 20 mg of Polyethylene glycol propionic acid-N-hydroxy succinimide ester was added. The reaction was mechanically shaken overnight and purified by Sephadex G-25 size exclusion and chromatography to yield 22 O.D. of product.

HPLC retention times (eluting with a gradient of 5% CH₃CN for 10 minutes then 5%-40% CH₃CN for 50 minutes in a C-18 Delta-Pak reverse phase column) were as follows:

| | **Retention Time** **(min.)** |
|---|---|
| Oligomer 52 | 24.05 |
| Conjugate 2 | 40.80 |
| Conjugate 3 | 26.04 |
| Conjugate 4 | 55.58 |

Changes in Tₘ due to pyrene conjugation were evaluated against both DNA and RNA. Tₘ was measured in 100 mM Na⁺, 10 nM phosphate, 0.1 mM EDTA, pH 7 at 4 µM strand concentration. The results were as follows:

| | **T**_{**m**} **v. DNA (°C)** | **T**_{**m**} **v. RNA (°C)** |
|---|---|---|
| Oligomer 52 | 50.9 | 55.5 |
| Conjugate 2 | 55.3 | 55.5 |
| | (4.4) | (0.0) |

The values in parentheses are changes in Tₘ compared to amino linker in oligomer 52 as a control.

### EXAMPLE 41

### Oligonucleotide synthesis using 2'-O-hexylamino(pyrene-butyrate)uridine phosphoramidite.

The amidite 5'-O-(dimethoxytrityl)-2'-O-[hexyl-N-(1-pyrene propyl carbonyl)amino]uridine-3'-O-(2-cyanoethyl-N,N-diisopropyl) phosphoramidite (0.2M in anhydrous acetonitrile) was used to synthesize the following oligomers, both for NMR studies: (**U***=2'-*O*-modified phosphoramidite)

These oligomers were purified trityl-on reverse-phase HPLC, detritylated in 80% acetic acid for one hour and then repurified by RP-HPLC and desalted by size-exclusion chromatography. NMR analysis showed the presence of pyrene peaks.

### EXAMPLE 42

### Oligonucleotide synthesis using 2'-O-hexylamino(dinitrophenyl)uridine phosphoramidite.

The amidite 5'-O-(dimethoxytrityl)-2'-O-[hexyl-N-(2,4-dinitrophenyl)amino]uridine-3'-O-(2-cyanoethyl-N,N,-diisopropyl)phosphoramidite (0.18 M in anhydrous acetonitrile) was used to synthesize oligonucleotides, Oligomers 56 to 63. All are analogues of an ICAM antisense sequence. These oligomers were purified trityl-on by RP-HPLC (Waters Delta-Pak C₁₈ column, 300 Å, 7.8 mm x 30 cm, linear 50-min gradient of 5-60% acetonitrile in 0.05 M TEAA pH 7.3), detritylated in 80% acetic acid for one hour and then purified by RP-HPLC and desalted by size-exclusion chromatography. Data are summarized below:

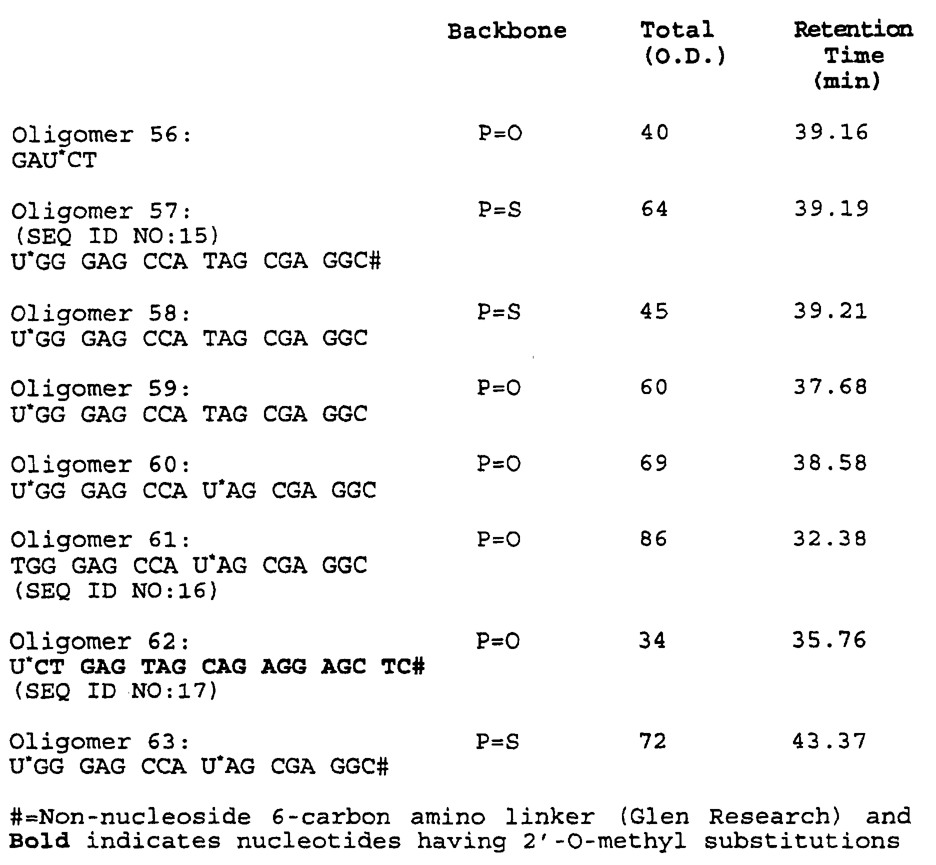

### EXAMPLE 43

### Oligonucleotide synthesis using 2'-O-[hexylamino-(cholesterol)]uridine phosphoramidite

The amidite 5'-O-dimethoxytrityl-2'-O-[hexyl-N-(3-oxycarbonyl-cholesteryl)amino]uridine-3'-O-[2-cyanoethyl-N,N,-diisopropyl]-phosphoramidite (0.2M in anhydrous acetonitrile/dichloromethane 2:1 v/v) was used to synthesize Oligomers 67-74. These oligomers are purified trityl-on by reverse-phase HPLC (Waters Delta-Pak C₁₈, 300Å, 7.8 mm x 30 cm, linear 55-min gradient of 5-80% acetonitrile in 0.05 M TEAA pH 7.3), detritylated in 80% acetic acid for one hour and then repurified by RP-HPLC and desalted by size-exclusion chromatography. Data are summarized below:

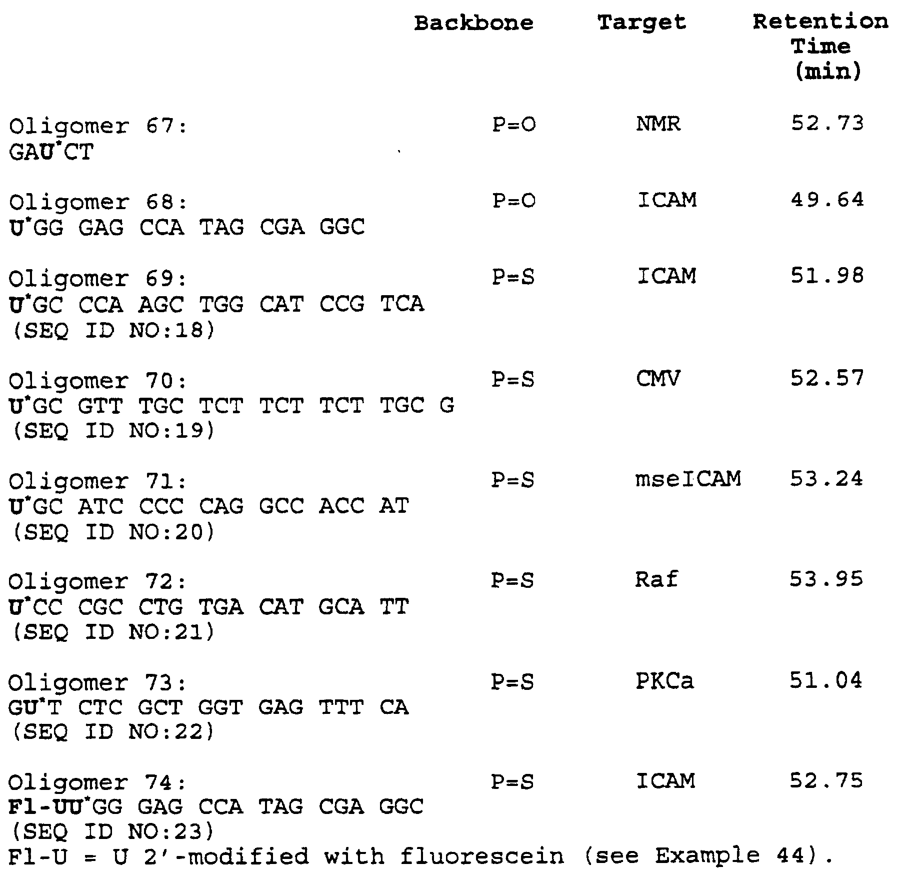

### EXAMPLE 44

### Synthesis of oligonucleotides using 2'-O-[hexylamino-(fluorescein)] amidite.

The amidite 5'-O-dimethoxytrityl-2'-O-[hexyl-N-(5-thiocarbonyl-3,6-dipivolyl fluorescein)amino]uridine-3'-O-(cyanoethyl-N,N-diisopropyl phosphoramidite) (0.2 M in anhydrous acetonitrile) was used to synthesize Oligomer 74 (above) and Oligomers 75-82 on a 1 x 10⁵ (Oligomer 75) or 1 x 10² (remaining Oligomers) µmol scale. These oligomers are purified trityl-on by reverse phase HPLC (Waters Delta-Pak C₁₈, 300Å, 7.8 mm x 30 cm, linear gradient of acetonitrile in 0.05 M TEAA pH 7.3), detritylated in 80% acetic acid for one hour and then repurified by RP-HPLC and desalted by size-exclusion chromatography.

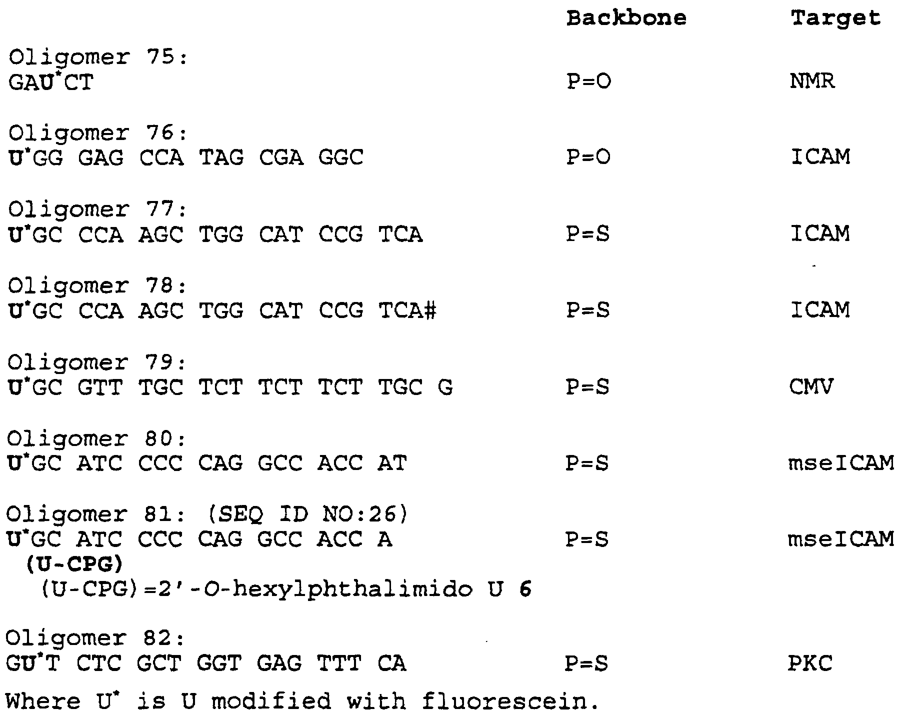

### EXAMPLE 45

### 1-Adamantane Acetic Acid Pentafluorophenyl Ester

1-Adamantane acetic acid (5 g, 25.77 mmol) was dissolved in 50 mL of CH₂Cl₂ and to this suspension pentafluorophenyl (5 g, 27.16 mmol) was added. Dicylohexylcarbodiimide (DCC) was then added (6 g, 29.12 mmol), at which point a highly exothermic reaction set in. The reaction flask was saturated with argon, sealed and then shaken in a wrist action shaker. After 5 hours the solution was filtered in a sintered glass funnel to filter off the dicylohexylurea formed during the reaction. The methylene chloride solution was evaporated and the pale yellow solid product (9g, 97% crude product yield) obtained was used in the next condensation step.

### EXAMPLE 46

### 5'-O-Dimethoxytrityl-2'-O-[hexyl-N-(1-adamantane acetamido)-amino]uridine

5'-*O*-Dimethoxytrityl-2'-*O*-(hexylamino)uridine (2 g, 3.15 mmol) was dissolved in 20 mL of methylene chloride. Adamantane acetic acid pentafluorophenyl ester (1.8 g from the previous step, 5 mmol crude product) was added followed by 3 mL of triethylamine. The reaction mixture was shaken for 4 hours in a wrist action shaker. TLC analysis (5% CH₃OH in CH₂Cl₂) showed the product formation as revealed by a moving trityl positive spot. When the amine at the origin was completely consumed (5 hours) the reaction mixture was evaporated and applied to a silica column loaded with chloroform solvent (20cm x 5cm diameter). The column was eluted with chloroform (1 liter) followed by 5% CHCl₃ in CH₃OH. The material which showed trityl positive spots in TLC was pooled together and evaporated to give 2.5 g of the nucleoside-adamantane conjugate (98% yield). ¹³C analysis showed the presence of both uridine and adamantane residues. R_{f}= 0.21 in 5% CH₃OH in CH₂Cl₂

### EXAMPLE 47

### 5'-O-Dimethoxytrityl-2'-O-[hexyl-N-(1-adamantane acetamido)-amino]uridine-3'-O-(2-cyanoethyl-N,N'-diisopropyl) Phosphoramidite

5'-*O*-Dimethoxytrityl-2'-*O*-[hexyl-N-(1-adamantane acetamido)amino]uridine (2.4 g, 2.96 mmol) was dissolved in 20 mL of CH₂Cl₂. To this solution 200 mg of diisopropylaminotetrazolide (1.16 mmol) was added followed by 3.1 mL of 2-cyanoethyl-N,N,N',N'-tetraisopropyl phosphoramidite (9.45 mmol) was added under argon atmosphere. The mixture was stirred under argon overnight after which time TLC (5% CH₃OH/CH₂Cl₂) indicated almost complete conversion of nucleoside to its phosphoramidite. The reaction mixture was washed with saturated NaHCO₃ followed by saturated NaCl solution and then dried over anhydrous MgSO₄. The solution was evaporated and the foamy residue was purified in a silica column using 5% CH₃OH/CHCl₃ elution system to give the purified phosphoramidite (2 g, 67% yield). ³¹P NMR: 150.741, 150.49 ppm.

### EXAMPLE 48

### Oligonucleotide Synthesis with Adamantane Modified Amidite

5'-*O*-Dimethoxytrityl-2'-*O*-[hexyl-N-(1-adamantane acetamido)amino]uridine phosphoramidite was dissolved in anhydrous CH₃CN (100 mg of amidite for each 1 mL of solvent to give 0.1 M solution of the amidite). For the novel amidite the coupling time was extended by increasing the amidite and delivery time to 7.5 minutes and the waiting time to 6.0, thus a total of 13.5 minutes were used.

The oligonucleotides were deprotected and purified using the procedure described in Example 2. Table I summarizes the different oligonucleotides along with their HPLC retention times.

### EXAMPLE 49

### Table of Oligonucleotides Synthesized with their HPLC Retention Times - Incorporation of 2'-O-(CH₂)₆-NHCO-O-adamantylmethyl Uridine using amidite:

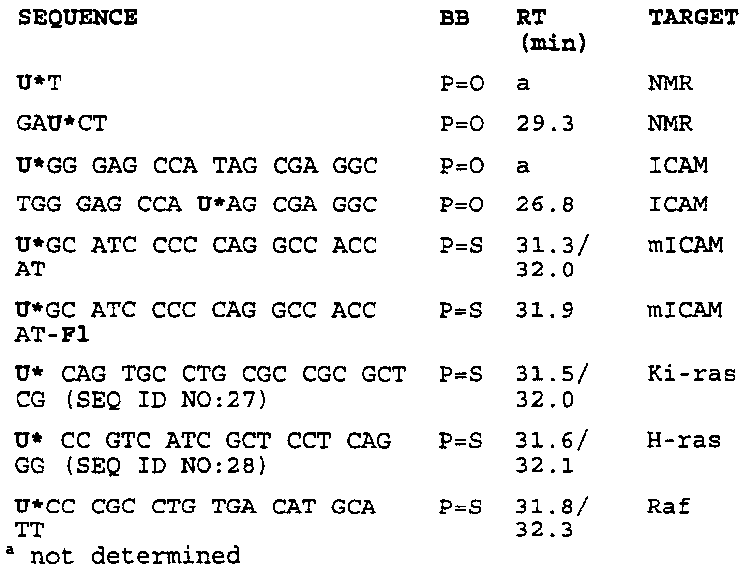

HPLC conditions: Waters DeltaPak C-18 Delta Pak reverse-phase 15m 300 Å column, 3.9x 300 mm size equipped with C-18 guard column; Solvent A: 50 mM TEAA, pH 7.0.; Solvent B: acetonitrile; Gradient: 0-10 min., 5% acetonitrile; 10-60 min., 5% to 60% acetonitrile linear increase.

### EXAMPLE 50

### cis-11-Eicosenoic Acid Pentafluorophenyl Ester

*cis-*11-Eicosenoic acid (Aldrich, 2 g, 6.44 mmol) was dissolved in 50 mL of THF and to this suspension 1.2 g of pentafluorophenyl (6.5 mmol) was added followed by 1.33 g of DCC. The suspension was saturated with argon, sealed with a rubber septum and then shaken in a wrist action shaker. After 4 hours a clear pale yellow solution results with creamy white precipitate. The precipitated dicyclohexylurea was filtered off, washed with methylene chloride and the combined organic solution was evaporated to give the desired product as a waxy solid (3.25 g, crude product yield). This solid was used in the next condensation step without any further purification.

### EXAMPLE 51

### 5'-O-Dimethoxytrityl-2'-O-[hexyl-N-(cis-11-eicosenoicamido)-amino]uridine

5'-*O*-Dimethoxytrityl-2'-*O*-(hexylamino)uridine (2 g, 3.15 mmol) was dissolved in 20 mL of methylene chloride. *cis*-11-Eicosenoic acid pentafluorophenyl ester (2.5 g from the previous step, 5 mmol crude product) was added followed by 3 mL of pyridine. The reaction mixture was shaken for 4 hours in a wrist action shaker. TLC analysis (5% CH₃OH in CH₂Cl₂) showed the product formation as revealed by a trityl positive spot above the origin. When the amine at the origin was completely consumed (5 hours) the reaction mixture was evaporated and applied to a silica column loaded with chloroform solvent (20 cm x 5 cm diameter). The column was eluted with chloroform (1 liter) followed by 5% CHCl₃ in CH₃OH. The material which showed trityl positive spots in TLC was pooled together and evaporated to give 2.72 g of the nucleoside-eicosenoic acid conjugate (93% yield). ¹³C analysis showed the presence of both uridine and *cis*-11-eicosenoic acid residues. R_{f}= 0.29 in 5% CH₃OH in CH₂Cl₂.

### EXAMPLE 52

### 5'-O-Dimethoxtrityl-2'-O-[hexyl-N-(cis-11-eicosenoic amido) amino]uridine-3'-O-(2-cyanoethyl-N,N'-diisopropyl) Phosphoramidite

The eicosenoic acid-uridine nucleoside from the previous step (2.5 g, 2.7 mmol) was dissolved in 20 mL of CH₂Cl₂. To this solution 200 mg of diisopropylaminotetrazolide (1.16 mmol) was added followed by 2.0 mL of 2-cyanoethyl-N,N,N',N'-tetraisopropyl phosphoramidite (6.1 mmol) was added under argon atmosphere. The mixture was stirred under argon overnight after which time TLC (5% CH₃OH/CH₂Cl₂) indicated almost complete conversion of nucleoside to its phosphoramidite. The reaction mixture was washed with saturated NaHCO₃ followed by saturated NaCl solution and then dried over anhydrous MgSO₄. The solution was evaporated and the foamy residue was purified in a silica column using 5% CH₃OH/CHCl₃ elution system to give the purified phosphoramidite (2 g, 67% yield). As the phosphoramidite was contaminated with the H-phosphorate from the phosphitylating reagent, it was repurified in a silica column, by eluting with 8:2 EtOAc: Hexane to give 1.8 g (60% yield) of the phosphoramidite. ³¹P NMR: 150.74 and 150.56 ppm.

### EXAMPLE 53

### Oligonucleotide Synthesis with cis-11-eicosenoic acid modified amidite

5'-*O*-Dimethoxytrityl-2'-*O*-[hexyl-N-(*cis*-11-eicosenoic amido)amino]uridine phosphoramidite was dissolved in anhydrous CH₃CN (100 mg of amidite for each 1 mL of solvent to give 0.09 M solution of the amidite). For the novel amidite the coupling time was extended by increasing the amidite and delivery time to 7.5 minutes and the waiting time to 6.0, thus a total of 13.5 minutes were used.

The oligonucleotides were deprotected and purified using the procedure described in Example 2. Table II summarizes the different oligonucleotides along with their HPLC retention times.

### EXAMPLE 54

### Table of Incorporation of 2'-O-(CH₂)₆-NHCO-Cis-11-Eicosenoic U using amidite:

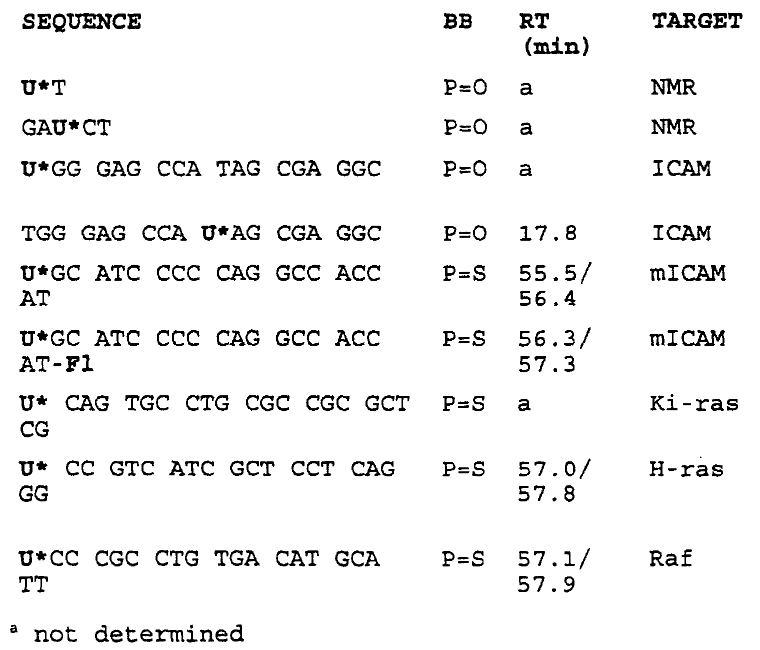

HPLC conditions: Waters DeltaPak C-18 Delta Pak reverse-phasen 15m 300 Å column, 3.9x 300 mm size equipped with C-18 guard column; Solvent A: 50mM TEAA, pH 7.0.; Solvent B: acetonitrile; Gradient: 0-10 min., 5% acetonitrile; 10-60 min., 5% to 60% acetonitrile linear increase.

### EXAMPLE 55

### 5'-O-Dimethoxytrityl-2'-O-[hexyl-N-(N-biotinyl-6-aminocaproyl)amino]uridine

5'-*O*-Dimethoxytrityl-2'-*O*-(hexylamino)uridine (1.05 g, 1.66 mmol) was dissolved in 20 mL of methylene chloride. N-Biotinyl-6-amino caproic acid (0.75 g, Fluka, 1.65 mmol) was added followed by 1 mL of pyridine. The reaction mixture was shaken for 4 hours in a wrist action shaker. The white suspension of the biotin reagent completely dissolved into solution by this time, TLC analysis (5% CH₃OH in CH₂Cl₂) showed the product formation as revealed by an upper trityl positive spot above the origin. When the amine at the origin was completely consumed (5 hours) the reaction mixture was evaporated and applied to a silica column loaded with chloroform solvent (20 cm x 5 cm diameter). The column was eluted with chloroform (1 liter) followed by 5% CHCl₃ in CH₃OH (1 liter), then followed by 10% CH₃OH in CHCl₃. The material, which elutes out with 10% CH₃OH, showed trityl positive spots in TLC was pooled together and evaporated to give 1.53 g of the nucleoside-biotin conjugate (9a% yield). ¹³C analysis showed the presence of both uridine and biotin residues. R_{f}= 0.214 in 10% CH₃OH in CH₂Cl₂

### EXAMPLE 56

### 5'-O-Dimethoxytrityl-2'-O-[hexyl-N-(N-biotinyl-6-aminocaproyl)amino]uridine 3'-O-(2-cyanoethyl-N,N'-diisopropyl) Phosphoramidite

The biotin-uridine nucleoside from the previous step was dissolved in 20 mL of CH₂Cl₂. To this solution 100 mg of diisopropylaminotetrazolide (.58 mmol) was added followed by 1.0 mL of 2-cyanoethyl-N,N,N',N'-tetraisopropyl phosphoramidite (3.05 mmol) was added under argon atmosphere. The mixture was stirred under argon overnight after which time TLC (5% CH₃OH/CH₂Cl₂) indicates almost complete conversion of nucleoside to its phosphoramidite. The reaction mixture was washed with saturated NaHCO₃ followed by saturated NaCl solution and then dried over anhydrous MgSO₄. The solution was evaporated and the foamy residue was purified in a silica column using 100% ethyl acetate elution system to give the purified phosphoramidite.

### EXAMPLE 57

### 5-O-Dimethoxytrityl-3'-O-(hexyl-N-(N-biotinyl-6-aminocaproyl)-amino]uridine

5'-*O*-Dimethoxytrityl-3'-*O*-(hexylamino)uridine (0.53 g, 0.83 mmol) was dissolved in 10 mL of methylene chloride. N-Biotinyl-6-aminocaproic acid (0.75 g, Fluka, 1.65 mmol) was added followed by 0.5 mL of pyridine. The reaction mixture was shaken for 4 hours in a wrist action shaker. The white suspension of the biotin reagent was completely dissolved into solution by this time. TLC analysis (10% CH₃OH in CH₂Cl₂) showed the product formation as revealed by an upper trityl positive spot above the origin. When the amine at the origin was completely consumed (5 hours) the reaction mixture was evaporated and applied to a silica column loaded with chloroform solvent (20 cm x 5 cm diameter). The column was eluted with chloroform (1 liter) followed by 5% CHCl₃ in CH₃OH (1 liter). The material, which elutes out with 10% CH₃OH, showed trityl positive spots in TLC was pooled together and evaporated to give 0.6 g of the nucleoside-biotin conjugate and biotin residues. R_{f}= 0.21 in 10% CH₃OH in CH₂Cl₂.

### EXAMPLE 58

### 5'-O-Dimethoxytrityl-3'-O-[hexyl-N-(N-biotinyl-6-aminocaproyl)amino]uridine-2'-O-(succinylaminopropyl)Controlled Pore Glass

Succinylated/capped aminopropyl controlled pore glass was dries under vacuum for 3 hours immediately before use. A portion (0.3 g, 24 µmol) was added to 3 mL anhydrous pyridine in a 50 mL round-bottom flask. DEC (0.12 g, 0.63 mmol), TEA (25 µL, distilled over CaH₂). DMAP (0.005 g, 40 µmol) and 5'-*O*-(dimethoxytrityl)-3'-*O*-[hexyl-N-(N-biotinyl-6-aminocaproyl) amino]uridine (0.22 g, 0.22 mmol) were added under argon and the mixture shaken mechanically for 19 hours. More nucleoside (0.025 g) was added and the mixture shaken for an additional 5.5 hours. Pentachlorophenol (0.045 g, 0.168 mmol) was add and the mixture shaken for 18 hours. CPG was filtered off and washed successively with dichloromethane, triethylamine, and dichloromethane. The resulting CPG was then dried under vacuum, suspended in 15 mL piperidine and shaken 30 minutes. CPG was filtered off, washed thoroughly with dichloromethane and again dried under vacuum. The extent of loading (determined by spectrophotometric assay of dimethoxytrityl cation in 0.3 M p-toluenesulfonic acid at 498 nm) was approximately 36 µmol/g. The product solid support was subsequently used to synthesize the oligomers, containing biotin at the 3' nucleotide unit.

### EXAMPLE 59

### Oligonucleotide Synthesis with Biotin Modified Amidite and Control Pore Glass

5'-*O*-Dimethoxytrityl-3'-*O*-[hexyl-N-(N-biotinyl-6-aminocaproyl)amino]uridine nucleoside phosphoramidite was dissolved in anhydrous CH₃CN (100 mg of amidite for each 1 mL of solvent to give 0.08-0.09 M solution of the amidite). For the novel amidite the coupling time was extended by increasing the amidite and delivery time to 7.5 minutes and the waiting time to 6.0, thus a total of 13.5 minutes were used.

The oligonucleotides were deprotected and purified using the procedure described in Example 2. The controlled pore glass containing biotin was used to synthesize 3'-biotin containing oligonucleotides.

### EXAMPLE 60

### 1-2-Di-O-hexadecyl-rac-glycerol-succinimidyl-carbonate

1,2-Di-*O*-hexadecyl-rac-glycerol (2g, 3.69 mmol) was dissolved in 3 mL of CH₂Cl₂ and to this solution disuccinimidyl carbonate (DSC) (1.42 g, 5.54 mmol) was added. Triethylamine was then added (2 mL, 14.3 mmol), followed by 20 mL of anhydrous acetonitrile. The reaction flask was saturated with argon, sealed and then shaken in a wrist action shaker. After 8 hours the reaction mixture was evaporated. The residue was dissolved in CH₂Cl₂ (100 mL), washed with saturated NaHCO₃ solution followed by saturated NaCl solution and the organic layer was dried over anhydrous MgSO₄. The solid residue (3.5 g) was used in the next step to condense with the amine. ¹³C NMR confirms the complete conversion of 1,2-di-*O*-hexadecyl-rac- glycerol to the corresponding succinimidyl carbonate.

### EXAMPLE 61

### 5'-O-Dimethoxytrityl-2'-O-[hexyl-N-(carbonoyl 1,2-di-O-hexadecyl-rac-glycerol)amino]uridine

5'-*O*-Dimethoxytrityl-2'-*O*-(hexylamino)uridine (2 g, 3.15 mmol) was dissolved in 20 mL of methylene chloride. 1,2-Di-*O*-hexadecyl-rac-glycerol succinimidyl carbonate (3.0 g from the previous step, 5 mmol crude product) was added followed by 3 mL of pyridine. The reaction mixture was shaken for 4 hours in a wrist action shaker. TLC analysis (5% CH₃OH in CH₂Cl₂) showed the product formation as revealed by a moving trityl positive spot. When the amine at the origin was completely consumed (5 hours) the reaction mixture was evaporated and applied to a silica column loaded with chloroform solvent (20 cm x 5 cm diameter). The column was eluted with chloroform (1 liter) followed by 5% CHCl₃ in CH₃OH. The material which showed trityl positive spots in TLC was pooled together and evaporated to give 3.0 g of the nucleoside-lipid conjugate (98% yield). ¹³C analysis showed the presence of both uridine and lipid residues.

### EXAMPLE 62

### 5'-O-Dimethoxytrityl-2'-O-[hexyl-N-(carbonoyl 1,2-di-O-hexadecyl-rac-glycerol-succinimidyl-carbonate]-3'-O-(2-cyanoethyl-N,N'-diisopropyl) Phosphoramidite

The lipid-uridine nucleoside from the previous step was dissolved in 20 mL of CH₂Cl₂. To this solution 200 mg of diisopropylaminotetrazolide (1.16 mmol) was added followed by 1.55 mL of 2-cyanoethyl-N,N,N',N'-tetraisopropyl phosphoramidite (4.75 mmol) was added under argon atmosphere. The mixture was stirred under argon overnight after which time TLC (5% CH₃OH/CH₂Cl₂) indicated almost complete conversion of nucleoside to its phosphoramidite. The reaction mixture was washed with saturated NaHCO₃ followed by saturated NaCl solution and then dried over anhydrous MgSO₄. The solution was evaporated and the foamy residue was purified in a silica column using 5% CH₃OH/CHCl₃ elution system to give the purified phosphoramidite.

### EXAMPLE 63

### Oligonucleotide Synthesis with Lipid Modified Amidite

5'-*O*-Dimethoxytrityl-2'-*O*-[hexyl-N-(carbonoyl-1,2-di-*O*-hexadecyl-*rac*-glycerol)amino]uridine phosphoramidite was dissolved in anhydrous CH₃CN (100 mg of amidite for each 1 mL of solvent to give 0.08-0.09 M solution of the amidite). For the novel amidite the coupling time was extended by increasing the amidite and delivery time to 7.5 minutes and the waiting time to 6.0, thus a total of 13.5 minutes were used. The oligonucleotides were deprotected and purified using the procedure described in Example 2.

### EXAMPLE 64

### Oligonucleotide Synthesis

5'-*O*-Dimethoxytrityl-2'-*O*-[hexyl-N-(1-pyrene butyroyl) amino]uridine phosphoramidite was dissolved in anhydrous CH₃CN (100 mg of amidite for each 1 mL of solvent to give 0.08-0.09 M solution of the amidite). For the novel amidite the coupling time was extended by increasing the amidite and delivery time to 7.5 minutes and the waiting time to 6.0 minutes, hence using a total of 13.5 minutes.

The oligonucleotides were deprotected and purified using the procedure described in Example 2. The Table below summarizes the different oligonucleotides (ICAM, ras, raf and PKC-a oligonucleotides).

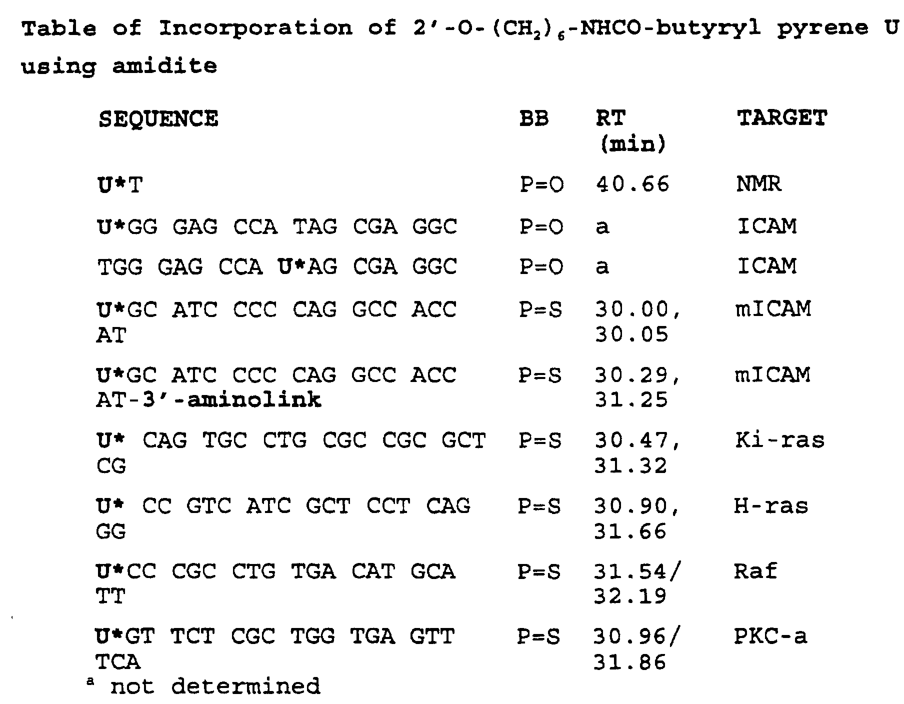

HPLC conditions: Waters DeltaPak C-4 Delta Pak reverse-phase 15m 300 Å column, 3.9x 300 mm size equipped with C-18 guard column; Solvent A: 50mM TEAA, pH 7.0.; Solvent B: acetonitrile; Gradient: 0-10 min., 5% acetonitrile; 10-60 min., 5% to 60% acetonitrile linear increase.

### EXAMPLE 65

### Incorporation of 2'-O-(CH₂)₆-NHCO-cholesterol Uridine at 5'-End of Oligonucleotides Using Amidites

### Synthesis:

Oligonucleotides were synthesized on an Expedite Nucleic Acid Synthesis System (Millipore Corp., Bedford, MA). Two 1-µmol syntheses or a single 15 µmol synthesis was performed for each oligonucleotide. Standard DNA synthesis conditions were used except during coupling of the modified amidite which was accomplished as follows: The novel amidite was dissolved in CH₂Cl₂/CH₃CN 50/50 (0.08-0.1M). For 10 µmol syntheses, a 6-min wait step was added to the coupling cycle, which was repeated once. For 1 µmol phosphodiester syntheses, the coupling step was also extended for 6 min, with periodic addition of fresh amidite. For 1 µmol phosphorothioate syntheses, the coupling step was extended for 13 min, with periodic addition of fresh amidite.

### Purification:

After standard cleavage from CPG and deprotection (ammonium hydroxide at 55 °C for 16-24 hours), oligonucleotides were trityl-on purified by preparative HPLC to remove failure sequences. HPLC conditions: Waters Delta-Pak C₄ column, 7.8 mm x 30 cm, 300 A, 15 u; 2.5 ml/min flow rate using a gradient of 5-80% AcCN in 50mM triethylammonium acetate pH 7.0 over 50 min. Following lyophilization, oligonucleotides were detritylated for 45 min in 80% acetic acid and lyophilized again. Oligonucleotides were then further purified by size exclusion on G-25 columns to remove salts and the trityl group.

### Analysis:

Except for 8002, oligonucleotides were analyzed by HPLC using a gradient analogous to the one above and a Waters Delta-Pak C₄ column (3.9 mm x 30 cm) with a flow rate of 1.5 ml/min over 50 min. 8002 was analyzed using a gradient of 5-60% AcCN. Purified phosphorothioate oligonucleotides appeared as two peaks by HPLC, phosphodiesters as one peak. Oligonucleotides were further analyzed by electrophoresis on 20% polyacrylamide denaturing (7M urea) gels. All were predominantly single bands of the appropriate size compared to standards. Gel analysis of 8012 (loaded without any pretreatment) shows a very high band, presumably the tetrameric form of this oligonucleotide, plus a faint band where one would expect a monomeric 8mer+cholesterol to run. (The parent compound, 5320, is known to form a G-quartet structure.) Some oligonucleotides were also analyzed by capillary electrophoresis. Selected oligonucleotides have been analyzed by electrospray mass spectrometry.

Results are shown in the following Table were * indicated site of selected modification.

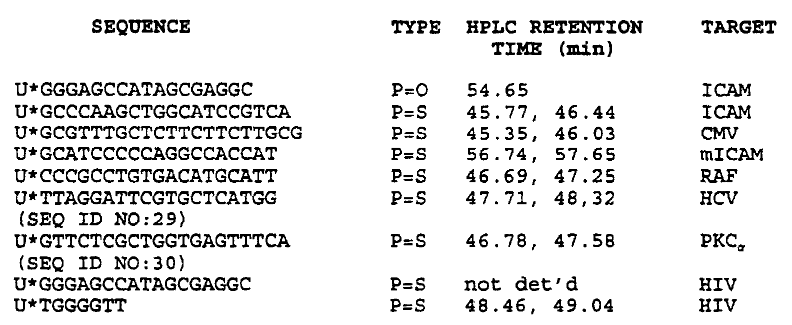

### Example 66

### Incorporation of 2'-O-(CH₂)₆-NHCO-cholesterol Uridine at nonterminal positions of oligonucleotides using amidite

Oligonucleotides incorporating 2'-O-(CH₂)₆-NHCO-cholesterol at nonterminal (not at either the 5' or the 3' terminus of the oligonucleotide) were synthesized at a 1 µmol syntheses scale, analyzed and purified as per the procedures of Example 65.

Results are shown in the following Table were * indicated site of selected modification.

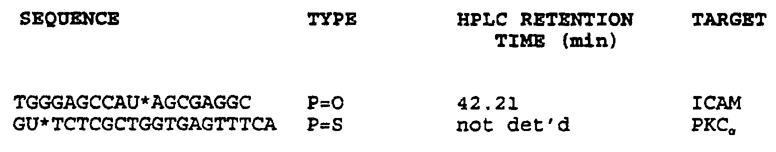

### Example 67

### Incorporation of 3'-O-(CH₂)₆-NHCO-cholesterol Uridine at 3' end of oligonucleotides using modified CPG

Cholesterol-modified CPG was packed into screw-top CPG columns (Glen Research). Oligonucleotides were synthesized on a 1-µmol syntheses scale, analyzed and purified as in Example 65 except that standard DNA synthesis conditions were used for all couplings.

Results are shown in the following Table were * indicated site of selected modification.

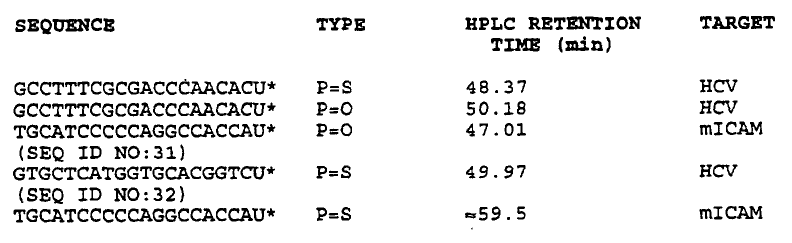

### Example 68

### Incorporation of 2'-O-(CH₂)₆-NHCO-cholesterol Uridine at 3'end of oligonucleotides using modified CPG

Cholesterol-modified CPG was packed into screw-top CPG columns (Glen Research). Oligonucleotides were synthesized on a 1-µmol syntheses scale, analyzed and purified as in Example 65 except that standard DNA synthesis conditions were used for all couplings.

Results are shown in the following Table were * indicated site of selected modification.

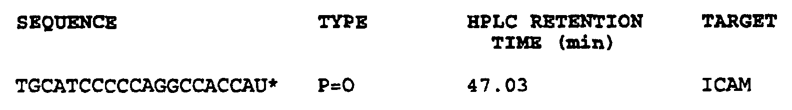

### Example 69

### Double Incorporation of Cholesterol by placement of (2'-O-(CH₂)₆-NHCO-cholesterol Uridine on 5' end and 3'-O-(CH₂)₆-NHCO-cholesterol Uridine on 3' end

Cholesterol-modified CPG was packed into screw-top CPG columns (Glen Research). Oligonucleotides were synthesized on a 1-µmol syntheses scale, analyzed and purified as in Example 65 except that the extended coupling step for the cholesterol amidite (see Example 65) was repeated once.

Results are shown in the following Table were * indicated site of selected modification.

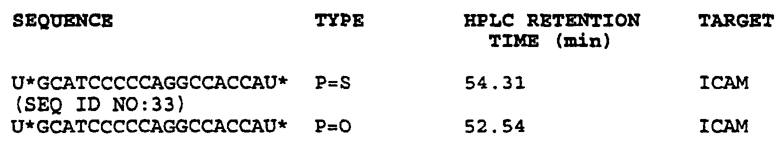

### Example 70

### Incorporation of Cholesterol and Aminohexyl Modifications into 2'F Chimeric Oligonucleotides

The parent oligonucleotide, complementary to messenger RNA of PKCα, is a chimeric phosphorothioate oligonucleotide having a central deoxyribonucleotide region that is "flanked" on both the 5' and the 3' sides with region that have 2'-deoxy-2'-fluoro nucleotides. These compound are alternately referenced as "gapped" oligonucleotides. The central "gapped" regions are shown in underlines in the Table below with the "flanks" (or alternatively the "wings") shown in unmodified type.

The oligonucleotides were synthesized, analyzed and purified as in Example 65, except for the following: Concentration of all amidites was 0.1 M. Two 1-µmol syntheses were performed for each oligonucleotide. The coupling step was extended 6 min, with periodic addition of fresh amidite; this extended coupling step was repeated once for the cholesterol amidite coupling. Trityl-on HPLC purification of Compound ID #9532 was accomplished using a linear gradient of 5-40% AcCN over 50 min. After detritylation and purification by size-exclusion, it was analyzed using a similar analytical-scale HPLC procedure gel and capillary electrophoresis. Purified compound ID #9535 appears as two peaks by HPLC, others as one peak. Compound ID#s 9532 and 9534 contained undeprotected 3'-hexylamine; probably about 20% according to previous results (phthalimide is the protecting group).
U₁* = 3'-O-(CH₂)₆-NH₂ U
U₂* = 3'-O-(CH₂)₆-NHCO-cholesterol U
U₃* = 2'-O-(CH₂)₆-NHCO-cholesterol U

Results are shown in the following Table were * indicated site of selected modification.

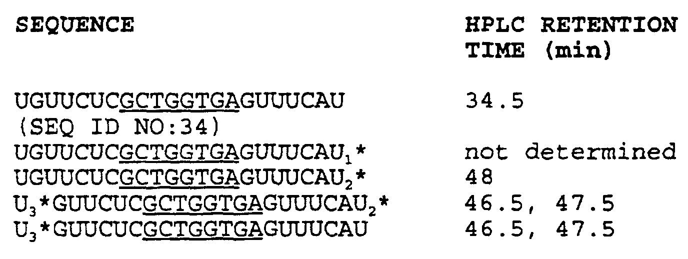

### EXAMPLE 71

### 5'-O-Dimethoxytrityl-3'-O-[hexyl-(omega-N-phtalimido)amino]uridine-2'-O-(2-cyanoethyl-N,N-diisopropyl) Phosphoramidite

The 5'-dimethoxytrityl-3'-*O*-[hexyl-(omega-N-phthalimido)-amino]uridine (2 g, 2.6 mmol) was dissolved in 20 mL anhydrous CH₂Cl₂. To this solution diisopropylaminotetrazolide (0.2 g, 1.16 mmol) and 2.0 mL 2-cyanoethyl-N,N,N',N'-tetraisopropyl phosphoramidite (6.3 mmol) were added and stirred overnight. TLC (1:1 EtOAc/hexane) showed complete disappearance of starting material. The reaction mixture was transferred with CH₂Cl₂ and washed with saturated NaHCO₃ (100 mL), followed by saturated NaCl solution. The organic layer was dried over anhydrous Na₂SO₄ and evaporated to yield 3.8 g of a crude product, which was purified in a silica column (200 g) using 1:1 hexane/EtOAc to give 1.9 g (1.95 mmol, 74% yield) of the desired phosphoramidite.

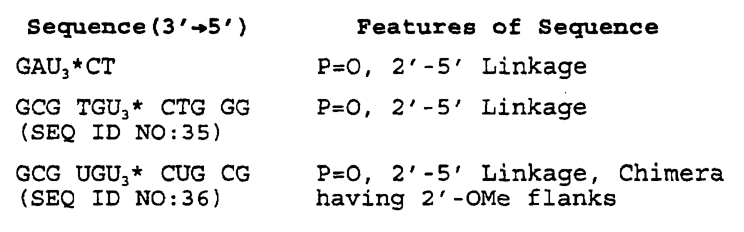

### EXAMPLE 72

### NMR and HPLC Analysis of 5'-3' versus 5'-2' Internucleotide Linkages and 2' substituents versus 3' substituents

The 400MHz ¹H spectrum of oligomer d(GAU₂*CT), where U₂= 2'-O-aminohexyluridine showed 8 signals between 7.5 and 9.0 ppm corresponding to the 8 aromatic protons. In addition, the anomeric proton of U* appears as a doublet at 5.9 ppm with J₁',₂' = 7.5Hz, indicative of C2'-endo sugar puckering. The corresponding 2'-5' linked isomer shows a similar structure with J₁',₂' = 3.85 Hz at 5.75 ppm, indicating an RNA type sugar puckering at the novel modification site favorable for hybridization to an mRNA target. The proton spectrum of the oligomer d(GACU₃*), where U₃*=3'-*O*- hexylamine, showed the expected 7 aromatic proton signals between 7.5 and 9.0 ppm and the anomeric proton doublet at 5.9 ppm with J₁',₂' = 4.4 Hz. This suggests more of a C3'-endo puckering for the 3'-*O*-alkylamino compounds compared to their 2' analogs. ³¹P NMR of these oligonucleotides showed the expected 4 and 3 signals from the internucleotide phosphate linkages for d(GAU*CT) and d(GACU*), respectively. 3'-5' linked vs. 2'-5' linked have different retention times in RP HPLC and hence different lipophilicities, implying potentially different extent of interactions with cell membranes.

### EXAMPLE 73

### 2'-5' Internucleotide Linked Oligonucleotide Conjugation Chemistry

The oligonucleotides 9274 and 9518 (20 ODs each) were dried and re-dissolved in 0.2M NaHCO₃ buffer (200 µL) in an eppendorf tube. 5 mg of pyrene butyric acid-N-hydroxy succinimide ester was dissolved in 400 µL anhydrous DMF. 200 µL of this solution was added to each of the eppendorf tubes and vortexed and left to stand overnight. The reaction mixtures were passed through a g-25 column to remove the excess reagent, an then purified by Reverse-Phase HPLC. The retention times of the starting material and the conjugates are summarized below:

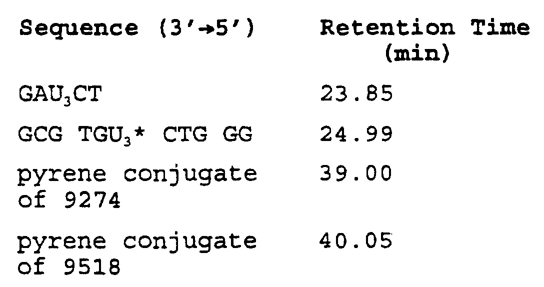

HPLC conditions: Waters DeltaPak C-18 Delta Pak reverse-phase 15m 300 Å column, 3.9x 300 mm size equipped with C-18 guard column; Solvent A: 50mM TEAA, pH 7.0.; Solvent B: acetonitrile; Gradient: 0-10 min., 5% acetonitrile; 10-60 min., 5% to 40% acetonitrile linear increase.

### EXAMPLE 74

### Tm Analysis of 2'-5' v. 3'-5' Connected Oligonucleotides and their Conjugates

Thermal melts were done as per the method of Procedure B(1)(b). Oligonucleotide identity is as follows:
Oligonucleotide 31-A is a normal 3'-5' linked phosphodiester oligodeoxyribonucleotide of the sequence d(GGC TGU* CTG CG) (SEQ ID NO:37) where the * indicates the attachment site of either a 2'-aminolinker or a 2'-aminolinker plus pyrene.
Oligonucleotide 31-B is a normal 3'-5' linked phosphodiester oligoribonucleotide of the sequence d(GGC TGU* CTG CG) where the * indicates the attachment site of either a 2'-aminolinker or a 2'-aminolinker plus pyrene. Each of the ribonucleotides of the oligonucleotide, except the one bearing the * substituent, are 2'-O-methyl ribonucleotides.
Oligonucleotide 31-C is has 2'-5' linkage at the * position in addition to either a 3'-aminolinker or a 3'-aminolinker plus pyrene at this site. The remainder of the oligonucleotide is a phosphodiester oligodeoxyribonucleotide of the sequence d(GGC TGU* CTG CG). The base oligonucleotide (no 2'-aminolinker) was not included in the study.

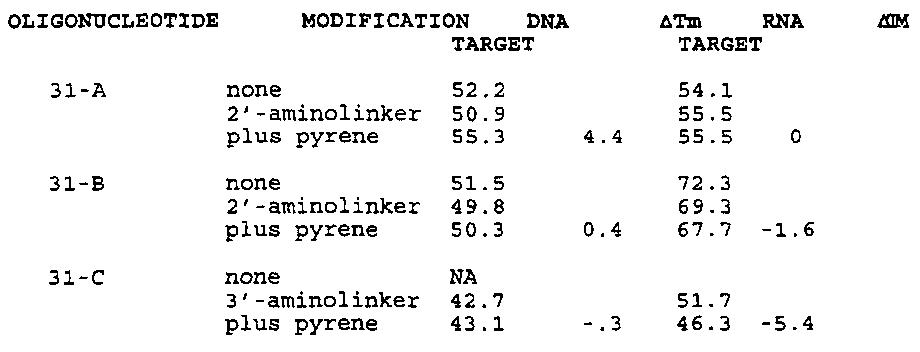

The 2'-5' linkages demonstrated a higher melting temperature against an RNA target compared to a DNA target.

### EXAMPLE 75

### 2'-O-(Propylphthalimide)adenosine and 3'-O-(Propylphthalimide)adenosine

To a solution of adenosine (20.0 g, 75 mmol) in dry dimethylformamide (550 ml) at 5°C was added sodium hydride (60% oil, 4.5 g, 112 mmol). After one hour, N-(3-bromopropyl)phthalimide (23.6 g, 86 mmol) was added and the temperature was raised to 30°C and held for 16 hours. Ice is added and the solution evaporated *in vacuo* to a gum. The gum was partitioned between water and ethyl acetate (4 x 300 ml). The organic phase was separated, dried, and evaporated *in vacuo* and the resultant gum chromatographed on silica gel (95/5 CH₂Cl₂/MeOH) to give a white solid (5.7 g) of the 2'-O-(propylphthalimide)adenosine. Those fractions containing the 3'-*O*-(propylphthalimide)adenosine were rechromatographed on silica gel using the same solvent system.

Crystallization of the 2'-*O*-(propylphthalimide)adenosine fractions from methanol gave a crystalline solid, m.p. 123-124°C. ¹H NMR (400 MHz: DMSO-d₆) δ 1.70(m, 2H, CH₂), 3.4-3.7 (m, 6H, C₅, CH₂, OCH₂, Phth CH₂), 3.95 (q, 1H, C₄,H), 4.30 (q, 1H, C₅,H), 4.46 (t, 1H, C₂,H), 5.15 (d, 1H, C₃,OH), 5.41 (t, 1H, C₅,OH), 5.95 (d, 1H, C₁,H) 7.35 (s, 2H, NH₂), 7.8 (brs, 4H, Ar), 8.08 (s, 1H, C₂H) and 8.37 (s, 1H, C₈H). Anal. Calcd. C₂₁H₂₂N₆O₆: C, 55.03; H, 4.88; N, 18.49. Found: C, 55.38; H, 4.85; N, 18.46.

Crystallization of the 3'-*O*-(propylphthalimide)adenosine fractions from H₂O afforded an analytical sample, m.p. 178-179°C. ¹H NMR (400 MHz: DMSO-d₆) δ 5.86 (d, 1H, H-1'),

### EXAMPLE 76

### 2'-O-(Propylphthalimide)-N⁶-benzoyladenosine

2'-*O*-(Propylphthalimide)adenosine was treated with benzoyl chloride in a manner similar to the procedure of Gaffney, *et al., Tetrahedron Lett.* **1982,** *23,* 2257. Chromatography on silica gel (ethyl acetate-methanol) gives the title compound. Anal. Calcd. C₂₈H₂₆N₆O₇: C, 60.21; H, 4.69; N, 15.05. Found: C, 59.94; H, 4.66; N, 14.76.

### EXAMPLE 77

### 2'-O-(Propylphthalimide)-5'-O-dimethoxytrityl-N-benzoyladenosine

To a solution of 2'-O-(propylphthalimide)-N⁶-benzoyladenosine (4.0 g) in pyridine (250 ml) was added 4,4'-dimethoxytrityl chloride (3.3 g). The reaction was stirred for 16 hours. The reaction was added to ice/water/ethyl acetate, the organic layer was separated, dried, and concentrated *in vacuo* and the resultant gum chromatographed on silica gel (ethyl acetate-methanol triethylamine) to give the title compound. Anal. Calcd. C₉₉H₄₄N₆O₉: C, 68.36; H, 5.15; N, 9.76. Found: C, 68.16; H, 5.03; N, 9.43.

### EXAMPLE 78

### N₆-Benzoyl-5'-O-dimethoxytrityl-2'-(propylphthalimide)-3'-O-(N,N-diisopropyl-β-cyanoethyl)adenosine Phosphoramidite

2'-*O*-(Propylphthalimide)-5'-*O*-dimethoxytrityl-N⁶-benzoyladenosine was treated with (β-cyanoethoxy)chloro-N,N-diisopropyl)aminophosphane in a manner similar to the procedure of Seela, *et al., Biochemistry* **1987**, *26*, 2233. Chromatography on silica gel (EtOAc/hexane) gave the title compound as a white foam. ³¹P NMR (CD₃CN) 150.88, 151.22.

### EXAMPLE 79

### 2'-O-(Propylamino)adenosine

A solution of 2'-*O*-(propylphthalimide)adenosine (8.8 g, 19 mmol), 95% ethanol (400 ml) and hydrazine (10 ml, 32 mmol) was stirred for 16 hrs at room temperature. The reaction mixture was filtered and filtrate concentrated *in vacuo*. Water (150 ml) was added and acidified with acetic acid to pH 5.0. The aqueous solution was extracted with EtOAc (2 x 30 ml) and the aqueous phase was concentrated *in vacuo* to afford 7.1 g (95%) of the titled compound as a HOAc salt. ¹H NMR (200 MHz, DMSO-d₆) δ 1.70 (m, 2H, CH₂), 2.76 (m, 2H, CH₂NH₂), 3.55-3.67 (m, 4H, C_{5'}CH₂, OCH₂), 3.00 (q, 1H, C_{4'}H), 4.30 (q, 1H, C_{3'}H), 4.47 (t, 1H, C_{2'}H), 6.0 (d, 1H, C_{1'}H), 7.39 (s, 2H, NH₂) and 8.16 (s, 1H, C₂H).

### EXAMPLE 80

### 2'-O-[3-(N-trifluoroacetamido)propyl]adenosine

A solution of 2'-*O*-(propylamino)adenosine in methanol (50 ml) and triethylamine (15 ml, 108 mmol) was treated with ethyl trifluoroacetate (18 ml, 151 mmol). The reaction was stirred for 16 hrs and then concentrated *in vacuo* and the resultant gum chromatographed on silica gel (9/1, EtOAc/MeOH) to give the title 3.3 g of the compound (54%). m.p. 200-201°C (CH₃CN). ¹H NMR (200 MHz, DMSO-d₆) δ 1.7 (m, 2H, CH₂), 3.2 (m, 2H, CH₂NHCO), 3.37-3.67 (m, 4H, C_{5'}CH₂, OCH₂), 4.00 (q, 1H, C_{4'}H), 4.35 (q, 1H, C_{3'}H), 4.49 (t, 1H, C_{2'}H), 5.26 (d, 1H, C_{3'}OH), 5.43 (t, 1H, C_{5'}OH), 6.02 (d, 1H, C_{1'}H), 7.38 (s, 2H, NH₂), 8.16 (s, 1H, C₂H), 8.40 (s, 1H, C₈H), 9.34. (s, 1H, NHCO). ¹⁹F NMR (200 MHz, DMSO) δ -108.76. Anal. Calcd. for C₁₅H₁₉F₃N₆O₅: C, 42.86; H, 4.56; N, 19.99. Found: C, 43.10; H, 4.52; N, 20.03.

### EXAMPLE 81

### N⁶-(Dibenzoyl) -2'-O-[3-(N-trifluoroacetamido)propyl]adenosine

2'-*O*-[3-(N-trifluoroacetamido)propyl]adenosine is treated as per Example 76 using a Jones modification wherein tetrabutylammonium fluoride is utilized in place of ammonia hydroxide in the work up. The crude product was purified using silica gel chromatography (EtOAc→EtOAc/MeOH 1/1) to give the title compound. ¹H NMR (200 MHz, DMSO-d₆) δ 1.7 (m, 2H, CH₂), 3.2 (m, 2H, CH₂NHCO), 3.45-3.68 (m, 4H, C₅,CH₂, OCH₂), 4.03 (q, 1H, C_{4'}H), 4.37 (q, 1H, C_{3'}H), 4.53 (t, 1H, C_{2'}H), 5.18 (d, 1H, C_{3'}OH), 5.34 (d, 1H, C₃,OH), 6.17 (d, 1H, C_{1'}H), 7.45-7.83 (m, 1H, Ar), 8.73 (s, 1H, C₂H), 8.90 (s, 1H, C₈H), 9.37. (s, 1H, NHCO). ¹⁹F NMR (200 MHz, DMSO) δ -108.76. Anal. Calcd. for C₂₈H₂₇F₃N₆O₇: C, 55.41; H, 4.33; N, 13.37. Found: C, 55.16 H, 4.24; N, 13.09.

### EXAMPLE 82

### N⁶-(Dibenzoyl)-5'-O-(dimethoxytrityl)-2'-O-[3- (N-trifluoroacetamido)propyl]adenosine

4,4'-Dimethoxytrityl chloride (3.6 g, 10.0 mmol.) was added to a solution of N⁶-(dibenzoyl)-2'-*O*-[3-(N-trifluoroacetamido)propyl)adenosine in pyridine (100 ml) at room temperature and stirred for 16 hrs. The solution was concentrated *in vacuo* and chromatographed on silica gel (EtOAc/TEA 99/1) to give the title compound. ¹H NMR (200 MHz, DMSO-d₆) δ 1.74 (m, 2H, CH₂), 3.23 (m, 2H, CH₂NHCO), 3.60-3.73 (m, 10H, ArOCH₃, C_{5'}CH₂, OCH₂), 4.09 (m, 1H, C_{4'}H), 4.51 (q, 1H, C_{3'}H), 4.66 (t, 1H, C_{2'}H), 5.34 (d, 1H, C_{3'}OH), 6.17 (d, 1H, C_{1'}H), 6.80-7.83 (m, 23H, Ar), 8.64 (s, 1H, C₂H), 8.78 (s, 1H, C₈H), 9.35. (s, 1H, NHCO). ¹⁹F NMR (200 MHz, DMSO) δ -108.76. Anal. Calcd. for C₄₉H₄₅F₃N₆O₉: C, 68.36; H, 5.15; N, 9,76. Found: C, 68.16 H, 5.03; N, 9.43.

### EXAMPLE 83

### N⁶-(Dibenzoyl)-5'-O-(dimethoxytrityl)-2'-O-[3-(N-trifluoroacetamido)propyl]-3'-O-(N,N-diisopropyl-β-cyanoethyl)adenosine Phosphoramidite

A solution of N⁶-(dibenzoyl)-5'-*O*-(dimethoxytrityl)-2'-*O*-[3-(N-trifluoroacetamido)propyl]adenosine in dry CH₂Cl₂ was treated with bis-N,N-diisopropylamino cyanoethyl phosphite (1.1 eqiv) and N,N-diisopropylaminotetrazolide (catalytic amount) at room temperature for 16 hrs. The reaction was concentrated *in vacuo* and chromatographed on silica gel (EtOAc/hexane/TEA 6/4/1) to give 0.5 g of the title compound. ³¹P NMR (200 MHz, CD₃CN) 150.87; (200 MHz, CDCL₃) 150.55, 150.83. ¹⁹F NMR (200 MHz, CD₃CN) 107.14, (200 MHz, CDCl₃) 102.67.

### EXAMPLE 84

### 2'-O-(Butylphthalimide)adenosine

The title compound is prepared as per example 75, using N-(4-bromobutyl)phthalimide in place of the 1-bromopropane. Chromatography on silica gel (EtOAC-MeOH) gives the title compound as a white solid. M.P. 199-200°C. Anal. Calcd. C₂₂H₂₄N₆O₆ : C, 56.42; H, 5.16; N, 17.94. Found: C, 56.31; H, 5.04; N, 17.95.

The 3' compound can be separated from the mother liquors as per Example 75 above. ¹H NMR (200 MHz, DMSO-d₆) δ 5.88 (d, 1H, C_{1'}H).

### EXAMPLE 85

### 2'-O-(Butylphthalimide-N⁶-benzoyladenosine)

Benzoylation of 2'-*O*-(butylphthalimide)adenosine as per Example 76 gave the title compound. ¹H NMR (200 MHz, DMSO-d₆) δ 6.18 (d, 1H, C_{1'}H). Anal. Calcd. C₂₉H₂N₆O₇: C, 60.83; H, 4.93; N, 14.68. Found: C, 60.48; N, 14.41.

### EXAMPLE 86

### 2'-O-(Butylphthalimide)-5'-O-dimethoxytrityl-N⁶-benzoyladenosine

The title compound was prepared from 2'-O-(butylphthalimide)-N⁶-benzoyladenosine as per Example 77. ¹H NMR (200 MHz, DMSO-d₆) δ 6.20 (d, 1H, C₁,H). Anal. Calcd. for C₅₀H₄₆N₆O₉: C, 68.64; H, 5.29; N, 9.60. Found: C, 68.47; H, 5.12; N, 9.37.

### EXAMPLE 87

### N⁶-Benzoyl-5'-O-(dimethoxytrityl)-2'-O-(butylphthalimide)-3'-O-(N,N-diisopropyl-β-cyanoethyl)adenosine Phosphoramidite

The title compound was prepared from 2'-*O*-(butylphthalimide)-5'-*O*-dimethoxytrityl-N⁶-benzoyladenosine as per Example 78. ³¹P NMR (CD₃CN) S 150.88, 151.22.

### EXAMPLE 88

### 2'-O-(Pentylphthalimido)adenosine

The title compound was prepared as per Example 75, using N-(5-bromopentyl)phthalimide. The crude material from the extraction was chromatographed on silica gel using CHCl₃/MeOH (95/5) to give a mixture of the 2' and 3' isomers. The 2' isomer was recrystallized from EtOH/MeOH 8/2. The mother liquor was rechromatographed on silica gel to afford the 3' isomer.

2'-*O*-(Pentylphthalimido)adenosine: M.P. 159-160°C. Anal. Calcd. for C₂₃H₂₄N₆O₅: C, 57.26; H, 5.43; N, 17.42. Found: C, 57.03; H, 5.46; N, 17.33.

3'-*O*-(Pentylphthalimido)adenosine: ¹H NMR (DMSO-d₆) δ 5.87 (d, 1H, H-1').

### EXAMPLE 89

### 2'-O-(Pentylphthalimido)-N⁶-benzoyladenosine

Benzoylation of 2'-*O*-(pentylphthalimido)adenosine was achieved as per the procedure of Example 76 to give the title compound. ¹H NMR (DMSO-d₆) : 6.10 (d, 1H, C₁, H). Anal. Calcd. for C₃₀H₃₀N₆O₇: C, 61.43; H, 5.16; N, 14.33. Found: C, 61.51; H, 4.97; N, 14.10.

### EXAMPLE 90

### 2'-O-(Pentylphthalimido)-5'-O-(dimethoxytrityl)-N⁶-benzoyladenosine

The title compound was prepared from 2'-*O*-(pentylphthalimide)-N⁶-benzoyladenosine as per the procedure of Example 77. Chromatography on silica gel (ethylacetate, hexane, triethylamine), gave the title compound. Anal. Calcd. for C₅₁H₄₈N₆O₉: C, 68.91; H, 5.44; N, 9.45. Found: C, 68.65; H, 5.45; N, 9.61.

### EXAMPLE 91

### N⁶-Benzoyl-5'-O-(dimethoxytrityl)-2'-O-(pentylphthalimido)-3'-(N,N-diisopropyl-β-cyanoethyl) Phosphoramidite

The title compound was prepared from 2'-O-(pentylphthalimide)-5'-*O*-(dimethoxytrityl)-N⁶-benzoyladenosine as per the procedure of Example 78 to give the compound as a white foam.

### EXAMPLE 92

### 2-Amino-2'-O-(propylphthalimide)adenosine and 2-Amino-3'-O-(propylphthalimide)adenosine

To a solution of 2-aminoadenosine (75 g, 281 mmol) in dry dimethylformamide (2000 ml) at room temperature was added sodium hydride 60% (13.0 g, 325 mmol). After one hour N-(3-bromopropane)phthalimide (86.6 g, 3.23 mmol) was added and the temperature was raised to 40°C and held for 16 hrs. A further aliquot of sodium hydride 60% (6.2 g, 155 mmol) and N-(3-bromopropyl)phthalimide (40 g, 150 mmol) was added and heating was continued for an additional 16 hrs. The reaction mixture was cooled, quenched with MeOH and concentrated *in vacuo*. The resultant gum was chromatographed on silica gel (CHCl₃/MeOH 9/1) to give 53.3 g of the 2' isomer (42%). Those fractions containing the 3' isomer were crystallized from EtOAc/MeOH to yield 20 g of the 3' isomer (16%).

2-Amino-2'-*O*-(propylphthalimide)adenosine: ¹H NMR (200 MHz, DMSO-d₆) δ 1.82 (m, 2H, CH₂), 3.35-3.63 (m, 4H, OCH₂, C₅,H₂), 3.91 (m, 1H, C₄,H), 4.26 (m, 1H, c₃,H), 4.39 (m, 1H, C_{2'}H), 5.10 (d, 1H, C_{3'}OH), 5.44 (t, 1H, C_{5'}OH), 5.77 (s, 2H, NH₂), 5.81 (d, 1H, C_{1'}H), 6.79 (bs, 2H, NH₂), 7.85 (bs, 4H, Ar) and 7.97 (s, 1H, C₈H). Anal. Calcd. for C₂₁H₂₃N₇O₆: C, 53.72;, H, 4.94; N, 20.88. Found: C, 53.70; H, 4.48; N, 20.56.

2-Amino-3'-*O*-(propylphthalimide)adenosine: m.p. 222-224°C, ¹H NMR (DMSO-d₆) δ 5.69 (d, 1H, H-1').

### EXAMPLE 93

### 2'-O-(Propylphthalimide)guanosine

2-Amino-2'-*O*-(propylphthalimide)adenosine (38.6 g, 82 mmol) was dissolved in dimethylsulfoxide (DMSO, 1000 ml). Sodium phosphate (0.1 M, 63 ml) and Tris buffer (0.5 M, 1200 ml) were added with stirring. The solution was adjusted to pH 7.0 with phosphoric acid. Adenosine deaminase (Sigma, 1.7 g) was added and the temperature raised to 35°C. The solution was stirred for 4 days with an additional aliquot of adenosine deaminase (0.2 g) added every 24 hrs. The solution was maintained at pH 7.0 with the addition of phosphoric acid. The reaction was cooled, filtered and concentrated *in vacuo*. The resultant gum was crystallized from MeOH to give 31.6 of the title compound (82%). ¹H NMR (200 MHz, DMSO-d₆) δ 1.82 (m, 2H, CH₂), 3.45 (m, 4H, OCH₂, C_{5'}H₂), 3.85 (m, 1H, C_{4'}H), 4.26 (m, 2H, C₃,H,C_{2'}H), 5.09 (M, 2H, C_{3'} OH, C_{5'}OH), 5.80 (d, 1H, C_{1'}H), 6.47 (s, 2H, NH₂), 7.84 (s, 4H, Ar), 7.97 (s, 1H, C₈H) AND 10.65 (s, 1H, ArNH). Anal. Calcd. for C₂₁H₂₁N₆O₇: C, 53.72;, H, 4.94; N, 20.88. Found: C, 54.02; H, 4.73; N, 20.53.

### EXAMPLE 94

### 2'-O-(Propylamino)guanosine

2'-*O*-(Propylphthalimide)guanosine was treated as per the procedure of Example 79 in water/methanol as the solvent to give the title compound in 91% yield. ¹H NMR (200 MHz, DMSO-d₆) δ 1.80 (m, 2H, CH₂), 3.35-3.70 (m, 4H, OCH₂, C_{5'}H₂), 3.85 (m, 1H, C_{4'}H), 4.27 (m, 2H, C_{3'}H, C_{2'}H), 5.10 (m, 2H, C_{3'} OH, C_{5'}OH), 5.78 (d, 1H, C_{1'}H), 6.49 (s, 2H, NH₂) and 7.97 (s, 1H, C₈H).

### EXAMPLE 95

### 2'-O-[3-(N-trifluoroacetamido)propyl]guanosine

2'-*O*-(Propylamino)guanosine was treated as per the procedure of Example 80 to give the title compound.

### EXAMPLE 96

### N²-(Dibenzoyl)-2'-O-[3-(N-trifluoroacetamido)propyl]guanosine

2'-*O*-[3-(N-trifluoroacetamido)propyl]guanosine was treated as per the procedure of Example 81 to give the title compound.

### EXAMPLE 97

### N²-(Dibenzoyl) -5'-O-(dimethoxytrityl)-2'-O-[3-(N-trifluoroacetamido)propyl]guanosine

N²-(Dibenzoyl)-2'-*O*-[3-(N-trifluoroacetamido)propyl]-guanosine was treated as per the procedure of Example 82 to give the title compound.

### EXAMPLE 98

### N²-(Dibenzoyl)-5'-O-(dimethoxytrityl)-2'-O-[3- (N-trifluoroacetamido)propyl]-3'-O-(N,N-diisopropyl-β-cyanoethyl)guanosine Phosphoramidite

N²-(Dibenzoyl)-5'-*O*-(dimethoxytrityl)-2'-*O*-[3-(N-trifluoroacetamido)propyl]guanosine was treated as per the procedure of Example 83 to give the title compound. ³¹P NMR (CDCl₃) 150.49, 150.91. ¹⁹F NMR (CDCl₃) 102.71, 102.75.

### EXAMPLE 99

### 2'-O-(Propylphthalimide)uridine and 3'-O-(Propylphthalimide)uridine

A solution of uridine-tin complex (prepared as per Example 3) (48.2 g, 115 mmol) in dry DMF (150 ml) and N-(3-bromopropyl)phthalimide (46 g, 172 mmol) was heated at 130°C for 6 hrs. The crude product was chromatographed directly on silica gel CHCl₃/MeOH 95/5. The isomer ration of the purified mixture was 2'/3' 81/19. The 2' isomer was recovered by crystallization from MeOH. The filtrate was rechromatographed on silica gel using CHCl₃→CHCl₃/MeOH (95/5) gave the 3' isomer as a foam.

2'-*O*-(Propylphthalimide)uridine: Analytical sample recrystallized from MeOH, m.p. 165.5-166.5°C, ¹H NMR (200 MHz, DMSO-d₆) δ 1.87 (m, 2H, CH₂), 3.49-3.65 (m, 4H, C_{2'}H), 3.80-3.90 (m, 2H, C₃,H₁C_{4'}H), 4.09(m, 1H, C_{2'}H), 5.07 (d, lh, C_{3'}OH), 5.16 (m, 1H, C_{5'}OH), 5.64 (d, 1H, CH=), 7.84 (d, 1H, C_{1'}H), 7.92 (bs, 4H, Ar), 7.95 (d, 1H, CH=) and 11.33 (s, 1H, ArNH). Anal. C₂₀H₂₁N₃H₈, Calcd. C, 55.69; H, 4.91;, N, 9.74. Found, C, 55.75; H, 5.12; N, 10.01.

3'-*O*-(Propylphthalimide)uridine: ¹H NMR (DMSO-d₆) δ 5.74 (d, 1H, H-1').

### EXAMPLE 100

### 2'-O-(Propylamino)uridine

The title compound was prepared as per the procedure of Example 79 (90% yield). ¹H NMR (200 MHz, DMSO-d₆) δ 1.80 (m, 2H, CH₂), 3.57-3.66 (m, 4H, OCH₂, C_{5'}H₂), 3.88 (m, 2H, C₃H, C_{4'}H), 4.15 (m, 1H, C_{2'}H), 5.64 (d, 1H, CH=), 5.82 (d, 1H, C_{1'}H), 7.02 (bs, 2H, NH₂) and 8.00 (d, 1H, C₈H).

### EXAMPLE 101

### 2'-O-[3-(N-trifluoroacetamido)propyl]uridine

2'-*O*-(Propylamino)uridine was treated as per the procedure of Example 80 to give the title compound. ¹H NMR (200 MHz, DMSO-d₆) δ 1.75 (m, 2H, CH₂), 3.22-3.35 (m, 2H, CH₂NH), 3.38-3.67 (m, 4H, OCH₂, C_{5'}H₂), 5.16 (m, 2H, C_{3'} OH, C_{5'}OH), 5.67 (d, 1H, CH=), 7.95 (d, 1H, CH=), 9.34 (bs, 1H, NHCO) and 11.36 (s, 1H, ArNH). ¹⁹F NMR (200 MHz, DMSO) 108.76. Anal. Calcd. for C₁₄H₁₈F₃N₃O₇: C, 42.31; H, 4.56; N, 10.60. Found: 42.56; H, 4.61; N, 10.25.

### EXAMPLE 102

### 5'-O-(dimethoxytrityl)-2'-O-[3-(N-trifluoroacetamido)propyl]-uridine

2'-*O*-[3-(N-trifluoroacetamido)propyl]uridine was treated as per the procedure of Example 82 to give the title compound. ¹H NMR (200 MHz, DMSO-d₆) δ 1.95 (m, 2H, CH₂), 3.42-3.80 (m, 12H, ArOCH₃, OCH₂, C_{5'}H₂), 4.03 (m, 2H, C_{2'}H), 4.45 (m, 1H, C_{3'}OH), 5.30 (d, 1H, CH=), 5.91 (s, 1H, C₁,H), 6.83-7.43 (m, 13H, ArH), 7.90 (bs, 1H, NHCO), 8.10 (d, 1H, CH=) and 10.3 (s, 1H, ArNH). ¹⁹F NMR (200 MHz, DMSO) 102.75.

### EXAMPLE 103

### 5'-O-(Dimethoxytrityl)-2'-O-[3-(N-trifluoroacetamido)propyl]-3'-O-(N,N-diisopropyl-β-cyanoethyl)uridine Phosphoramidite

5'-*O*-(Dimethoxytrityl)-2'-*O*-[3-(N-trifluoroacetamido)-propyl]uridine was treated as per the procedure of Example 83 to give the title compound. ³¹P NMR (200 MHz, CD₃CN) 154.87, 155.62. ¹⁹F NMR (200 MHz, CD₃CN) 107.19.

### EXAMPLE 104

### 2'-O-(Propylphthalimide)cytidine and 3'-O-(Propylphthalimide)-cytidine

The title compounds were prepared as per the procedure of Example 75.

2'-*O*-(propylphthalimide)cytidine: ¹H NMR (200 MHz, DMSO-d₆) δ 5.82 (d, 1H, C_{1'}H).

3'-*O*-(propylphthalimide)cytidine: ¹H NMR (200 MHz, DMSO-d₆) δ 5.72 (d, 1H, C_{1'}H).

### EXAMPLE 105

### 2'-O-(Propylamino)cytidine

2'-O-(Propylphthalimide)cytidine was treated as per the procedure of Example 79 to give the title compound. ¹H NMR (200 MHz, DMSO-d₆) δ 5.82 (d, 1H, C_{1'}H).

### EXAMPLE 106

### 2'-O-[3-(N-trifluoroacetamido)propyl]cytidine

2'-*O*-(Propylamino)cytidine was treated as per the procedure of Example 80 to give the title compound. ¹⁹F NMR (DMSO-d₆) 108.73.

### EXAMPLE 107

### N⁴-(Benzoyl)-2'-O-[3-(N-trifluoroacetamido)propyl]cytidine

2'-*O*-[3- (N-trifluoroacetamido) propyl] cytidine was treated as per the procedure of Example 81 to give the title compound. ¹⁹F NMR (DMSO-d₆) 108.79.

### EXAMPLE 108

### N⁴-(Benzoyl)-5'-O-(dimethoxytrityl) -2'-O-[3- (N-trifluoroacetamido)propyl]cytidine

N⁴-(Benzoyl)-2'-*O*-[3-(N-trifluoroacetamido)propyl]-cytidine was treated as per the procedure of Example 82 to give the title compound.

### EXAMPLE 109

### N⁴-(Benzoyl)-5'-O-(dimethoxytrityl)-2'-O-[3-(N-trifluoroacetamido)propyl]-3'-O-(N,N-diisopropyl-β-cyanoethyl)cytidine

**Phosphoramidite** N⁴-(Benzoyl)-5'-*O*-(dimethoxytrityl)-2'-*O*-[3-(N-trifluoroacetamido)propyl]cytidine was treated as per the procedure of Example 83 to give the title compound. ³¹P NMR (CDCl₃) 150.03, 151.36. ¹⁹F NMR (CDCl₃) 102.76.

### EXAMPLE 110

### 2'-O-[3-(Imidizo-1-yl)propyl]adenosine

The title 2'-*O*-substituted compound was prepared as per the procedure of Example 75 using 1-(4-bromopropyl)imidazole. ¹H NMR (200 MHz, DMSO-d₆) δ 6.02 (d, 1H, C_{1'}H).

The corresponding 3'-*O*- substituted compound can be separated from the mother liquors as per Example 75. ¹H NMR (200 MHz, DMSO-d₆) δ 5.91 (d, 1H, C_{1'}H).

### EXAMPLE 111

### 2'-O-[4-(Imidizo-1-yl)butyl)]adenosine and 3'-O-[4-(Imidizo-1-yl)butyl]adenosine

A solution of adenosine (6.97 g, 26 mmol) in dry DMF (250 ml) was treated with NaH 60% (3.9 g, 97 mmol) for 15 minutes. N-(4-Bromobutyl)imidazoline acetate salt (9.4 g, 2.1 equiv. HOAc, 29 mmol) was added and the reaction mixture stirred at room temperature for 2 days. The reaction was quenched (MeOH) and concentrated *in vacuo* to a semi-solid. The solid was dissolved in water (250 ml) and extracted with EtOAc (2 x 100 ml). The aqueous phase was concentrated *in vacuo* and chromatographed on silica gel using EtOAc/MeOH (6/4) to give a mixture of 2' and 3' isomers (70/30, 62%). Crystallization of this solid from EtOAc/MeOH 8/2 gave the 2' isomer. Concentration of the mother liquors and subsequent crystallization from MeOH afforded the 3' isomer in 12% yield.

2'-*O*-[4-(Imidizo-1-yl)butyl]adenosine: ¹H NMR (200 MHz, DMSO-d₆) δ 6.01 (d, 1H, C_{1'}H).

3'-*O*-[4-(Imidizo-1-yl)butyl]adenosine: m.p. 183-184°C. ¹H NMR (DMSO-d₆) δ 5.89 (d, 1H, H-1').

### EXAMPLE 112

### 2'-O-[4-(Imidizo-1-yl)butyl]-N⁶-benzoyladenosine

The title compound was prepared 2'-*O*-[4-(imidizo-1-yl)-butyl]adenosine as per Example 76.

### EXAMPLE 113

### 2'-O-[4-(Imidizo-1-yl)butyl]-5'-O-dimethoxytrityl-N⁶-benzoyladenosine

The title compound was prepared from N⁶-benzoyl-2'-*O*-[4-(imidizo-1-yl)butyl]adenosine as per Example 77.

### EXAMPLE 114

### N⁶-Benzoyl-5'-O-(dimethoxytrityl)-2'-O-[4-(imidizo-1-yl)-(butyl]-3'-O-(N,N-diisopropyl-β-cyanoethyl)adenosine Phosphoramidite

The title compound was prepared from 2'-*O*-[4-(imidizo-1-yl)butyl]-5'-*O*-dimethoxytrityl-N⁶-benzoyladenosine as per Example 78. ³¹P NMR (CDCl₃) 150.48, 151.06.

### EXAMPLE 115

### 2-Amino-2'-O-[4-(imidizo-1-yl)butyl]adenosine and 2-Amino-3'-O-[4-(imidizo-1-yl)butyl]adenosine

The title compounds are prepared as per Example 111 with the substitution of 2-aminoadenosine for adenosine. The isomer ratio of 2'/3' was 74/26.

2-Amino-2'-*O*-[4-(imidizo-1-yl)butyl]guanosine: ¹H NMR (200 MHz, DMSO-d₆) δ 5.84 (d, 1H, C_{1'}H).

2-Amino-3'-*O*-[4-(imidizo-1-yl)butyl]guanosine: ¹H NMR (200 MHz, DMSO-d₆) δ 5.68 (d, 1H, C_{1'}H).

### EXAMPLE 116

### 2'-O-[4-(Imidazo-1-yl)butyl]guanosine

In the manner described in Example 93, 2-amino-2'-*O*-[4-(imidazo-1-yl)butyl]adenosine in sodium phosphate (0.1 M) and Tris buffer (0.5 M) at pH 7.0 is deaminated with adenosine deaminase (Sigma) at 35°C to give the title compound.

### EXAMPLE 117

### N²-(Dibenzoyl) -2'-O-[4-(imidazo-1-yl)butyl]guanosine

2'-*O*-[4-(Imidazo-1-yl)butyl]guanosine was treated as per the procedure of Example 81 to give the title compound.

### EXAMPLE 118

### N²-(Dibenzoyl)-5'-O-(dimethoxytrityl)-2'-O-[4-(imidazo-1-yl)butyl]guanosine

N²-(Dibenzoyl)-2'-*O*-[4-(imidizo-1-yl)butyl]guanosine was treated as per the procedure of Example 82 to give the title compound.

### EXAMPLE 119

### N²-(Dibenzoyl)-5'-O-(dimethoxytrityl)-2'-O-[4-(imidazo-1-yl)butyl]-3'-O-(N,N-diisopropyl-β-cyanoethyl)guanosine Phosphoramidite

N²-(Dibenzoyl) -5'-*O*-(dimethoxytrityl)-2'-*O*-[4- (imidazo-1-yl)butyl]guanosine was treated as per the procedure of Example 83 to give the title compound. ³¹P NMR (CDCl₃) 150.67, 150.80

### EXAMPLE 120

### 2'-O-[4- (Imidizo-1-yl)butyl]cytidine and 3'-O-[4-(imidizo-1-yl)-butyl]cytidine

The title compounds were prepared as per the procedure of Example 111 with the substitution of cytidine for adenosine. The crude product was chromatographed on silica gel using EtOAc/MeOH/HOAc. The isomer ratio of 2'/3' was 60/40.

2'-*O*-[4-(Imidizo-1-yl)butyl]cytidine: ¹H NMR (200 MHz, DMSO-d₆) δ 5.88 (d, 1H, C_{1'}H).

3'-*O*-[4-(Imidizo-1-yl)butyl]cytidine: ¹H NMR (DMSO-d₆) δ 5.78 (d, 1H, H'1')

### EXAMPLE 121

### 2'-O-[4-(Imidizo-1-yl)butyl]-N⁴-benzoylcytidine

The title compound was prepared 2'-*O*-[4-(imidizo-1-yl)-butyl]cytidine as per Example 76.

### EXAMPLE 122

### 2'-O-[4-(Imidizo-1-yl)butyl]-5'-O-dimethoxytrityl-N⁴-benzoylcytidine

The title compound was prepared from N⁴-benzoyl-2'-*O*-[4-(imidizo-1-yl)butyl]cytidine as per Example 77.

### EXAMPLE 123

### N⁴-Benzoyl-5'-O-(dimethoxytrityl)-2'-O-[4-(imidizo-1-yl)-(butyl]-3'-O-(N,N-diisopropyl-β-cyanoethyl)cytidinePhosphoramidite

The title compound was prepared from 2'-*O*-[4-(imidizo-1-yl)butyl]-5'-*O*-dimethoxytrityl-N⁴-benzoylcytidine as per Example 78. ³¹P NMR (CDCl₃) 150.39, 150.92.

### EXAMPLE 124

### 3-Benzyloxymethyl-5-methyluridine

A solution of 5-methyluridine (51.6, 200 mmol) in DMF (100 ml) was treated with sodium hydride (60% oil, 9.9 g, 248 mmol). After 1 hr at room temperature, benzylchloromethyl ether (34.4 g, 220 mmol) was added dropwise and the reaction mixture heated to 35°C for 4 hrs. The reaction mixture was cooled, quenched with MeOH (5 ml), filtered and concentrated *in vacuo*. The resultant gum was partitioned between EtOAc/sat. NaCl, separated, dried and the organic phase concentrated whereupon the title compound crystallized. ¹H NMR (DMSO-d₆) δ 5.83 (d, 1H, C_{1'}-H).

### EXAMPLE 125

### 3-Benzyloxymethyl-5-methyl-2'-O-(4-chlorobutyl)uridine

A solution of 3-benzyloxymethyl-5-methyluridine (25.9 g, 68 mmol) was treated with NaH (60% oil, 7.9 g, 200 mmol) for 1 hr at room temp. 1-Bromo-4-chlorobutane (19.7 g, 115 mmol) was added slowly and the reaction stirred at room temp. for 16 hrs. The reaction was then quenched (MeOH), concentrated *in vacuo* and the residue chromatographed on silica gel CHCl₃/MeOH 95/5 to give the title compound in 41% yield. ¹H NMR (DMSO-d₆) δ 5.87 (d, 1H, C_{1'}H).

### EXAMPLE 126

### 3-Benzyloxymethyl-5-methyl-2'-O-[4-(imidazo-1-yl)butyl]uridine

To a solution of imidazole (6.8 g, 100 mmol) in DMF (150 ml) was added NaH (60% in oil, 4 g, 100 mmol). After 4 hrs, this suspension was added to a suspension of 3-benzyloxymethyl-5-methyl-2'-*O*-(4-chlorobutyl)uridine (8.7 g, 18 mmol), NaI (2.6 g) and DMF (200 ml). The reaction was stirred 16 hrs at room temp., quenched with MeOH, concentrated *in vacuo* and the residue chromatographed on silica gel CHCl₃/MeOH 85/15→50/50 to give the title compound in 31% yield. ¹H NMR (DMSO-d₆) δ 5.87 (d, 1H, C_{1'}H).

### EXAMPLE 127

### 2'-O-[4-(Imidazo-1-yl)butyl]-5-methyluridine

A solution of 3-benzyloxymethyl-5-methyl-2'-*O*-[4-(imidazo-1-yl)butyl]uridine (5.4 g, 10 mmol) in MeOH (200 ml) was hydrogenated with 20% palladium hydroxide (9.0 g) at 1 atmosphere H₂ for 16 hrs. The reaction was filtered through celite and the mother liquor concentrated to afford the product in 83% yield. ¹H NMR (DMSO-d₆) 6 5.86 (d, 1H, C_{1'}H).

### EXAMPLE 128

### 5'-O-(Dimethoxytrityl)-2'-O-[4-(imidazo-1-yl)butyl]-5-methyluridine

5-Methyl-2'-*O*-[4-(imidazo-1-yl)butyl]uridine was treated as per the procedure of Example 77 to give the title compound in 91% yield. ¹H NMR (DMSO-d₆) δ 5.86 (d, 1H, C_{1'}H).

### EXAMPLE 129

### 5'-O-(Dimethoxytrityl)-2'-O-[4-(imidazo-1-yl)butyl]-3'-O-(N,N-diisopropyl-β-cyanoethyl)-5-methyluridine Phosphoramidite

5'-*O*-(Dimethoxytrityl)-5-methyl-2'-*O*-[4-(imidazo-1-yl)butyl]uridine was treated as per the procedure of Example 78 to give the title compound in 83% yield. ¹H NMR (DMSO-d₆) δ 5.85 (d, 1H, C_{1'}H). ³¹P NMR (DMSO-d₆) 153.99, 154.45.

### Example 130

### Effect Of Derivatization On Melting Temperature

Melting temperatures (Tm) for a variety of modified oligonucleotides according to the invention were determined according the method set forth in Procedure B(1)(b). The resulting data is shown in Tables 5-8, wherein all listed oligonucleotides contain 2'-deoxy sugars and phosphodiester backbones unless otherwise indicated.

Table 5 shows changes in Tm that result by modifying the 2'-position of adenosine nucleotides to include a variety of substituent groups. For example, the first entry shows that the introduction of 2'-O-aminopropyl adenosine substituents at two adenosine nucleotides resulted in a heteroduplex having a Tm that is 1.13°C greater than the heteroduplex formed by an unmodified oligonucleotide having the same sequence.

Table 6 shows changes in Tm that result by modifying one or more 2'- and 3'-positions of the indicated parent oligonucleotides to include linkers and pyrene conjugates. These modifications were made to terminal and internal (*i.e*., non-terminal) positions within the oligonucleotide. The notation "PS" indicates the presence of a phosphorothioate backbone, and the notation "2'-5' linkage" indicates 2'-5' intersugar linkages. As can be seen from the first entry, the introduction of a 2'-aminopentoxy linker to a single, internal adenosine nucleotide of the parent oligonucleotide resulted in a Tm decrease of 3.1°C whereas the introduction of a 2'-pyrenyl-aminopentoxy conjugate resulted in a Tm increase of 3.3°C.

Tables 7a-c show changes in Tm that result by modifying one or more positions of the indicated parent oligonucleotides to include linkers and cholate or cholesterol conjugates. As can be seen from the first entry, the introduction of an aminolink linker at the 3'-position of a single terminal nucleotide of the parent phosphorothioate oligonucleotide resulted in a Tm increase of 1.3°C and the addition of a cholate group to the linker resulted in a further Tm increase of 1.1°C.

Table 8 shows changes in Tm that result by modifying one or more 3'-positions of the indicated parent oligonucleotides to include linkers and PEG conjugates. The entries under the heading "PEG vs Unmodified" represent preliminary findings. As can be seen, the first entry indicates that introduction of an aminolink linker at the 3'-position of a single terminal nucleotide of the parent phosphodiester oligonucleotide resulted in a Tm increase of 0.9°C and that the addition of a PEG 550 group to the linker did not change the Tm of the resulting product.

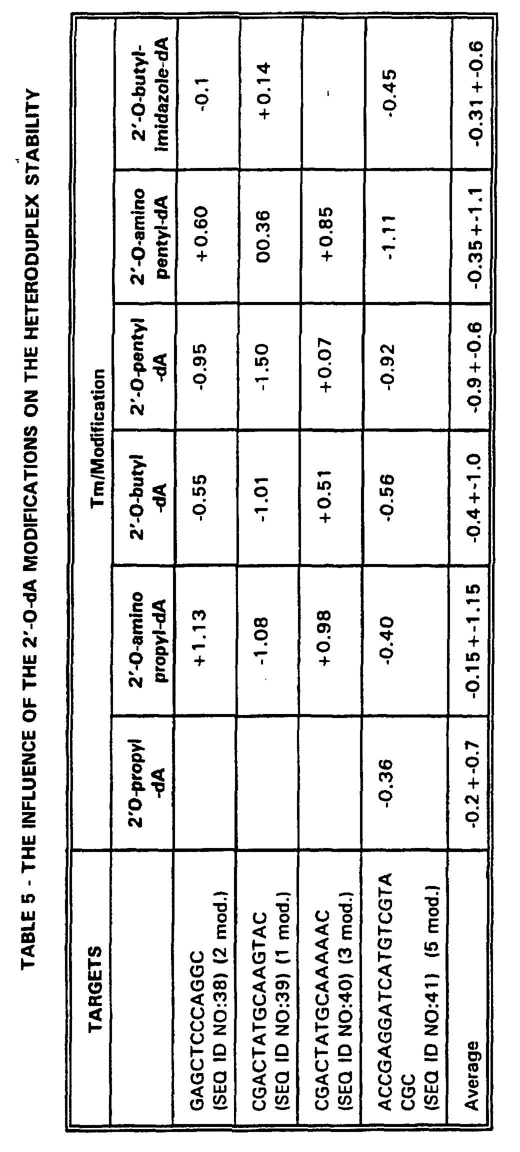

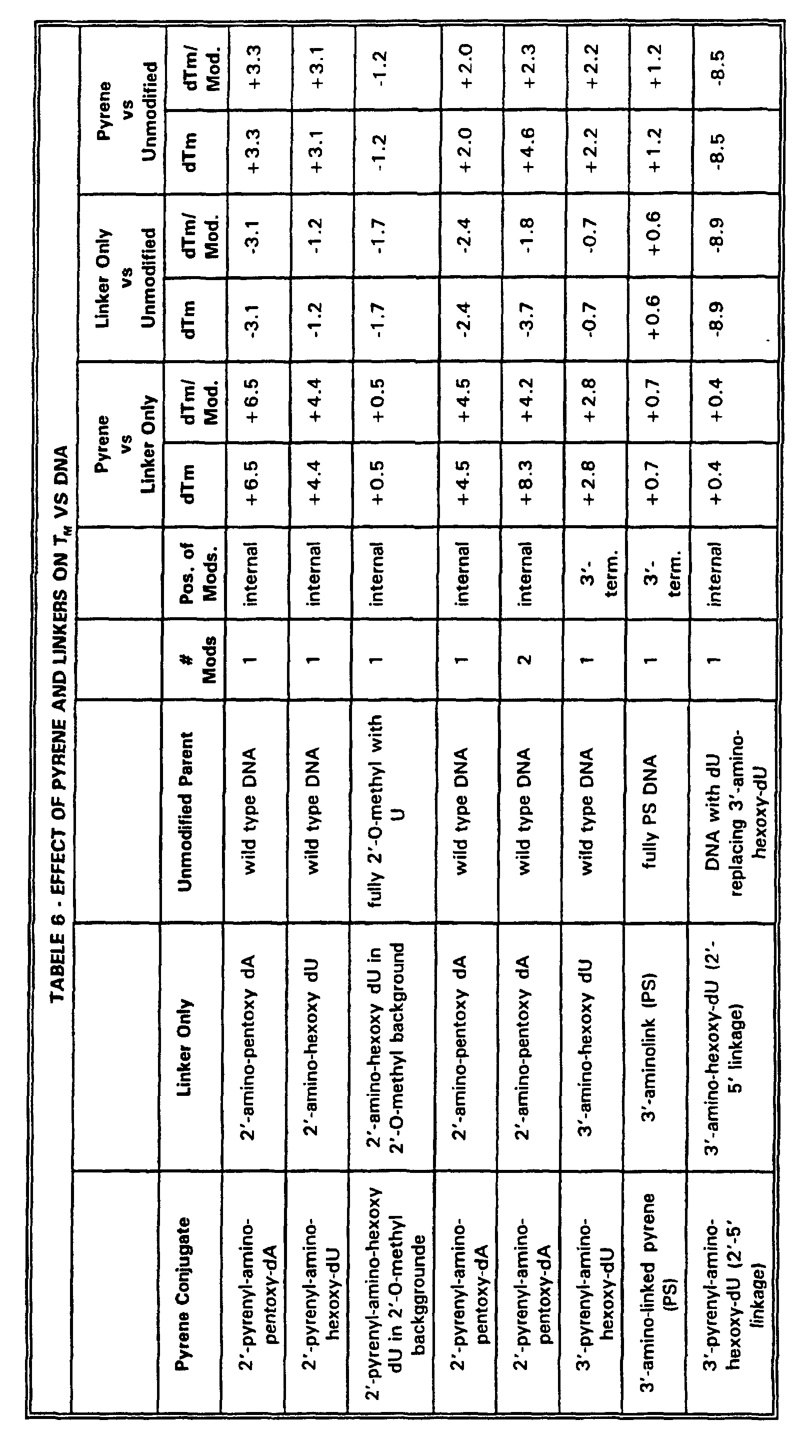

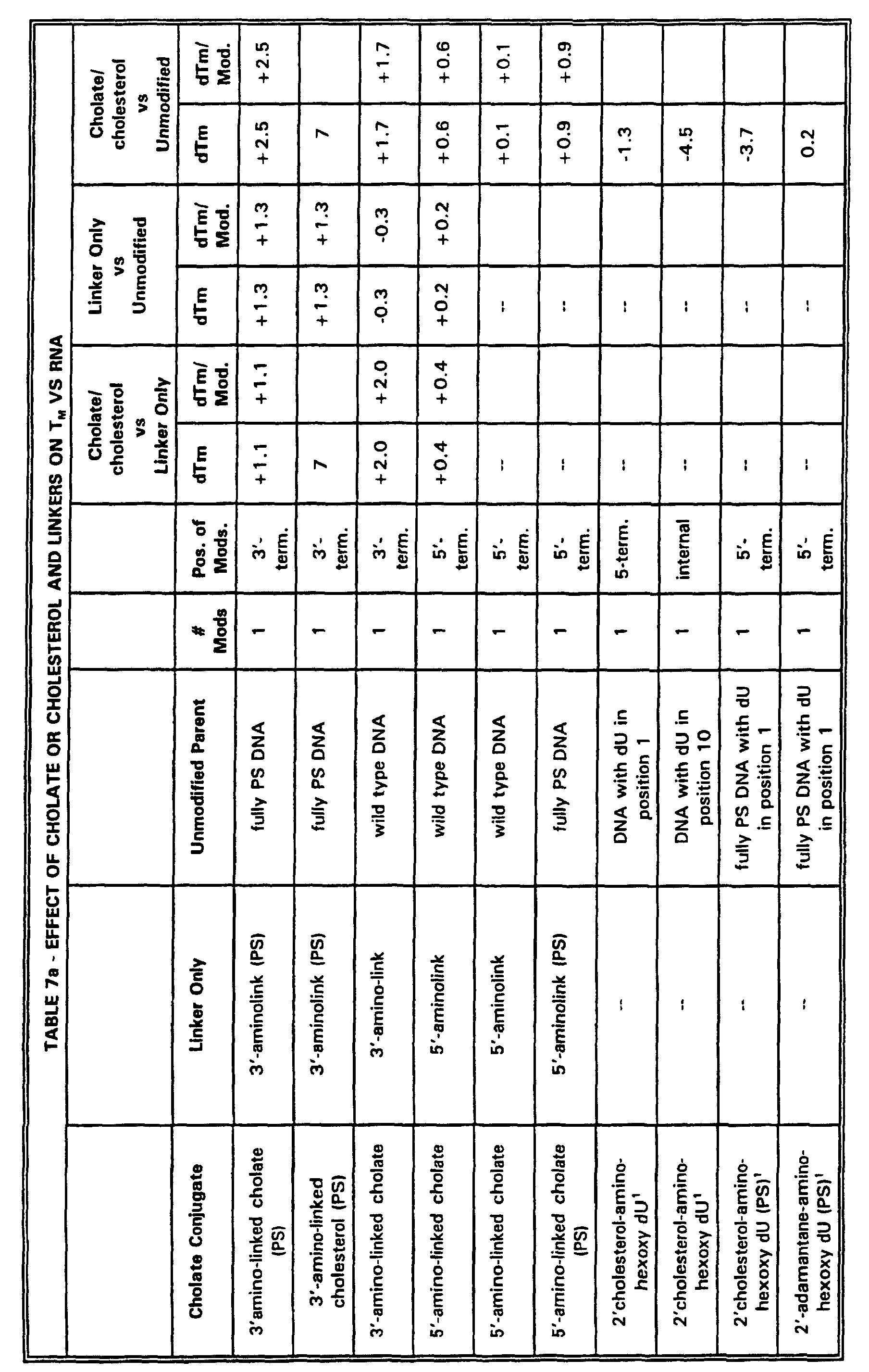

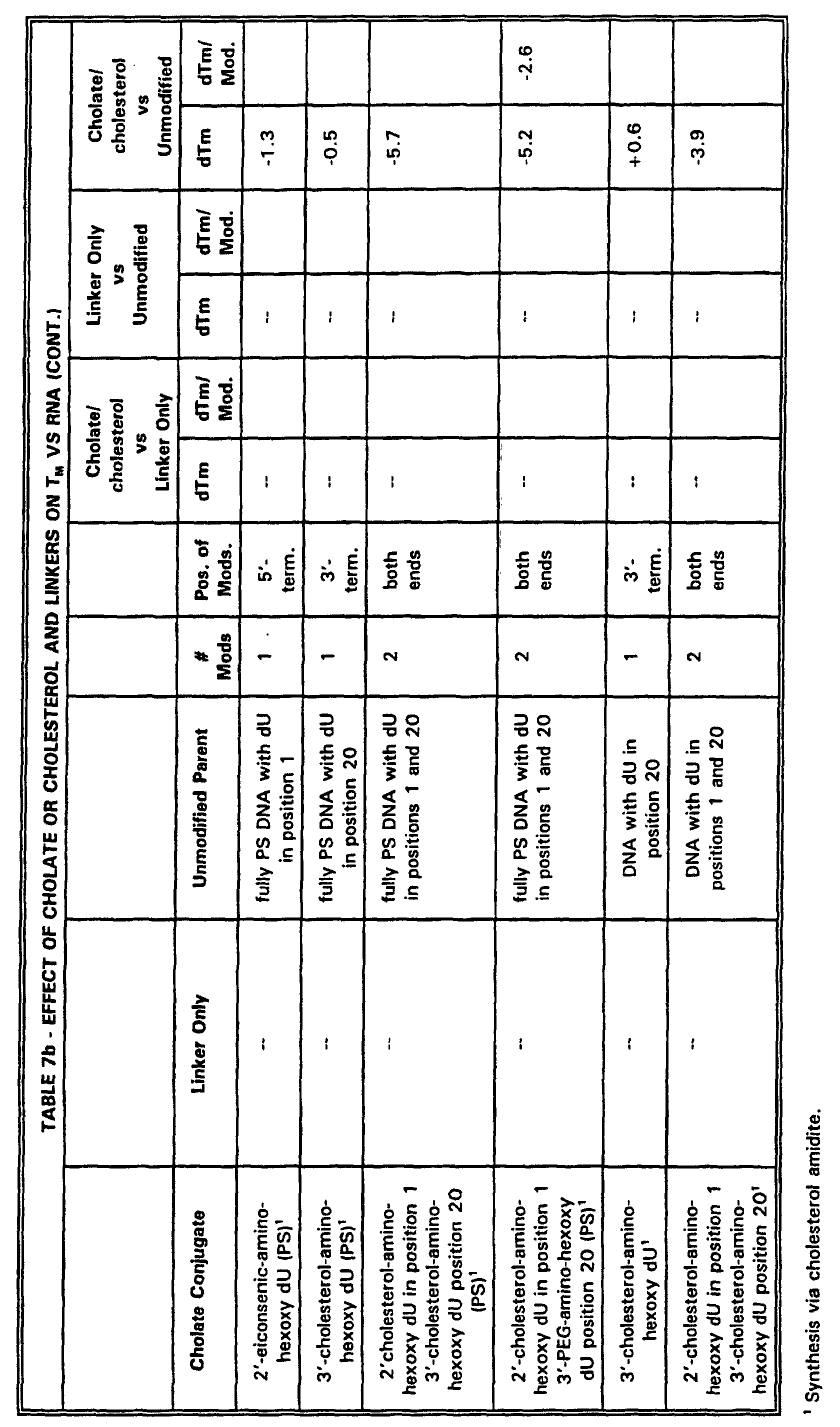

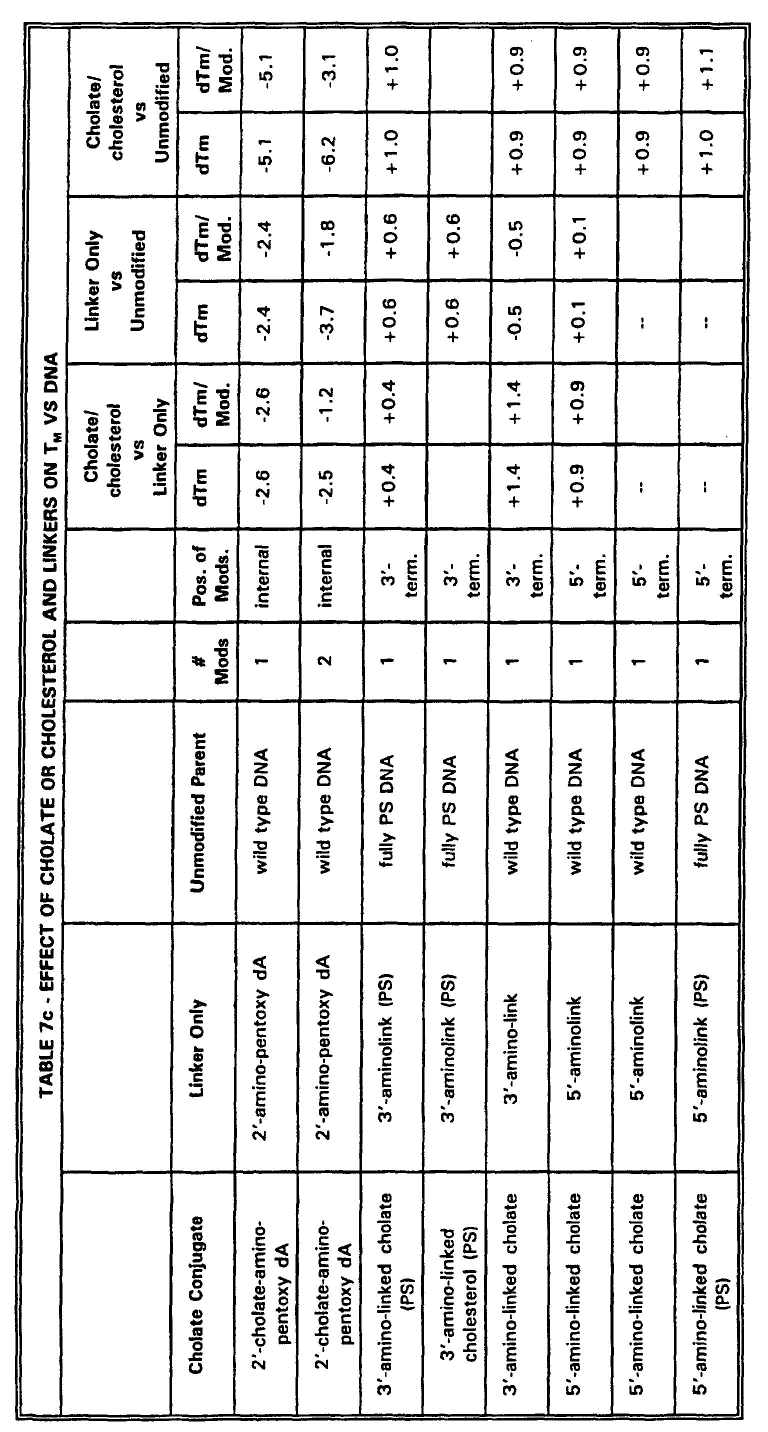

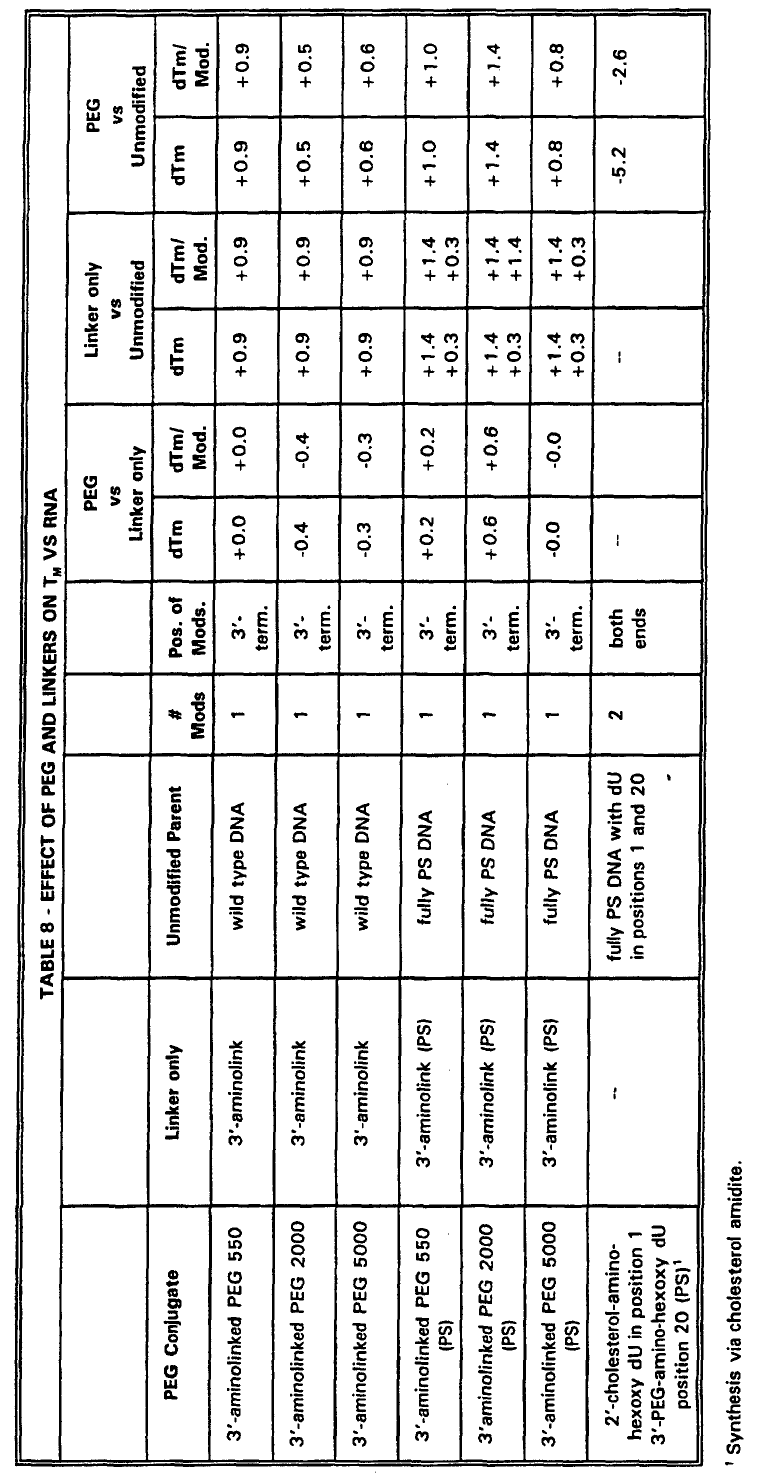

## Claims

1. A compound comprising a plurality of linked nucleosides, wherein each nucleoside includes a ribofuranosyl sugar portion and a base portion, and at least one of said nucleosides bears at a 3'-O-position or a 5'-O-position a substituent having formula:
-R_{A}-N(R₁ₐ)(R_{1b})
where:
R_{A} is alkyl having from 1 to 10 carbon atoms or (CH₂-CH₂-Q)ₓ;
R₁ₐ and R_{1b} are selected such that
R₁ₐ is H, R₂, an amine protecting group, or has formula C(X)-R₂, C(X)-R_{A}-R₂, C(X)-Q-R_{A}-R₂, or C(X)-Q-R₂,
R_{1b} is R₂, an amine protecting group, or has formula C(X)-R₂, C(X)-R_{A}-R₂, C(X)-Q-R_{A}-R₂, or C(X)-Q-R₂, or
R₁ₐ and R_{1b}, together, complete a cycloalkyl or cycloaryl moiety having two to six carbon atoms and one or two nitrogen atoms;
R₂ is a folate, a steroid molecule, a reporter molecule, a lipophilic molecule, a reporter enzyme, a peptide, a protein, or has formula -Q-(CH₂CH₂-Q-)ₓ-R₃;
X is O or S;
each Q is, independently, NH, O, or S;
x is 1 to 200;
R₃ is H, R_{A}, C(O)OH, C(O)OR_{A}, C(O)R₄, RA-N₃, or R_{A}-NH₂;
R₄ is Cl, Br, I, SO₂R₅ or has structure: 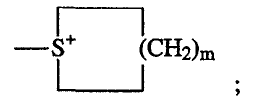
m is 2 to 7; and
R₅ is alkyl having 1 to 10 carbon atoms.

2. The compound of claim 1, wherein more than one of said nucleosides bear said substituent at a 2'-O-position, a 3'-O-position or a 5'-O-position.

3. The compound of claim 1, wherein R_{A} is (CH₂)ₙ where n is an integer from 1 to 10.

4. The compound of claim 1, wherein said R₁ₐ and R_{1b}, together, are phthalimido.

5. The compound of claim 1, wherein R₁ₐ is H and R_{1b} is R₂.

6. The compound of claim 1, wherein R₁ₐ is H and R_{1b} is C(O)-O-R₂.

7. The compound of claim 1, wherein R₁ₐ is H and R_{1b} is C(O)-(CH₂)ₙ-R₂ where n is an integer from 1 to 10.

8. The compound of claim 1, wherein R₁ₐ is H and R_{1b} is C(S)-NH-R₂.

9. The compound of claim 1, wherein R₂ includes pyrene, fluorescein, dinitrophenyl, cholesterol, acridine.

10. The compound of claim 1, wherein R₁ₐ is H and R_{1b} is C(O)-R₂.

11. The compound of claim 10, wherein R₂ has formula -O-(CH₂CH₂-O-)ₓ-R₃.

12. The compound of claim 1, wherein a 3'-position of at least one of said nucleosides is linked to a 5'-position of an adjacent nucleoside.

13. The compound of claim 1, wherein a 2'-position of at least one of said nucleosides is linked to a 5'-position of an adjacent nucleoside.

14. The compound of claim 1, wherein R_{A} is alkyl having from 1 to 7 carbon atoms; and R₁ₐ and R_{1b}, together, complete a cycloaryl moiety having two to six carbon atoms and one or two nitrogen atoms.

15. The compound of claim 14, wherein R_{A} is butyl; and R₁ₐ and R_{1b}, together, are imidazole.

16. A nucleoside comprising a ribofuranosyl sugar portion and a base portion, wherein said nucleoside bears at a 3'-O-position or a 5'-O-position a substituent having formula:
-R_{A}-N(R₁ₐ)(R_{1b})
where:
R_{A} is alkyl having from 1 to 10 carbon atoms;
R₁ₐ and R_{1b}, independently, are H, R₂, R_{A}, or an amine protecting group, or have formula C(X)-R₂, C(X)-R_{A}-R₂, C(X)-Q-R_{A}-R₂, C(X)-Q-R₂;
R₂ is a folate, a steroid molecule, a reporter molecule, a lipophilic molecule, a reporter enzyme, a peptide, a protein, or has formula -Q-(CH₂CH₂-Q-)ₓ-R₃;
X is O or S;
each Q is, independently, NH, O, or S;
x is 1 to 200;
R₃ is H, R_{A}, C(O)OH, C(O)OR_{A}, C(O)R₄, R_{A}-N₃, or R_{A}-NH₂; and
R₄ is Cl, Br, I, SO₂R₅ or has structure: 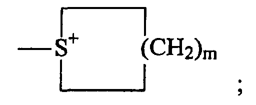
m is 2 to 7; and
R₅ is alkyl having 1 to 10 carbon atoms,
provided that when one of R₁ₐ or R_{1b} is H, the other of R₁ₐ or R_{1b} is not H.

17. The nucleoside of claim 16, wherein R_{A} is (CH₂)ₙ where n is an integer from 1 to 10.

18. The nucleoside of claim 16, wherein R₁ₐ and R_{1b}, together, are phthalimido.

19. The nucleoside of claim 16, wherein R₁ₐ is H and R_{1b} is C(O)-(CH₂)ₙ-R₂ where n is an integer from 1 to 10.

20. The nucleoside of claim 16, wherein R₁ₐ is H and R_{1b} is R₂.

21. The nucleoside of claim 16, wherein R₁ₐ is H and R_{1b} C(O)-O-R₂.

22. The nucleoside of claim 16, wherein R₁ₐ and R_{1b} both are alkyl.

23. The nucleoside of claim 16, wherein R₁ₐ is H and R_{1b} C(S)-NH-R₂.

24. The nucleoside of claim 16, wherein R₂ includes pyrene, fluorescein, dinitrophenyl, cholesterol, acridine.

25. The nucleoside of claim 16, wherein R₁ₐ is H and R_{1b} is C(O)-R₂.

26. The nucleoside of claim 25, wherein R₂ has formula -O-(CH₂CH₂-O-)ₓ-R₃.

27. An in vitro method for detecting the presence or absence of an RNA in a biological sample suspected of containing said RNA comprising contacting said sample with a compound of claim 1.

28. An in vitro method for detecting the presence or absence of an RNA in a biological sample suspected of containing said RNA comprising contacting said sample with a nucleoside of claim 16.

## Patentansprüche

1. Verbindung, umfassend eine Vielzahl von verknüpften Nucleosiden, wobei jedes Nucleosid einen Ribofuranosyl-Zuckerteil und einen Basenteil einschließt und mindestens eines der Nucleoside an einer 3'-O-Position oder einer 5'-O-Position einen Substituenten mit der Formel trägt:
-R_{A}-N(R₁ₐ)(R_{1b})
wobei
R_{A} ein Alkylrest mit 1 bis 10 Kohlenstoffatomen oder (CH₂-CH₂-Q)ₓ ist,
R₁ₐ und R_{1b} so gewählt sind, daß
R₁ₐ H ist, R₂ eine Aminschutzgruppe ist oder die Formeln C(X)-R₂, C(X)-R_{A}-R₂, C(X)-Q-R_{A}-R₂ oder C(X)-Q-R₂ aufweist,
R_{1b} R₂, eine Aminschutzgruppe ist oder die Formeln C(X)-R₂, C(X)-Rₐ-R₂, C(X)-Q-R_{A}-R₂ oder C(X)-Q-R₂ aufweist oder
R₁ₐ und R_{1b} zusammen eine Cycloalkyl- oder Cycloaryleinheit mit 2 bis sechs Kohlenstoffatomen und einem oder zwei Stickstoffatomen vervollständigen,
R₂ ein Folat, ein Steroidmolekül, ein Reportermolekül, ein lipophiles Molekül, ein Reporterenzym, ein Peptid, ein Protein ist oder die Formel -Q-(CH₂CH₂-Q-)ₓ-R₃ aufweist,
X O oder S ist,
jedes Q unabhängig NH, O oder S ist,
x 1 bis 200 ist,
R₃ H, R_{A}, C(O)OH, C(O)OR_{A}, C(O)R₄, R_{A}-N₃ oder R_{A}-NH₂ ist,
R₄ Cl, Br, I, SO₂R₅ ist oder die Struktur aufweist: 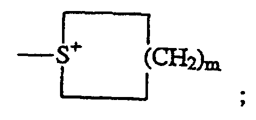
m 2 bis 7 ist und
R₅ ein Alkylrest mit 1 bis 10 Kohlenstoffatomen ist.

2. Verbindung nach Anspruch 1, wobei mehr als eines der Nucleoside den Substituenten an einer 2'-O-Position, einer 3'-O-Position oder einer 5'-O-Position trägt.

3. Verbindung nach Anspruch 1, wobei R_{A} (CH₂)ₙ ist, wobei n eine ganze Zahl von 1 bis 10 ist.

4. Verbindung nach Anspruch 1, wobei R₁ₐ und R_{1b} zusammen Phthalimid sind.

5. Verbindung nach Anspruch 1, wobei R₁ₐ H ist und R_{1b} R₂ ist.

6. Verbindung nach Anspruch 1, wobei R₁ₐ H ist und R_{1b} C(O)-O-R₂ ist.

7. Verbindung nach Anspruch 1, wobei R₁ₐ H ist und R_{1b} C(O)-(CH₂)ₙ-R₂ ist, wobei n eine ganze Zahl von 1 bis 10 ist.

8. Verbindung nach Anspruch 1, wobei R₁ₐ H und R_{1b} C(S)-NH-R₂ ist.

9. Verbindung nach Anspruch 1, wobei R₂ Pyren, Fluorescein, Dinitrophenyl, Cholesterol, Acridin einschließt.

10. Verbindung nach Anspruch 1, wobei R₁ₐ H ist und R_{1b} C(O)-R₂ ist.

11. Verbindung nach Anspruch 10, wobei R₂ die Formel -O-(CH₂CH₂-O-)ₓ-R₃ aufweist.

12. Verbindung nach Anspruch 1, wobei eine 3'-Position von mindestens einem der Nucleoside mit einer 5'-Position eines benachbarten Nucleosids verknüpft ist.

13. Verbindung nach Anspruch 1, wobei eine 2'-Position von mindestens einem der Nucleoside mit einer 5'-Position eines benachbarten Nucleosids verknüpft ist.

14. Verbindung nach Anspruch 1, wobei R_{A} eine Alkylgruppe mit 1 bis 7 Kohlenstoffatomen ist und R₁ₐ und R_{1b} zusammen eine Cycloaryleinheit mit zwei bis sechs Kohlenstoffatomen und einem oder zwei Stickstoffatomen vervollständigen.

15. Verbindung nach Anspruch 14, wobei R_{A} Butyl ist und R₁ₐ und R_{1b} zusammen Imidazol sind.

16. Nucleosid, umfassend einen Ribofuranosyl-Zuckerteil und einen Basenteil, wobei das Nucleosid an einer 3'-O-Position oder einer 5'-O-Position einen Substituenten mit der Formel:
-R_{A}-N(R₁ₐ)(R_{1b})
trägt, wobei
R_{A} eine Alkylgruppe mit 1 bis 10 Kohlenstoffatomen ist,
R₁ₐ und R_{1b} unabhängig voneinander H, R₂, R_{A} oder eine Aminschutzgruppe sind oder die Formel C(X)-R₂, C(X)-R_{A}-R₂, C(X)-Q-R_{A}-R₂, C(X)-Q-R₂ aufweisen,
R₂ ein Folat, ein Steroidmolekül, ein Reportermolekül, ein lipophiles Molekül, ein Reporterenzym, ein Peptid, ein Protein ist oder die Formel -Q-(CH₂CH₂-Q-)ₓ-R₃ aufweist,
X O oder S ist,
jedes Q unabhängig voneinander NH, O oder S ist,
x 1 bis 200 ist,
R₃ H, R_{A}, C(O)OH, C(O)OR_{A}, C(O)R₄, R_{A}-N₃ oder R_{A}-NH₂ ist und
R₄ Cl, Br, I, SO₂R₅ ist oder die Struktur aufweist: 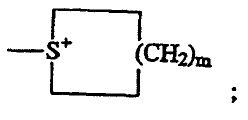
m 2 bis 7 ist und
R₅ eine Alkylgruppe mit 1 bis 10 Kohlenstoffatomen ist,
mit der Maßgabe, daß wenn einer von R₁ₐ oder R_{1b} H ist, der andere von R₁ₐ oder R_{1b} nicht H ist.

17. Nucleosid nach Anspruch 16, wobei R_{A} (CH₂)ₙ ist, wobei n eine ganze Zahl von 1 bis 10 ist.

18. Nucleosid nach Anspruch 16, wobei R₁ₐ und R_{1b} zusammen Phthalimid sind.

19. Nucleosid nach Anspruch 16, wobei R₁ₐ H ist und R_{1b} C(O)-(CH₂)ₙ-R₂ ist, wobei n eine ganze Zahl von 1 bis 10 ist.

20. Nucleosid nach Anspruch 16, wobei R₁ₐ H ist und R_{1b} R₂ ist.

21. Nucleosid nach Anspruch 16, wobei R₁ₐ H ist und R_{1b} C(O)-O-R₂ ist.

22. Nucleosid nach Anspruch 16, wobei R₁ₐ und R_{1b} beide Alkylgruppen sind.

23. Nucleosid nach Anspruch 16, wobei R₁ₐ H ist und R_{1b} C(S)-NH-R₂ ist.

24. Nucleosid nach Anspruch 16, wobei R₂ Pyren, Fluorescein, Dinitrophenyl, Cholesterol, Acridin einschließt.

25. Nucleosid nach Anspruch 16, wobei R₁ₐ H ist und R_{1b} C(O)-R₂ ist.

26. Nucleosid nach Anspruch 25, wobei R₂ die Formel -O-(CH₂CH₂-O-)ₓ-R₃ aufweist.

27. In-vitro-Verfahren zum Bestimmen der Anwesenheit oder Abwesenheit einer RNA in einer biologischen Probe, von der angenommen wird, die RNA zu enthalten, umfassend das Kontaktieren der Probe mit einer Verbindung nach Anspruch 1.

28. In-vitro-Verfahren zum Bestimmen der Anwesenheit oder Abwesenheit einer RNA in einer biologischen Probe, von der angenommen wird, die RNA zu enthalten, umfassend das Kontaktieren der Probe mit einem Nucleosid nach Anspruch 16.

## Revendications

1. Composé comprenant une pluralité de nucléosides liés, dans lequel chaque nucléoside inclut une portion sucre ribofurannosyle et une portion base, et au moins l'un desdits nucléosides porte en une position 3'-O ou une position 5'-O un substituant de formule
-R_{A}-N(R₁ₐ)(R_{1b})
dans laquelle
R_{A} est un groupe alkyle ayant de 1 à 10 atomes de carbone ou (CH₂-CH₂-Q)ₓ ;
R₁ₐ et R_{1b} sont choisis de telle façon que
R₁ₐ représente H, R₂, un groupe protecteur de fonction amino, ou un groupe de formule C(X)-R₂, C(X)-R_{A}-R₂, C(X)-Q-R_{A}-R₂ ou C(X)-Q-R₂,
R_{1b} représente R₂, un groupe protecteur de fonction amino, ou un groupe de formule C(X)-R₂, C(X)-R_{A}-R₂, C(X)-Q-R_{A}-R₂ ou C(X)-Q-R₂, ou
R₁ₐ et R_{1b} ensemble complètent un groupe cycloalkyle ou cycloaryle ayant de deux à six atomes de carbone et un ou deux atomes d'azote ;
R₂ est un folate, une molécule de stéroïde, une molécule rapporteuse, une molécule lipophile, une enzyme rapporteuse, un peptide, une protéine ou un groupe de formule -Q-(CH₂CH₂-Q-)ₓ-R₃ ;
X est O ou S ;
chaque symbole Q représente indépendamment NH, O ou S ;
x va de 1 à 200 ;
R₃ est H, R_{A}, C(O)OH, C(O)OR_{A}, C(O)R_{A}, R_{A}-N₃ ou R_{A}-NH₃ ;
R₄ est Cl, Br, I, SO₂R₅ ou correspond à la formule : 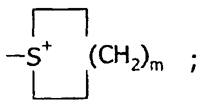
m va de 2 à 7 ; et
R₅ est un groupe alkyle ayant de 1 à 10 atomes de carbone.

2. Composé de la revendication 1, dans lequel plus d'un desdits nucléosides portent ledit substituant en une position 2'-O, une position 3'-O ou une position 5'-O.

3. Composé de la revendication 1, dans lequel R_{A} est (CH₂)ₙ où n est un nombre entier allant de 1 à 10.

4. Composé de la revendication 1, dans lequel lesdits groupes R₁ₐ et R_{1b} forment ensemble le groupe phtalimido.

5. Composé de la revendication 1, dans lequel R₁ₐ est H et R_{1b} est R₂.

6. Composé de la revendication 1, dans lequel R₁ₐ est H et R_{1b} est C(O)-O-R₂.

7. Composé de la revendication 1, dans lequel R₁ₐ est H et R_{1b} est C(O)-(CH₂)ₙ-R₂ où n est un nombre entier allant de 1 à 10.

8. Composé de la revendication 1, dans lequel R₁ₐ est H et R_{1b} est C(S)-NH-R₂.

9. Composé de la revendication 1, dans lequel R₂ inclut les restes pyrène, fluorescéine, dinitrophényle, cholestérol, acridine.

10. Composé de la revendication 1, dans lequel R₁ₐ est H et R_{1b} est C(O)-R₂.

11. Composé de la revendication 10, dans lequel R₂ correspond à la formule -O-(CH₂CH₂-O-)ₓ-R₃.

12. Composé de la revendication 1, dans lequel une position 3' d'au moins un desdits nucléosides est liée à une position 5' d'un nucléoside adjacent.

13. Composé de la revendication 1, dans lequel une position 2' d'au moins un desdits nucléosides est liée à une position 5' d'un nucléoside adjacent.

14. Composé de la revendication 1, dans lequel R_{A} est un groupe alkyle ayant de 1 à 7 atomes de carbone; et R₁ₐ et R_{1b} ensemble complètent un groupe cycloaryle ayant de deux à six atomes de carbone et un ou deux atomes d'azote.

15. Composé de la revendication 14, dans lequel R_{A} est le groupe butyle ; et R₁ₐ et R_{1b} ensemble forment le groupe imidazole.

16. Nucléoside comprenant un fragment sucre ribofurannosyle et un fragment base, dans lequel ledit nucléoside porte en une position 3'-O ou une position 5'-O un substituant de formule
-R_{A}-N(R₁ₐ)(R_{1b})
dans laquelle
R_{A} est un groupe alkyle ayant de 1 à 10 atomes de carbone ;
R₁ₐ et R_{1b} représentent indépendamment H, R₂, R_{A} ou un groupe protecteur de fonction amino, ou un groupe de formule C(X)-R₂, C(X)-R_{A}-R₂, C(X)-Q-R_{A}-R₂, C(X)-Q-R₂ ;
R₂ est un folate, une molécule de stéroïde, une molécule rapporteuse, une molécule lipophile, une enzyme rapporteuse, un peptide, une protéine ou un groupe de formule -Q-(CH₂CH₂-Q-)ₓ-R₃ ;
X est O ou S;
chaque symbole Q représente indépendamment NH, O ou S ;
x va de 1 à 200 ;
R₃ est H, R_{A}, C(O)OH, C(O)OR_{A}, C(O)R_{A}, R_{A}-N₃ ou R_{A}-NH₃ ; et
R₄ est Cl, Br, I, SO₂R₅ ou correspond à la formule : 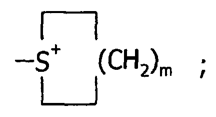
m va de 2 à 7 ; et
R₅ est un groupe alkyle ayant de 1 à 10 atomes de carbone,
étant entendu que si l'un des radicaux R₁ₐ et R_{1b} est H, l'autre est différent de H.

17. Nucléoside de la revendication 16, dans lequel R_{A} est (CH₂)ₙ où n est un nombre entier allant de 1 à 10.

18. Nucléoside de la revendication 16, dans lequel les groupes R₁ₐ et R_{1b} forment ensemble le groupe phtalimido.

19. Nucléoside de la revendication 16, dans lequel R₁ₐ est H et R_{1b} est C(O)-(CH₂)ₙ-R₂ où n est un nombre entier allant de 1 à 10.

20. Nucléoside de la revendication 16, dans lequel R₁ₐ est H et R_{1b} est R₂.

21. Nucléoside de la revendication 16, dans lequel R₁ₐ est H et R_{1b} est C(O)-O-R₂.

22. Nucléoside de la revendication 16, dans lequel R₁ₐ et R_{1b} représentent l'un et l'autre un groupe alkyle.

23. Nucléoside de la revendication 16, dans lequel R₁ₐ est H et R_{1b} est C(S)-NH-R₂.

24. Nucléoside de la revendication 16, dans lequel R₂ inclut les restes pyrène, fluorescéine, dinitrophényle, cholestérol, acridine.

25. Nucléoside de la revendication 16, dans lequel R₁ₐ est H et R_{1b} est C(O)-R₂.

26. Nucléoside de la revendication 25, dans lequel R₂ correspond à la formule -O-(CH₂CH₂-O-)ₓ-R₃.

27. Procédé in vitro pour la détection de la présence ou de l'absence d'un ARN dans un échantillon biologique suspecté de contenir ledit ARN, comprenant la mise en contact dudit échantillon avec un composé de la revendication 1.

28. Procédé in vitro pour la détection de la présence ou de l'absence d'un ARN dans un échantillon biologique suspecté de contenir ledit ARN, comprenant la mise en contact dudit échantillon avec un composé de la revendication 16.
